# EUROPÄISCHE PATENTSCHRIFT

(11) **EP 1 284 969 B1**
(45) Veröffentlichungstag und Bekanntmachung des Hinweises auf die Patenterteilung: **18.08.2004**
(21) Anmeldenummer: 01936353.0
(22) Anmeldetag: 11.05.2001
(51) Int. Cl.: C07D 261/04, A01N 43/80

(54) **3-(4,5-DIHYDROISOXAZOL-5-YL)BENZOYLCYCLOHEXENONE UND IHRE VERWENDUNG ALS HERBIZIDE**
3-(4,5-DIHYDROISOXAZOLE-5-YL)BENZOYLCYCLOHEXENONES AND THE USE THEREOF AS HERBICIDES
3-(4,5-DIHYDROISOXAZOL-5-YL)BENZOYLCYCLOHEXENONES ET LEUR UTILISATION COMME HERBICIDES

(30) Priorität: 18.05.2000 DE 10024107
(43) Veröffentlichungstag der Anmeldung: 26.02.2003
(73) Patentinhaber: BASF AKTIENGESELLSCHAFT, 67056 Ludwigshafen (DE)
(72) Erfinder: BAUMANN, Ernst, 67373 Dudenhofen (DE); VON DEYN, Wolfgang, 67435 Neustadt (DE); KUDIS, Steffen, 68167 Mannheim (DE); LANGEMANN, Klaus, 34270 Schauenburg (DE); MAYER, Guido, 67161 Gönnheim (DE); MISSLITZ, Ulf, 67433 Neustadt (DE); NEIDLEIN, Ulf, 68165 Mannheim (DE); WALTER, Helmut, 67283 Obrigheim (DE); ZAGAR, Cyrill, 68167 Mannheim (DE); WITSCHEL, Matthias, 67098 Bad Dürkheim (DE)
(86) Internationale Anmeldenummer: PCT/EP2001/005390
(87) Internationale Veröffentlichungsnummer: WO 2001/087856

(56) Entgegenhaltungen:
- WO-A-96/26200

## Beschreibung

Die vorliegende Erfindung betrifft 3-(4,5-Dihydroisoxazol-5-yl)benzoylcyclohexenone der Formel I 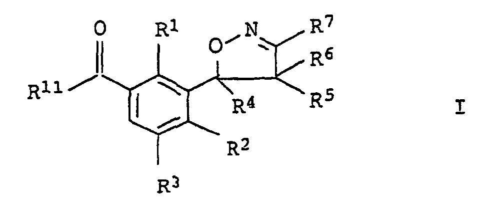 in der die Variablen folgende Bedeutungen haben:
- R¹, R²: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R³: Wasserstoff, Halogen oder C₁-C₆-Alkyl;
- R⁴: Wasserstoff oder C₁-C₄-Alkyl;
- R⁵, R⁶: Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di- (C₁-C₄-alkyl)-amino-C₁-C₄-alkyl, Di-(C₁-C₄-alkyl)-amino-imino-C₁-C₄-alkyl, Hydroxyimino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl,
C₁-C₄-Alkoxy oder C₁-C₄-Halogenalkoxy;
oder
- R⁵ und R⁶: bilden gemeinsam eine C₂-C₆-Alkandiyl-Kette, die einbis vierfach durch C₁-C₄-Alkyl substituiert sein kann und/oder durch Sauerstoff oder einen gegebenenfalls C₁-C₄-Alkyl substituierten Stickstoff unterbrochen sein kann;
- R⁷: Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di-(C₁-C₆-alkyl)amino, Di-(C₁-C₄-alkoxy)methyl, Hydroxylamino-C₁-C₄-alkyl, C₁-C₆-Alkoxyimino-C₁-C₄-alkyl oder COR⁸;
- R⁸: Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
- R⁹: Wasserstoff oder C₁-C₄-Alkyl;
- R¹⁰: C₁-C₄-Alkyl;
- R¹¹: ein in 2-Stellung verknüpftes Cyclohexenon der Formel II 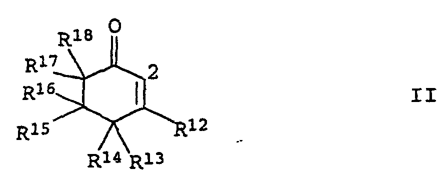 wobei
- R¹²: Hydroxy, Mercapto, Halogen, OR¹⁹, SR¹⁹, SOR²⁰ oder SO₂R²⁰;
- R¹³, R¹⁷: Wasserstoff, C₁-C₄-Alkyl, C₁-C₄-Alkylthio oder C₁-C₄-Alkoxycarbonyl;
- R¹⁴, R¹⁶, R¹⁸: Wasserstoff oder C₁-C₄-Alkyl;
- R¹⁵: Wasserstoff, Hydroxy, Halogen, C₁-C₆-Alkyl, C₁-C₆-Halogen-alkyl, Di(C₁-C₆-alkoxy)methyl, (C₁-C₆-Alkoxy) (C₁-C₆-alkylthio)methyl, Di(C₁-C₆-alkylthio)methyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Halogenalkoxycarbonyl,
1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die sechs letztgenannten Reste einen, zwei oder drei unter C₁-C₄-Alkyl ausgewählte Substituenten tragen könnnen;
oder
- R¹³ und R¹⁴ oder R¹⁷ und R¹⁸: gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
- R¹⁴ und R¹⁵ oder R¹⁵ und R¹⁸: gemeinsam für eine chemische Bindung oder C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹⁴ und R¹⁸: gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹⁵ und R¹⁶: gemeinsam für -O-(CH₂)ₚ-O-, -O-(CH₂)ₚ-S-, -S-(CH₂)ₚ-S-, -O-(CH₂)_{q}- oder -S-(CH₂)_{q}- stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹⁵ und R¹⁶: gemeinsam für ein Sauerstoffatom stehen;
- R¹⁹: C₁-C₆-Alkyl, C₃-C₆-Alkenyl, C₃-C₆-Halogenalkenyl, C₃-C₆-Alkinyl, C₃-C₆-Cycloalkyl, C₁-C₂₀-Alkylcarbonyl, C₂-C₂₀-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl, C₃-C₆-Alkenylaminocarbonyl, C₃-C₆-Alkinylaminocarbonyl, N,N-Di(C₁-C₆-alkyl)aminocarbonyl, N-(C₃-C₆-Alkenyl)-N-(C₁-C₆-alkyl)aminocarbonyl, N-(C₃-C₆-Alkinyl)-N- (C₁-C₆-alkyl) aminocarbonyl, N-(C₁-C₆-Alk-oxy)-N-(C₁-C₆-alkyl)aminocarbonyl, N,N-Di(C₁-C₆-alkyl)aminothiocarbonyl, C₁-C₆-Alkoxyimino-C₁-C₆-alkyl steht, wobei die genannten Alkyl-, Alkoxy- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können; Phenyl, Phenyl-C₁-C₆-alkyl, Phenylcarbonyl-C₁-C₆-alkyl, Phenylcarbonyl, Phenoxycarbonyl, Phenoxythiocarbonyl, Heterocycyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
- R²⁰: C₁-C₆-Alkyl, C₃-C₆-Alkenyl, C₃-C₆-Halogenalkenyl, C₃-C₆-Alkinyl oder C₃-C₆-Cycloalkyl steht, wobei die genannten Alkyl- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können;
Phenyl, Phenyl-C₁-C₆-alkyl, Phenylcarbonyl-C₁-C₆-alkyl, Heterocycyl, Heterocyclyl-C₁-C₆-alkyl oder Heterocyclylcarbonyl-C₁-C₆-alkyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
- p: 2, 3 oder 4;
- q: 1, 2, 3, 4 oder 5;
bedeuten;
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus WO 96/26200 sind Benzoylcyclohexenone bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 3-(4,5-Dihydroisoxazol-5-yl)benzoylcyclohexenone der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Ammonium, Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R²⁰ oder als Reste an Phenylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cyanoalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, Alkylcarbonyl, Halogenalkylcarbonyl, Alkenylcarbonyl-, Alkinylcarbonyl-, Alkoxycarbonyl-, Halogenalkoxycarbonyl, Alkenyloxycarbonyl-, Alkinyloxycarbonyl-, Alkylthiocarbonyl-, Alkylaminocarbonyl-, Alkenylaminocarbonyl-, Alkinylaminocarbonyl-, Dialkylamniocarbonyl-, (Alkenyl) (alkyl)aminocarbonyl-, (Alkinyl) (alkyl)ammiocarbonyl-, N-Alkoxy-N-alkylaminocarbonyl-, N,N-Dialkylaminothiocarbonyl-, Alkoxyalkoxycarbonyl-, Alkenyloxy-, Alkinyloxy-, Alkylamino, Dialkylamino-, Alkoxyalkyl-, Dialkoxymethyl, (Alkoxy)(alkylthio)mehtyl-, Dialkoxyalkyl-, Alkylthioalkyl-, Di(alkylthio)methyl-, Dialkylaminoalkyl-, Dialkylaminoiminoalkyl-, Hydroxyiminoalkyl, Alkoxyiminoalkyl-, Alkoxycarbonylalkyl-, Alkoxyalkoxy-, Alkenyl-, Halogenalkenyl-, Alkenyloxy, Alkinyl und Alkinyloxyteile sowie die Kohlenwasserstoffketten von Phenylalkyl, Phenylcarbonylalkyl, Heterocyclylalkyl und Heterocyclcarbonylalkyl, C₁-C₄-Alkylaminocarbonyl, Di-(C₁-C₄-alkyl)aminocarbonyl, Phenyl-C₁-C₄-alkyl, Phenylcarbonyl-C₁-C₄-alkyl, Heterocyclyl-C₁-C₄-alkyl und Heterocyclcarbonyl-C₁-C₄-alkyl können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: sowie die Alkylteile von C₁-C₄-Alkylcarbonyl, Di-(C₁-C₄-alkoxy)-C₁-C₄-alkyl, Di-(C₁-C₄-alkyl)-aminoimino-C₁-C₄-alkyl, C₁-C₆-Alkoxyimino-C₁-C₄-alkyl, Hydroxyimino-C₁-C₄-alkyl, C₁-C₄-Alkylaminocarbonyl, Di-(C₁-C₄-alkyl)aminocarbonyl, Phenyl-C₁-C₄-alkyl, Phenylcarbonyl-C₁-C₄-alkyl, Heterocyclyl-C₁-C₄-alkyl und Heterocyclylcarbonyl-C₁-C₄-alkyl: z.B. Methyl, Ethyl, Propyl, 1-Methylethyl, Butyl, 1-Methylpropyl, 2-Methylpropyl und 1,1-Dimethylethyl;
- C₁-C₆-Alkyl sowie die Alkylteile von C₁-C₆-Alkoxyimino-C₁-C₆-alkyl, N-C₃-C₆-Alkenyl-N-C₁-C₆-alkylaminocarbonyl, N-C₁-C₆-Alkinyl-N-C₁-C₆-alkylaminocarbonyl, N-C₁-C₆-Alkoxy-N-C₁-C₆-alkylaminocarbonyl, Phenyl-C₁-C₆-alkyl, Phenylcarbonyl-C₁-C₆-alkyl, Heterocyclyl-C₁-C₆-alkyl und Heterocyclylcarbonyl-C₁-C₆-alkyl: C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₂₀ Alkyl als Alkylteil von C₁-C₂₀-Alkylcarbonyl: C₁-C₆-Alkyl, wie voranstehend genannt sowie Heptyl, Octyl, Pentadecyl oder Heptadecyl;
- C₁-C₄-Halogenalkyl sowie die Halogenalkylteile von C₁-C₄-Halogenalkylcarbonyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Bromethyl, Iodmethyl, 1-Fluorethyl, 1-Chlorethyl, 1-Bromethyl, 1-Iodethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl: C₁-C₄-Halogenalkyl wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Cyanoalkyl: z.B. Cyanomethyl, 1-Cyanoeth-1-yl, 2-Cyanoeth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 1-Cyanobut-3-yl, 2-Cyanobut-3-yl, 1-Cyano-2-methyl-prop-3-yl, 2-Cyano-2-methylprop-3-yl, 3-Cyano-2-methyl-prop-3-yl und 2-Cyanomethylprop-2-yl;
- C₃-C₆-Alkenyl, sowie die Alkenylteile von C₃-C₆-Alkenylcarbonyl, C₃-C₆-Alkenylaminocarbonyl, N-(C₃-C₆-Alkenyl)-N-(C₁-C₆-alkyl)aminocarbonyl: Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en- 1-yl, 2-Methyl-prop-2-en-1-yl, Penten-1-yl, Penten-2-yl, Penten-3-yl, Penten-4-yl, 1-Methylbut-1-en-1-yl, 2-Methyl-but-1-en-1-yl, 3-Methylbut-1-en-1-yl, 1-Methyl-but-2-en-1-yl, 2-Methylbut-2-en-1-yl, 3-Methyl-but-2-en-1-yl, 1-Methylbut-3-en-1-yl, 2-Methyl-but-3-en-1-yl, 3-Methylbut-3-en-1-yl, 1,1-Dimethyl-prop-2-en-1-yl, 1,2-Dimethylprop-1-en-1-yl, 1,2-Dimethyl-prop-2-en-1-yl, 1-Ethylprop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₂-C₆-Alkenyl als Alkenylteile von C₂-C₆-Alkenylcarbonyl: C₃-C₆-Alkenyl, wie voranstehend genannt, sowie Ethenyl;
- C₂-C₂₀-Alkenyl als Alkenylteile von C₂-C₂₀-Alkenylcarbonyl: C₂-C₆-Alkenyl, wie voranstehend genannt, sowie 8-Pentadecen-1-yl, 8-Heptadecen-1-yl und 8,11-Heptadecadien-1-yl;
- C₃-C₆-Halogenalkenyl: einen C₃-C₆-Alkenylrest, wie voranstehend genannt, der partiell oder vollständig durch Fluor, Chlor, Brom und/oder Iod substituiert ist, also z. B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl, sowie die Alkinylteile von C₃-C₆-Alkinylcarbonyl, C₃-C₆-Alkinylaminocarbonyl, N-(C₃-C₆-Alkinyl)-N-(C₁-C₆-alkyl)-aminocarbonyl: z. B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methylbut-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methylpent-1-in-4-yl, 3-Methyl-pent-1-in-5-yl, 4-Methylpent-2-in-4-yl oder 4-Methyl-pent-2-in-5-yl;
- C₂-C₆-Alkinyl als Alkinylteil von C₂-C₆-Alkinylcarbonyl: C₃-C₆-Alkinyl, wie voranstehend genannt, sowie Ethinyl;
- C₁-C₄-Alkoxy sowie die Alkoxyteile von Di-(C₁-C₄-alkoxy)methyl und Di-(C₁-C₄-alkoxy)-C₁-C₄-alkyl: z.B. Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy sowie die Alkoxyteile von C₁-C₆-Alkoxyimino-C₁-C₄-alkyl, C₁-C₆-Alkoxyimino C₁-C₆-alkyl, N-C₁-C₆-Alkoxy-N-C₁-C₆-alkylaminocarbonyl, Di-(C₁-C₆-alkoxy)methyl und (C₁-C₆-Alkoxy) (C₁-C₆-alkylthio)methyl: C₁-C₄-Alkoxy wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy: C₁-C₄-Halogenalkoxy wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
- C₁-C₄-Alkylthio: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio sowie die Alkylthioteile von (C₁-C₆-Alkoxy) (C₁-C₆-alkylthio)methyl, Di(C₁-C₆-alkylthio)methyl und C₁-C₆-Alkylthiocarbonyl: C₁-C₄-Alkylthio wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
- C₁-C₄-Halogenalkylthio: einen C₁-C₄-Alkylthiorest wie voranstehend genannt, der partiell oder vollständig durch Fluor, Chlor; Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio und Nonafluorbutylthio;
- C₁-C₆-Halogenalkylthio: C₁-C₄-Halogenalkylthio wie voranstehend genannt, sowie z.B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
- C₁-C₆-Halogenalkylsulfinyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
- C₁-C₄-Alkylsulfonyl (C₁-C₆-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie z.B. Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,2,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₄-Halogenalkylsulfonyl: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl und Nonafluorbutylsulfonyl;
- C₁-C₆-Halogenalkylsulfonyl: einen C₁-C₄-Halogenalkylsulfonylrest wie voranstehend genannt, sowie 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl und 1,1-Dimethoxycarbonyl;
- C₁-C₆-Alkoxycarbonyl: ein C₁-C₄-Alkoxycarbonylrest wie voranstehend genannt sowie z. B: Pentoxycarbonyl, 1-Methylbutoxycarbonyl, 2-Methylbutoxycarbonyl, 3-Methylbutoxycarbonyl, 1,1-Dimethylpropoxycarbonyl, 1,2-Dimethylpropoxycarbonyl, 2,2-Dimethylpropoxycarbonyl, 1-Ethylpropoxycarbonyl, Hexoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,2,2-Timethylpropoxycarbonyl, 1,2,2-Timethylpropoxycarbonyl, 1-Ethyl-1-methylpropoxycarbonyl und 1-Ethyl-2-methylpropoxycarbonyl;
- C₁-C₄-Halogenalkoxycarbonyl: einen C₁-C₄-Alkoxycarbonylrest wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl, 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl und 4-Iodbutoxycarbonyl;
- C₁-C₆-Halogenalkoxycarbonyl: einen C₁-C₄-Halogenalkoxycarbonylrest wei voranstehend genannt sowie F-Fluorpentoxycarbonyl, 5-Chlorpentoxycarbonyl, 5-Brompentoxycarbonyl, 5-Jodpentoxycarbonyl, 6-Fluorhexoxycarbonyl, 6-Bromhexoxycarbonyl, 6-Jodhexoxycarbonyl und Dodecafluorhexoxycarbonyl;
- C₃-C₆-Alkenyloxy sowie die C₃-C₆-Alkenyloxyteile von C₃-C₆-Alkenyloxycarbonyl: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methylprop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methylbut-2-en-1-yloxy, 1-Methyl-but-3-en-1-yloxy, 2-Methylbut-3-en-1-yloxy, 3-Methyl-but-3-en-1-yloxy, 1,1-Dimethylprop-2-en-1-yloxy, 1,2-Dimethyl-prop-1-en-1-yloxy, 1,2-Dimethyl-prop-2-en-1-yloxy, 1-Ethyl-pxop-1-en-2-yloxy, 1-Ethylprop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methylpent-1-en-1-yloxy, 4-Methyl-pent-1-en-1-yloxy, 1-Methylpent-2-en-1-yloxy, 2-Methyl-pent-2-en-1-yloxy, 3-Methylpent-2-en-1-yloxy, 4-Methyl-pent-2-en-1-yloxy, 1-Methylpent-3-en-1-yloxy, 2-Methyl-pent-3-en-1-yloxy, 3-Methylpent-3-en-1-yloxy, 4-Methyl-pent-3-en-1-yloxy, 1-Methylpent-4-en-1-yloxy, 2-Methyl-pent-4-en-1-yloxy, 3-Methylpent-4-en-1-yloxy, 4-Methyl-pent-4-en-1-yloxy, 1,1-Dimethylbut-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethylbut-3-en-1-yloxy, 2-Ethyl-but-1-en-1-yloxy, 2-Ethylbut-2-en-1-yloxy, 2-Ethyl-but-3-en-1-yloxy, 1,1,2-Trimethylprop-2-en-1-yloxy, 1-Ethyl-1-methyl-prop-2-en-1-yloxy, 1-Ethyl-2-methyl-prop-1-en-1-yloxy und 1-Ethyl-2-methylprop-2-en-1-yloxy;
- C₃-C₆-Alkinyloxy sowie die Alkinyloxyteile von C₃-C₆-Alkinyloxycarbonyl: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methylpent-1-in-3-yloxy, 3-Methyl-pent-1-in-4-yloxy, 3-Methylpent-1-in-5-yloxy, 4-Methyl-pent-1-in-1-yloxy, 4-Methylpent-2-in-4-yloxy und 4-Methylpent-2-in-5-yloxy;
- C₁-C₆-Alkylamino sowie die Alkylaminoteile von C₁-C₆-Alkylaminocarbonyl: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- Di-(C₁-C₄-alkyl)-amino sowie die Dialkylamino-Teile von Di-(C₁-C₄-alkyl)aminoimino-C₁-C₄-alkyl und Di-(C₁-C₄-alkyl)aminocarbonyl: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)-amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di-(C₁-C₆-alkyl)amino sowie die Dialkylaminoteile von Di-(C₁-C₆-alkyl)aminocarbonyl und Di(C₁-C₆-alkyl)aminothiocarbonyl: Di-(C₁-C₄-alkyl)amino wie voranstehend genannt, sowie N,N-Dipentylamino, N,N-Dihexylamino, N-Methyl-N-pentylamino, N-Ethyl-N-pentylamino, N-Methyl-N-hexylamino oder N-Ethyl-N-hexylaminon;
- Di-(C₁-C₄-alkyl)-amino-C₁-C₄-alkyl: durch Di-(C₁-C₄-alkyl)-amino wie voranstehend genannt substituiertes C₁-C₄-Alkyl, also z.B. N,N-Dimethylaminomethyl, N,N-Diethylaminomethyl, N,N-Dipropylaminomethyl, N,N-Di-(1-methylethyl)aminomethyl, N,N-Dibutylaminomethyl, N,N-Di-(1-methylpropyl)aminomethyl, N,N-Di-(2-methylpropyl)aminomethyl, N,N-Di-(1,1-dimethylethyl)aminomethyl, N-Ethyl-N-methylaminomethyl, N-Methyl-N-propylaminomethyl, N-Methyl-N-(1-methylethyl)aminomethyl, N-Butyl-N-methylaminomethyl, N-Methyl-N-(1-methylpropyl)aminomethyl, N-Methyl-N-(2-methylpropyl)aminomethyl, N- (1,1-Dimethylethyl)-N-methylaminomethyl, N-Ethyl-N-propylaminomethyl, N-Ethyl-N-(1-methylethyl)aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-(1,1-dimethylethyl)aminomethyl, N-(1-Methylethyl)-N-propylaminomethyl, N-Butyl-N-propylaminomethyl, N-(1-Methylpropyl)-N-propylaminomethyl, N-(2-Methylpropyl)-N-propylaminomethyl, N-(1,1-dimethylethyl)-N-propylaminomethyl, N-Butyl-N-(1-methylethyl)aminomethyl, N-(1-Methylethyl)-N-(1-methylpropyl)aminomethyl, N-(1-Methylethyl)-N-(2-methylpropyl)aminomethyl, N-(1,1-Dimethylethyl)-N-(1-methylethyl)aminomethyl, N-Butyl-N-(1-methylpropyl)aminomethyl, N-Butyl-N-(2-methylpropyl)aminomethyl, N-Butyl-N-(1,1-dimethylethyl)aminomethyl, N-(1-Methylpropyl)-N-(2-methylpropyl)aminomethyl, N-(1,1-Dimethylethyl)-N-(1-methylpropyl)aminomethyl, N-(1,1-Dimethylethyl)-N-(2-methylpropyl)aminomethyl, 2-(N,N-Dimethylamino)-ethyl, 2-(N,N-Diethylamino)ethyl, 2-(N,N-Dipropylamino)-ethyl, 2-[N,N-Di-(1-methylethyl)amino]ethyl, 2- [N,N-Dibutylamino]ethyl, 2-[N,N-Di-(1-methylpropyl)amino]ethyl, 2-[N,N-Di-(2-methylpropyl)amino]ethyl, 2-[N,N-Di-(1,1-Dimethylethyl)amino]ethyl, 2-[N-Ethyl-N-methylamino]ethyl, 2-[N-Methyl-N-propylamino]ethyl, 2-[N-Methyl-N-(1-methylethyl) amino]ethyl, 2- [N-Butyl-N-methylamino]ethyl, 2-[N-Methyl-N-(1-methylpropyl)amino]ethyl, 2-[N-Methyl-N-(2-methylpropyl)amino]ethyl, 2- [N- (1,1-Dimethylethyl)-N-methylamino]ethyl, 2-[N-Ethyl-N-propylamino]ethyl, 2-[N-Ethyl-N-(1-methylethyl)amino]ethyl, 2-[N-Butyl-N-ethylamino]ethyl, 2-[N-Ethyl-N-(1-methylpropyl)amino]ethyl, 2-[N-Ethyl-N-(2-methylpropyl)amino]ethyl, 2-[N-Ethyl-N-(1,1-dimethylethylamino]ethyl, 2-[N-(1-Methylethyl)-N-propylamino]ethyl, 2-[N-Butyl-N-propylamino]ethyl, 2-[N-(1-Methylpropyl)-N-propylamino]ethyl, 2-[N-(2-Methylpropyl)-N-propylamino]ethyl, 2-[N-(1,1-Dimethylethyl)-N-propylamino]ethyl, 2-[N-Butyl-N-(1-methylethyl)amino]ethyl, 2-[N-(1-Methylethyl)-N-(1-methylpropyl)amino]ethyl, 2-[N-(1-Methylethyl)-N-(2-methylpropyl)amino]ethyl, 2-[N-(1,1-Dimethylethyl)-N-(1-methylethyl) amino] ethyl, 2- [N-Butyl-N-(1-methylpropyl)amino]ethyl, 2-[N-Butyl-N-(2-methylpropyl)amino]ethyl, 2-[N-Butyl-N-(1,1-Dimethylethyl)amino]ethyl, 2-[N-(1-Methylpropyl)-N-(2-methylpropyl)amino]ethyl, 2-[N-(1,1-Dimethylethyl)-N-(1-methylpropyl)amino]ethyl, 2-[N-(1,1-Dimethylethyl)-N-(2-methylpropyl)amino]ethyl, 3-(N,N-Dimethylamino)propyl, 3-(N,N-Diethylamino)propyl, 4-(N,N-Dimethylamino)butyl und 4-(N,N-Diethylamino)butyl;
- C₁-C₄-Alkoxy-C₁-C₄-alkyl: durch C₁-C₄-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy) ethyl, 2-(1,2-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)butyl;
- C₁-C₄-Alkylthio-C₁-C₄-alkyl: durch C₁-C₄-Alkylthio wie vorstehend genannt substituiertes C₁-C₄-Alkyl, also z.B. für Methylthiomethyl, Ethylthiomethyl, Propylthiomethyl, (1-Methylethylthio)methyl, Butylthiomethyl, (1-Methylpropylthio)methyl, (2-Methylpropylthio)methyl, (1,1-Dimethylethylthio)methyl, 2-Methylthioethyl, 2-Ethylthioethyl, 2-(Propylthio)ethyl, 2-(1-Methylethylthio)ethyl, 2-(Butylthio)ethyl, 2-(1-Methylpropylthio)ethyl, 2-(2-Methylpropylthio)ethyl, 2-(1,1-Dimethylethylthio)ethyl, 2-(Methylthio)propyl, 3-(Methylthio)propyl, 2-(Ethylthio)propyl, 3-(Ethylthio)propyl, 3-(Propylthio)propyl, 3-(Butylthio)propyl, 4-(Methylthio)butyl, 4-(Ethylthio)butyl, 4-(Propylthio)butyl und 4-(Butylthio)butyl;
- C₁-C₄-Alkoxycarbonyl-C₁-C₄-alkyl: durch C₁-C₄-Alkoxycarbonyl substituiertes C₁-C₄-Alkyl, also z.B. für Methoxycarbonylmethyl, Ethoxycarbonylmethyl, Propoxycarbonylmethyl, (1-Methylethoxycarbonyl)methyl, Butoxycarbonylmethyl, (1-Methylpropoxycarbonyl)methyl, (2-Methylpropoxycarbonyl)methyl, (1,1-Dimethylethoxycarbonyl)methyl, 2-(Methoxycarbonyl)ethyl, 2-(Ethoxycarbonyl)ethyl, 2-(Propoxycarbonyl)ethyl, 2-(1-Methylethoxycarbonyl)ethyl, 2-(Butoxycarbonyl)ethyl, 2-(1-Methylpropoxycarbonyl)ethyl, 2-(2-Methylpropoxycarbonyl)ethyl, 2-(1,1-Dimethylethoxycarbonyl) ethyl, 2-(Methoxycarbonyl)propyl, 2-(Ethoxycarbonyl)propyl, 2-(Propoxycarbonyl)propyl, 2-(1-Methylethoxycarbonyl)propyl, 2-(Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3- (Methoxycarbonyl) - propyl, 3-(Ethoxycarbonyl)propyl, 3-(Propoxycarbonyl)propyl, 3-(1-Methylethoxycarbonyl)propyl, 3-(Butoxycarbonyl)propyl, 3-(1-Methylpropoxycarbonyl)propyl, 3- (2-Methylpropoxycarbonyl)propyl, 3-(1,1-Dimethylethoxycarbonyl)propyl, 2-(Methoxycarbonyl)butyl, 2-(Ethoxycarbonyl)butyl, 2-(Propoxycarbonyl)butyl, 2-(1-Methylethoxycarbonyl)butyl, 2-(Butoxycarbonyl)butyl, 2-(1-Methylpropoxycarbonyl)butyl, 2-(2-Methylpropoxycarbonyl)butyl, 2-(1,1-Dimethylethoxycarbonyl)butyl, 3-(Methoxycarbonyl)butyl, 3-(Ethoxycarbonyl)butyl, 3-(Propoxycarbonyl)butyl, 3-(1-Methylethoxycarbonyl)butyl, 3-(Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)-butyl, 4-(Ethoxycarbonyl)butyl, 4-(Propoxycarbonyl)butyl, 4-(1-Methylethoxycarbonyl)butyl, 4-(Butoxycarbonyl)butyl, 4-(1-Methylpropoxy)butoxy, 4-(2-Methylpropoxy)butoxy und 4-(1,1-Dimethylethoxycarbonyl)butyl;
- C₁-C₄-Alkoxy-C₂-C₄-alkoxy: durch C₁-C₄-Alkoxy wie vorstehend genannt substituiertes C₂-C₄-Alkoxy, also z.B. für 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy)-butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy)-butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy und 4-(1,1-Dimethylethoxy)butoxy;
- C₂-C₅-Alkandiyl: z.B. Ethan-1,2-diyl, Propan-1,3-diyl, Butan-1,4-diyl und Pentan-1,5-diyl;
- C₁-C₅-Alkandiyl: C₂-C₅-Alkandiyl wie voranstehend genannt sowie Methandiyl;
- C₂-C₆-Alkandiyl: C₂-5-Alkandiyl wie voranstehend genannt sowie Hexan-1,6-diyl;
- C₃-C₆-Cycloalkyl: z.B. Cyclopropyl, Cyclobutyl, Cyclopentyl und Cyclohexyl;
- C₃-C₈-Cycloalkyl: C₃-C₆-Cycloalkyl wie voranstehend genannt sowie Cycloheptyl und Cyclooctyl;
- Heterocyclyl, sowie Heterocyclylteile von Heterocyclylcarbonyl, Heterocyclyl-C₁-C₆-alkyl, Heterocyclylcarbonyl-C₁-C₆-alkyl: ein gesättigter, partiell gesättigter oder ungesättigter 5- oder 6-gliedriger heterocyclischer Ring, der ein bis vier gleiche oder verschiedene Heteroatome, ausgewählt aus folgender Gruppe: Sauerstoff, Schwefel oder Stickstoff, enthält, also z. B. 5-gliedrige, gesättigte Ringe, wie:
   Tetrahydrofuran-2-yl, Tetrahydrofuran-3-yl, Tetrahydrothien-2-yl, Tetrahydrothien-3-yl,Tetrahydropyrrol-2-yl, Tetrahydropyrrol-3-yl, Tetrahydropyrazol-3-yl, Tetrahydropyrazol-4-yl, Tetrahydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl, 1,3,2-Dioxathiolan-4-yl;
   5-gliedrige, partiell gesättigte Ringe, wie:
      2,3-Dihydrofuran-2-yl, 2,3-Dihydrofuran-3-yl, 2,5-Dihydrofuran-2-yl, 2,5-Dihydrofuran-3-yl, 4,5-Dihydrofuran-2-yl, 4,5-Dihydrofuran-3-yl, 2,3-Dihydrothien-2-yl, 2,3-Dihydrothien-3-yl, 2,5-Dihydrothien-2-yl, 2,5-Dihydrothien-3-yl, 4,5-Dihydrothien-2-yl, 4,5-Dihydrothien-3-yl, 2,3-Dihydro-1Hpyrrol-2-yl, 2,3-Dihydro-1H-pyrrol-3-yl, 2,5-Dihydro-1Hpyrrol-2-yl, 2,5-Dihydro-1H-pyrrol-3-yl, 4,5-Dihydro-1Hpyrrol-2-yl, 4,5-Dihydro-1H-pyrrol-3-yl, 3,4-Dihydro-2Hpyrrol-2-yl, 3,4-Dihydro-2H-pyrrol-3-yl, 3,4-Dihydro-5Hpyrrol-2-yl, 3,4-Dihydro-5H-pyrrol-3-yl, 4,5-Dihydro-1Hpyrazol-3-yl, 4,5-Dihydro-1H-pyrazol-4-yl, 4,5-Dihydro-1Hpyrazol-5-yl, 2,5-Dihydro-1H-pyrazol-3-yl, 2,5-Dihydro-1Hpyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1,3-Dithiol-4-yl, 1,3-Oxathiol-2-yl, 1,3-Oxathiol-4-yl, 1,3-Oxathiol-5-yl, 1,2,3-Δ²-Oxadiazolin-4-yl, 1,2,3-Δ²-Oxadiazolin-5-yl, 1,2,4-Δ⁴-Oxadiazolin-3-yl, 1,2,4-Δ⁴-Oxadiazolin-5-yl, 1,2,4-Δ²-Oxadiazolin-3-yl, 1,2,4-Δ²-Oxadiazolin-5-yl, 1,2,4-Δ³-Oxadiazolin-3-yl, 1,2,4-Δ³-Oxadiazolin-5-yl, 1,3,4-Δ²-Oxadiazolin-2-yl, 1,3,4-Δ²-Oxadiazolin-5-yl, 1,3,4-Δ³-Oxadiazolin-2-yl, 1,3,4-Oxadiazolin-2-yl, 1,2,4-Δ⁴-Thiadiazolin-3-yl, 1,2,4-Δ⁴-Thiadiazolin-5-yl, 1,2,4-Δ³-Thiadiazolin-3-yl, 1,2,4-Δ³-Thiadiazolin-5-yl, 1,2,4-Δ²-Thiadiazolin-3-yl, 1,2,4-Δ²-Thiadiazolin-5-yl, 1,3,4-Δ²-Thiadiazolin-2-yl, 1,3,4-Δ²-Thiadiazolin-5-yl, 1,3,4-Δ³-Thiadiazolin-2-yl, 1,3,4-Thiadiazolin-2-yl, 1,2,3-Δ²-Triazolin-4-yl, 1,2,3-Δ²-Triazolin-5-yl, 1,2,4-Δ²-Triazolin-3-yl, 1,2,4-Δ²-Triazolin-5-yl, 1,2,4-Δ³-Triazolin-3-yl, 1,2,4-Δ³-Triazolin-5-yl, 1,2,4-Δ¹-Triazolin-2-yl, 1,2,4-Triazolin-3-yl, 3H-1,2,4-Dithiazol-5-yl, 2H-1,3,4-Dithiazol-5-yl, 2H-1,3;4-Oxathiazol-5-yl;
   5-gliedrige, ungesättigte Ringe, wie:
      2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl, 1,2,3-Oxadiazol-4-yl, 1,2,3-Oxadiazol-5-yl, 1,2,4-Oxadiazol-3-yl, 1,2,4-Oxadiazol-5-yl, 1,3,4-Oxadiazol-2-yl, 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl, 1,2,3-Triazol-4-yl, 1,2,4-Triazol-3-yl, Tetrazol-5-yl;
   6-gliedrige, gesättigte Ringe, wie:
      Tetrahydropyran-2-yl, Tetrahydropyran-3-yl, Tetrahydropyran-4-yl, Piperidin-2-yl, Piperidin-3-yl, Piperidin-4-yl, Tetrahydrothiopyran-2-yl, Tetrahydrothiopyran-3-yl, Tetrahydrothiopyran-4-yl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-4-yl, 1,3-Dithian-5-yl, 1,4-Dithian-2-yl, 1,3-Oxathian-2-yl, 1,3-Oxathian-4-yl, 1,3-Oxathian-5-yl, 1,3-Oxathian-6-yl, 1,4-Oxathian-2-yl, 1,4-Oxathian-3-yl, 1,2-Dithian-3-yl, 1,2-Dithian-4-yl, Hexahydropyrimidin-2-yl, Hexahydropyrimidin-4-yl, Hexahydropyrimidin-5-yl, Hexahydropyrazin-2-yl, Hexahydropyridazin-3-yl, Hexahydropyridazin-4-yl, Tetrahydro-1,3-oxazin-2-yl, Tetrahydro-1,3-oxazin-4-yl, Tetrahydro-1,3-oxazin-5-yl, Tetrahydro-1,3-oxazin-6-yl, Tetrahydro-1,3-thiazin-2-yl, Tetrahydro-1,3-thiazin-4-yl, Tetrahydro-1,3-thiazin-5-yl, Tetrahydro-1,3-thiazin-6-yl, Tetrahydro-1,4-thiazin-2-yl, Tetrahydro-1,4-thiazin-3-yl, Tetrahydro-1,4-oxazin-2-yl, Tetrahydro-1,4-oxazin-3-yl, Tetrahydro-1,2-oxazin-3-yl, Tetrahydro-1,2-oxazin-4-yl, Tetrahydro-1,2-oxazin-5-yl, Tetrahydro-1,2-oxazin-6-yl;
   6-gliedrige, partiell gesättigte Ringe, wie:
      2H-3,4-Dihydropyran-6-yl, 2H-3,4-Dihydropyran-5-yl, 2H-3,4-Dihydropyran-4-yl, 2H-3,4-Dihydropyran-3-yl, 2H-3,4-Dihydropyran-2-yl, 2H-3,4-Dihydropyran-6-yl, 2H-3,4-Dihydrothiopyran-5-yl, 2H-3,4-Dihydrothiopyran-4-yl, 2H-3,4-Dihydropyran-3-yl, 2H-3,4-Dihydropyran-2-yl, 1,2,3,4-Tetrahydropyridin-6-yl, 1,2,3,4-Tetrahydropyridin-5-yl, 1,2,3,4-Tetrahydropyridin-4-yl, 1,2,3,4-Tetrahydropyridin-3-yl, 1,2,3,4-Tetrahydropyridin-2-yl, 2H-5,6-Dihydropyran-2-yl, 2H-5,6-Dihydropyran-3-yl, 2H-5,6-Dihydropyran-4-yl, 2H-5,6-Dihydropyran-5-yl, 2H-5,6-Dihydropyran-6-yl, 2H-5,6-Dihydrothiopyran-2-yl, 2H-5,6-Dihydrothiopyran-3-yl, 2H-5,6-Dihydrothiopyran-4-yl, 2H-5,6-Dihydrothiopyran-5-yl, 2H-5,6-Dihydrothiopyran-6-yl, 1,2,5,6-Tetrahydropyridin-2-yl, 1,2,5,6-Tetrahydropyridin-3-yl, 1,2,5,6-Tetrahydropyridin-4-yl, 1,2,5,6-Tetrahydropyridin-5-yl, 1,2,5,6-Tetrahydropyridin-6-yl, 2,3,4,5-Tetrahydropyridin-2-yl, 2,3,4,5-Tetrahydropyridin-3-yl, 2,3,4,5-Tetrahydropyridin-4-yl, 2,3,4,5-Tetrahydropyridin-5-yl, 2,3,4,5-Tetrahydropyridin-6-yl, 4H-Pyran-2-yl, 4H-Pyran-3-yl-, 4H-Pyran-4-yl, 4H-Thiopyran-2-yl, 4H-Thiopyran-3-yl, 4H-Thiopyran-4-yl, 1,4-Dihydropyridin-2-yl, 1,4-Dihydropyridin-3-yl, 1,4-Dihydropyridin-4-yl, 2H-Pyran-2-yl, 2H-Pyran-3-yl, 2H-Pyran-4-yl, 2H-Pyran-5-yl, 2H-Pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 1,2-Dihydropyridin-2-yl, 1,2-Dihydropyridin-3-yl, 1,2-Dihydropyridin-4-yl, 1,2-Dihydropyridin-5-yl, 1,2-Dihydropyridin-6-yl, 3,4-Dihydropyridin-2-yl, 3,4-Dihydropyridin-3-yl, 3,4-Dihydropyridin-4-yl, 3,4-Dihydropyridin-5-yl, 3,4-Dihydropyridin-6-yl, 2,5-Dihydropyridin-2-yl, 2,5-Dihydropyridin-3-yl, 2,5-Dihydropyridin-4-yl, 2,5-Dihydropyridin-5-yl, 2,5-Dihydropyridin-6-yl, 2,3-Dihydropyridin-2-yl, 2,3-Dihydropyridin-3-yl, 2,3-Dihydropyridin-4-yl, 2,3-Dihydropyridin-5-yl, 2,3-Dihydropyridin-6-yl, 2H-5,6-Dihydro-1,2-oxazin-3-yl, 2H-5,6-Dihydro-1,2-oxazin-4-yl, 2H-5,6-Dihydro-1,2-oxazin-5-yl, 2H-5,6-Dihydro-1,2-oxazin-6-yl, 2H-5,6-Dihydro-1,2-thiazin-3-yl, 2H-5,6-Dihydro-1,2-thiazin-4-yl, 2H-5,6-Dihydro-1,2-thiazin-5-yl, 2H-5,6-Dihydro-1,2-thiazin-6-yl, 4H-5,6-Dihydro-1,2-oxazin-3-yl, 4H-5,6-Dihydro-1,2-oxazin-4-yl, 4H-5,6-Dihydro-1,2-oxazin-5-yl, 4H-5,6-Dihydro-1,2-oxazin-6-yl, 4H-5,6-Dihydro-1,2-thiazin-3-yl, 4H-5,6-Dihydro-1,2-thiazin-4-yl, 4H-5,6-Dihydro-1,2-thiazin-5-yl, 4H-5,6-Dihydro-1,2-thiazin-6-yl, 2H-3,6-Dihydro-1,2-oxazin-3-yl, 2H-3,6-Dihydro-1,2-oxazin-4-yl, 2H-3,6-Dihydro-1,2-oxazin-5-yl, 2H-3,6-Dihydro-1,2-oxazin-6-yl, 2H-3,6-Dihydro-1,2-thiazin-3-yl, 2H-3,6-Dihydro-1,2-thiazin-4-yl, 2H-3,6-Dihydro-1,2-thiazin-5-yl, 2H-3,6-Dihydro-1,2-thiazin-6-yl, 2H-3,4-Dihydro-1,2-oxazin-3-yl, 2H-3,4-Dihydro-1,2-oxazin-4-yl, 2H-3,4-Dihydro-1,2-oxazin-5-yl, 2H-3,4-Dihydro-1,2-oxazin-6-yl, 2H-3,4-Dihydro-1,2-thiazin-3-yl, 2H-3,4-Dihydro-2,2-thiazin-4-yl, 2H-3,4-Dihydro-1,2-thiazin-5-yl, 2H-3,4-Dihydro-1,2-thiazin-6-yl, 2,3,4,5-Tetrahydropyridazin-3-yl, 2,3,4,5-Tetrahydropyridazin-4-yl, 2,3,4,5-Tetrahydropyridazin-5-yl, 2,3,4,5-Tetrahydropyridazin-6-yl, 3,4,5,6-Tetrahydropyridazin-3-yl, 3,4,5,6-Tetrahydropyridazin-4-yl, 1,2,5,6-Tetrahydropyridazin-3-yl, 1,2,5,6-Tetrahydropyridazin-4-yl, 1,2,5,6-Tetrahydropyridazin-5-yl, 1,2,5,6-Tetrahydropyridazin-6-yl, 1,2,3,6-Tetrahydropyridazin-3-yl, 1,2,3,6-Tetrahydropyridazin-4-yl, 4H-5,6-Dihydro-2,3-oxazin-2-yl, 4H-5,6-Dihydro-1,3-oxazin-4-yl, 4H-5,6-Dihydro-1,3-oxazin-5-yl, 4H-5,6-Dihydro-1,3-oxazin-6-yl, 4H-5,6-Dihydro-1,3-thiazin-2-yl, 4H-5,6-Dihydro-1,3-thiazin-4-yl, 4H-5,6-Dihydro-1,3-thiazin-5-yl, 4H-5,6-Dihydro-1,3-thiazin-6-yl, 3,4,5-6-Tetrahydropyrimidin-2-yl, 3,4,5,6-Tetrahydropyrimidin-4-yl, 3,4,5,6-Tetrahydropyrimidin-5-yl, 3,4,5,6-Tetrahydropyrimidin-6-yl, 1,2,3,4-Tetrahydropyrazin-2-yl, 1,2,3,4-Tetrahydropyrazin-5-yl, 1,2,3,4-Tetrahydropyrimidin-2-yl, 1,2,3,4-Tetrahydropyrimidin-4-yl, 1,2,3,4-Tetrahydropyrimidin-5-yl, 1,2,3,4-Tetrahydropyrimidin-6-yl, 2,3-Dihydro-1,4-thiazin-2-yl, 2,3-Dihydro-1,4-thiazin-3-yl, 2,3-Dihydro-1,4-thiazin-5-yl, 2,3-Dihydro-1,4-thiazin-6-yl, 2H-1,2-Oxazin-3-yl, 2H-1,2-Oxazin-4-yl, 2H-1,2-Oxazin-5-yl, 2H-1,2-Oxazin-6-yl, 2H-1,2-Thiazin-3-yl, 2H-1,2-Thiazin-4-yl, 2H-1,2-Thiazin-5-yl, 2H-1,2-Thiazin-6-yl, 4H-1,2-Oxazin-3-yl, 4H-1,2-Oxazin-4-yl, 4H-1,2-Oxazin-5-yl, 4H-1,2-Oxazin-6-yl, 4H-1,2-Thiazin-3-yl, 4H-1,2-Thiazin-4-yl, 4H-1,2-Thiazin-5-yl, 4H-1,2-Thiazin-6-yl, 6H-1,2-Oxazin-3-yl, 6H-1,2-oxazin-4-yl, 6H-1,2-Oxazin-5-yl, 6H-1,2-oxazin-6-yl, 6H-1,2-Thiazin-3-yl, 6H-1,2-Thiazin-4-yl, 6H-1,2-Thiazin-5-yl, 6H-1,2-Thiazin-6-yl, 2H-1,3-Oxazin-2-yl, 2H-1,3-Oxazin-4-yl, 2H-1,3-Oxazin-5-yl, 2H-1,3-Oxazin-6-yl, 2H-1,3-Thiazin-2-yl, 2H-1,3-Thiazin-4-yl, 2H-1,3-Thiazin-5-yl, 2H-1,3-Thiazin-6-yl, 4H-1,3-Oxazin-2-yl, 4H-1,3-Oxazin-4-yl, 4H-1,3-Oxazin-5-yl, 4H-1,3-Oxazin-6-yl, 4H-1,3-Thiazin-2-yl, 4H-1,3-Thiazin-4-yl, 4H-1,3-Thiazin-5-yl, 4H-1,3-Thiazin-6-yl, 6H-1,3-Oxazin-2-yl, 6H-1,3-Oxazin-4-yl, 6H-1,3-oxazin-5-yl, 6H-1,3-Oxazin-6-yl, 6H-1,3-Thiazin-2-yl, 6H-1,3-Oxazin-4-yl, 6H-1,3-Oxazin-5-yl, 6H-1,3-Thiazin-6-yl, 2H-1,4-Oxazin-2-yl, 2H-1,4-Oxazin-3-yl, 2H-1,4-Oxazin-5-yl, 2H-1,4-Oxazin-6-yl, 2H-1,4-Thiazin-2-yl, 2H-1,4-Thiazin-3-yl, 2H-1,4-Thiazin-5-yl, 2H-1,4-Thiazin-6-yl, 4H-1,4-Oxazin-2-yl, 4H-1,4-Oxazin-3-yl, 4H-1,4-Thiazin-2-yl, 4H-1,4-Thiazin-3-yl, 1,4-Dihydropyridazin-3-yl, 1,4-Dihydropyridazin-4-yl, 1,4-Dihydropyridazin-5-yl, 1,4-Dihydropyridazin-6-yl, 1,4-Dihydropyrazin-2-yl, 1,2-Dihydropyrazin-2-yl, 1,2-Dihydropyrazin-3-yl, 1,2-Dihydropyrazin-5-yl, 1,2-Dihydropyrazin-6-yl, 1,4-Dihydropyrimidin-2-yl, 1,4-Dihydropyrimidin-4-yl, 1,4-Dihydropyrimidin-5-yl, 1,4-Dihydropyrimidin-6-yl, 3,4-Dihydropyrimidin-2-yl, 3,4-Dihydropyrimidin-4-yl, 3,4-Dihydropyrimidin-5-yl oder 3,4-Dihydropyrimidin-6-yl;
   6-gliedrige, ungesättigte Ringe, wie:
      Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl, 1,2,4-Triazin-3-yl, 1,2,4-Triazin-5-yl, 1,2,4-Triazin-6-yl, 1,2,4,5-Tetrazin-3-yl;
wobei gegebenenfalls der Schwefel der genannten Heterocyclen zu S=O oder S(=O)₂ oxidiert sein kann
und wobei mit einem ankondensierten Phenylring oder mit einem C₃-C₆-Carboxyclus oder mit einem weiteren 5- bis 6-gliedrigen Heterocyclus ein bicyclisches Ringsystem ausgebildet werden kann.

Bevorzugt werden folgende Heterocyclylreste verwendet:
5-gliedrige, ungesättigte Ringe wie voranstehend genannt, insbesondere 2-Furyl, 3-Furyl, 2-Thienyl, 3-Thienyl, Pyrrol-2-yl, Pyrrol-3-yl, Pyrazol-3-yl, Pyrazol-4-yl, Isoxazol-3-yl, Isoxazol-4-yl, Isoxazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl oder 1,2,4-Triazol-3-yl;
6-gliedrige, ungesättigte Ringe wie voranstehend genannt, insbesondere Pyridin-2-yl, Pyridin-3-yl, Pyridin-4-yl, Pyrimidin-2-yl, Pyrimidin-4-yl, Pyrimidin-5-yl, Pyrazin-2-yl oder 1,3,5-Triazin-2-yl;

Alle Pheny- bzw. Heterocyclylringe sind vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹, R²: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
besonders bevorzugt Nitro, Halogen, wie z.B. Chlor und Brom, C₁-C₆-Alkyl, wie z.B. Methyl und Ethyl, C₁-C₆-Alkoxy, wie z.B. Methoxy und Ethoxy, C₁-C₆-Halogenalkyl, wie z.B. Difluormethyl und Trifluormethyl, C₁-C₆-Alkylthio, wie z.B. Methylthio und Ethylthio, C₁-C₆-Alkylsulfinyl, wie z.B. Methylsulfinyl und Ethylsulfinyl, C₁-C₆-Alkylsulfonyl, wie z.B. Methylsulfonyl, Ethylsulfonyl und Propylsulfonyl oder C₁-C₆-Halogenalkylsulfonyl, wie z.B. Trifluormethylsulfonyl und Pentafluorethylsulfonyl;
- R³: Wasserstoff;
- R⁴: Wasserstoff;
- R⁵, R⁶: Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letzgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl,
C₁-C₄-Alkoxy oder C₁-C₄-Halogenalkoxy;
oder
- R⁵ und R⁶: bilden gemeinsam eine C₂-C₆-Alkandiyl-Kette, die ein bis vierfach durch C₁-C₄-Alkyl substituiert sein kann und/oder durch Sauerstoff oder einen gegebenenfalls C₁-C₄-Alkyl substituierten Stickstoff unterbrochen sein kann;
besonders bevorzugt steht R⁵ für Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl, C₁-C₄-Alkoxy oder C₁-C₄-Halogenalkoxy;
besonders bevorzugt steht R⁶ für Wasserstoff oder C₁-C₄-Alkyl;
insbesonders bevorzugt steht R⁵ für Wasserstoff, C₁-C₄-Alkyl, C₁-C₄-Halogenalkyl, C₁-C₄-Alkoxycarbonyl oder CONR⁹R¹⁰;
insbesonders bevorzugt steht R⁶ für Wasserstoff oder C₁-C₄-Alkyl;
außerordentlich bevorzugt steht R⁵ für Wasserstoff oder C₁-C₄-Alkyl, insbesondere für Wasserstoff;
außerordentlich bevorzugt steht R⁶ für Wasserstoff;
- R⁷: Halogen, C₁-C₆-Alkyl, C₁-C₆-Halogenalkyl, C₁-C₆-Alkoxy, C₁-C₆-Alkylthio, C₁-C₆-Alkylsulfinyl, C₁-C₆-Alkylsulfonyl, Amino, C₁-C₆-Alkylamino, Di-(C₁-C₆-alkyl)-amino, Di-(C₁-C₄-alkoxy)methyl oder COR⁸; besonders bevorzugt C₁-C₄-Alkyl, wie z.B. Methyl, Ethyl, 1-Methylethyl oder 1,1-Dimethylethyl, C₁-C₄-Alkylcarbonyl, wie z.B. Methylcarbonyl oder Ethylcarbonyl, Hydroxycarbonyl oder C₁-C₄-Alkoxycarbonyl, wie z.B. Methoxycarbonyl oder Ethoxycarbonyl; insbesondere bevorzugt C₁-C₄-Alkyl, wie z.B. Methyl, Ethyl, 1-Methylethyl oder 1,1-Dimethylethyl, Hydroxycarbonyl oder C₁-C₄-Alkoxycarbonyl; außerordentlich bevorzugt C₁-C₄-Alkyl, wie z.B. Methyl, Ethyl, 1-Methylethyl oder 1,1-Dimethylethyl;
- R⁸: C₁-C₄-Alkyl, Hydroxy, C₁-C₄-Alkoxy oder NR⁹R¹⁰;
- R⁹: Wasserstoff oder C₁-C₄-Alkyl;
- R¹⁰: C₁-C₄-Alkyl;
- R¹²: Hydroxy, OR¹⁹, SR¹⁹, SOR²⁰ oder SO₂R²⁰; insbesondere Hydroxy, OR¹⁹ oder SR¹⁹; besonders bevorzugt Hydroxy;
- R¹³, R¹⁷: Wasserstoff, C₁-C₄-Alkyl oder C₁-C₄-Alkylthio; insbesondere Wasserstoff, Methyl oder Methylthio; besonders bevorzugt Wasserstoff oder Methyl, insbesonders bevorzugt Wasserstoff;
- R¹⁴, R¹⁶, R¹⁸: Wasserstoff oder Methyl;
- R¹⁵: Wasserstoff, Hydroxy, C₁-C₆-Alkyl oder Di(C₁-C₆-alkoxy)methyl;
insbesondere Wasserstoff oder C₁-C₄-Alkyl;
oder
- R¹³ und R¹⁴ oder R¹⁴ und R¹⁵ oder R¹⁵ und R¹⁸ oder R¹⁴ und R¹⁸ oder R¹⁷ und R¹⁸: gemeinsam für C₁-C₅-Alkandiyl, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹⁵ und R¹⁶: gemeinsam für ein Sauerstoffatom;
- R¹⁹: C₁-C₆-Alkyl, C₁-C₂₀-Alkylcarbonyl, vorzugsweise C₁-C₆-Alkylcarbonyl, C₁-C₆-Alkoxycarbonyl, C₁-C₆-Alkylthiocarbonyl, wobei die genannten Alkyl- und Alkoxyreste partiell oder vollständig halogeniert sein können und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen können;
Phenyl, Phenyl-C₁-C₄-alkyl, Phenylcarbonyl-C₁-C₄-alkyl, Phenylcarbonyl, Phenoxycarbonyl, Heterocyclyl, Heterocyclyl-C₁-C₄-alkyl, Heterocyclylcarbonyl-C₁-C₄-alkyl, Heterocyclyloxycarbonyl, wobei der Phenyl- oder Heterocyclylrest der genannten Reste partiell oder vollständig halogeniert sein kann und/oder einen, zwei oder drei unter Nitro, Cyano, C₁-C₄-Alkyl,
C₁-C₄-Halogenalkyl, C₁-C₄-Alkoxy oder C₁-C₄-Halogenalkoxy ausgewählten Substituenten tragen kann.
- R²⁰: C₁-C₆-Alkyl, das partiell oder vollständig halogeniert sein kann und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen kann; Phenyl, Phenyl-C₁-C₄-alkyl, Phenylcarbonyl-C₁-C₄-alkyl, Heterocyclyl, Heterocyclyl-C₁-C₄-alkyl oder Heterocyclylcarbonyl-C₁-C₄-alkyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählten Substituenten tragen kann;

Folgende Ausführungsformen der 3-(4,5-Dihydroisoxazol-5-yl)-benzoylcyclohexenone der Formel I sind hervorzuheben:
1. In einer bevorzugten Ausführungsform der 3-(4,5-Dihydroisoxazol-5-yl)-benzoylcyclohexenone der Formel I stehen
   - R¹: für Nitro, Halogen, C₁-C₆-Alkyl, C₁-C₆-Halogenalkyl, C₁-C₆-Alkoxy oder C₁-C₆-Halogenalkoxy; besonders bevorzugt für Nitro, Halogen wie Chlor oder Brom, C₁-C₄-Alkyl, wie Methyl oder Ethyl, C₁-C₄-Halogenalkyl, wie Difluormethyl oder Trifluormethyl, C₁-C₄-Alkoxy, wie Methoxy oder Ethoxy, oder C₁-C₄-Halogenalkoxy wie Difluormethoxy oder Trifluormethoxy;
   insbesondere bevorzugt für Halogen, wie Chlor oder Brom, C₁-C₄-Alkyl, wie Methyl oder Ethyl oder C₁-C₄-Alkoxy, wie Methoxy oder Ethoxy; außerordentlich bevorzugt für Halogen, wie Chlor oder Brom, oder C₁-C₄-Alkyl, wie Methyl oder Ethyl;
   - R²: für Halogen, C₁-C₆-Halogenalkyl, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
   besonders bevorzugt für Halogen wie Fluor oder Chlor, C₁-C₄-Halogenalkyl, wie Difluormethyl oder Trifluormethyl, oder C₁-C₄-Alkylsulfonyl, wie Methylsulfonyl oder Ethylsulfonyl;
   insbesondere bevorzugt für Halogen wie Fluor oder Chlor, oder C₁-C₄-Alkylsulfonyl, wie Methylsulfonyl oder Ethylsulfonyl;
   außerordentlich bevorzugt für C₁-C₄-Alkylsulfonyl wie Methylsulfonyl oder Ethylsulfonyl;
2. In einer weiteren bevorzugten Ausführungsform der 3-(4,5-Dihydroisoxazol-5-yl)-benzoylcyclohexenone der Formel I steht
   - R³: für Wasserstoff.
3. In einer weiteren bevorzugten Ausführungsform der 3-(4,5-Dihydroisoxazol-5-yl)-benzoylcyclohexenone der Formel I steht
   - R³: für Halogen oder C₁-C₄-Alkyl; besonders bevorzugt für Chlor oder Methyl.
4. In einer weiteren Ausführungsform der 3-(4,5-Dihydroisoxazol-5-yl)-beznoylcyclohexenone der Formel I steht
   - R⁴: für Wasserstoff.
5. In einer weiteren Ausführungsform der 3-(4,5-Dihydroisoxazol-5-yl)-benzoylcyclohexenone der Formel I stehen
   - R⁴: R⁴ für Wasserstoff;
   - R⁵: für Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di(C₁-C₄-alkyl)-amino, C₁-C₄-Alkylcarbonyl, C₁-C₄-Alkoxycarbonyl, CONR⁹R¹⁰, Phenyl oder Benzyl, wobei die beiden letztgenannten Substituenten partiell oder vollständig halogeniert sein können und/oder eine bis drei der folgenden Gruppen tragen können:
   Nitro, Cyano, C₁-C₄-Alkyl, C₁-C₄-Halogenalkyl, C₁-C₄-Alkoxy oder C₁-C₄-Haloge-nalkoxy;
   besonders bevorzugt für Wasserstoff, C₁-C₄-Alkyl, C₁-C₄-Halogenalkyl, C₁-C₄-Alkoxycarbonyl oder CONR⁹R¹⁰;
   insbesondere bevorzugt für Wasserstoff oder C₁-C₄-Alkyl, wie Methyl oder Ethyl;
   außerordentlich bevorzugt für Wasserstoff;
   - R⁶: für Wasserstoff oder C₁-C₄-Alkyl, wie Methyl oder Ethyl;
   besonders bevorzugt für Wasserstoff.
6. In einer weiteren bevorzugten Ausführungsform der 3-(4,5-Dihydroisoxazol-5-yl)-benzoylcyclohexenone der Formel I steht
   - R⁷: für Halogen, C₁-C₆-Alkyl, C₁-C₆-Halogenalkyl, C₁-C₆-Alkoxy, C₁-C₆-Alkylthio, C₁-C₆-Alkylsulfinyl, C₁-C₆-Alkylsulfonyl, Amino, C₁-C₆-Alkylamino, Di-(C₁-C₆-alkyl)amino, Di(C₁-C₆-alkoxy)methyl, Formyl, C₁-C₄-Alkylcarbonyl, C₁-C₄-Halogenalkylcarbonyl, Hydroxycarbonyl, C₁-C₄-Alkoxycarbonyl, C₁-C₄-Halogenalkoxycarbonyl oder CONR⁹R¹⁰; besonders bevorzugt Halogen, wie Chlor oder Brom, C₁-C₄-Alkyl, wie Methyl, Ethyl, 1-Methylethyl oder 1,1-Dimethylethyl, C₁-C₄-Alkoxy, wie Methoxy oder Ethoxy, C₁-C₄-Alkylthio, wie Methylthio oder Ethylthio, C₁-C₄-Alkylsulfinyl, wie Methylsulfinyl oder Ethylsulfinyl, C₁-C₄-Alkylsulfonyl, wie Methylsulfonyl oder Ethylsulfonyl, Amino, C₁-C₄-Alkylamino, wie Methylamino oder Ethylamino, Di-(C₁-C₄-alkyl)amino, wie Dimethylamino oder Diethylamino, Di(C₁-C₄-alkoxy)methyl, wie Dimethoxymethyl oder Diethoxymethyl, C₁-C₄-Alkylcarbonyl, wie Methylcarbonyl oder Ethylcarbonyl, Hydroxycarbonyl, C₁-C₄-Alkoxycarbonyl, wie Methoxycarbonyl oder Ethoxycarbonyl oder CONR⁹R¹⁰; insbesonders bevorzugt C₁-C₄-Alkyl, wie Methyl, Ethyl, 1-Methylethyl oder 1,1-Dimethylethyl, C₁-C₄-Alkylcarbonyl, wie Methylcarbonyl oder Ethylcarbonyl, Hydroxycarbonyl oder C₁-C₄-Alkoxycarbonyl, wie Methoxycarbonyl oder Ethoxycarbonyl; außerordentlich bevorzugt C₁-C₄-Alkyl, wie Methyl, Ethyl, 1-Methylethyl oder 1,1-Dimethylethyl, Hydroxycarbonyl oder C₁-C₄-Alkoxycarbonyl oder Ethoxycarbonyl;
   außerordentlich besonders bevorzugt C₁-C₄-Alkyl, wie Methyl, Ethyl, 1-Methylethyl oder 1,1-Dimethylethyl.
7. In einer weiteren bevorzugten Ausführungsform der 3-(4,5-Dihydroisoxazol-5-yl)benzoylcyclohexenone der Formel I stehen
   - R¹²: für Hydroxy, OR¹⁹ oder SR¹⁹; und
   - R¹⁹: für C₁-C₆-Alkyl, C₁-C₂₀-Alkylcarbonyl, C₁-C₆-Alkoxycarbonyl, C₁-C₆-Alkylthiocarbonyl, wobei die Alkylund Alkoxyreste partiell oder vollständig halogeniert sein können und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen können;
   Phenyl, Phenyl-C₁-C₄-alkyl, Phenylcarbonyl-C₁-C₄alkyl, Phenylcarbonyl, Phenoxycarbonyl, Heterocyclyl, Heterocyclyl-C₁-C₄-alkyl, Heterocyclylcarbonyl-C₁-C₄-alkyl, Heterocyclyloxycarbonyl steht, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählten Substituenten tragen kann.
   Heterocyclyl (alleine oder in Kombination) steht dabei vorzugsweise für einen 5-gliedrigen ungesättigten Ring oder einen 6-gliedrigen ungesättigten Ring, insbesondere Pyridin-2-yl oder Pyridin-3-yl.
   Insbesondere bevorzugt steht Heterocyclyl (alleine oder in Kombination) für 5-gliedrige ungesättigte Ringe mit einem Heteroatom wie 2-Furyl, 3-Furyl, 2-Thilnyl, 3-Thilnyl, Pyrrol-2-yl oder Pyrrol-3-yl;
   außerordentlich bevorzugt steht Heterocyclyl für 2-Thienyl oder 3-Thienyl.
8. In einer weiteren bevorzugten Ausführungsfom der 3-(4,5-Dihydroisoxazol-5-yl)benzoylcyclohexenone der Formel I steht R¹² für Hydroxy.
9. In einer weiteren bevorzugten Ausführungsfom der 3-(4,5-Dihydroisoxazol-5-yl)benzoylcyclohexenone der Formel I stehen
   - R¹³ und R¹⁷: für Wasserstoff, C₁-C₄-Alkyl oder C₁-C₄-Alkylthio;
   - R¹⁴, R¹⁶, R¹⁸: für Wasserstoff oder Methyl;
   - R¹⁵: für Wasserstoff, Hydroxy, C₁-C₆-Alkyl oder Di(C₁-C₆-alkoxy) methyl;
   oder
   - R¹³ und R¹⁴ oder R¹⁴ und R¹⁵ oder R¹⁵ und R¹⁶ oder R¹⁴ und R¹⁸ oder R¹⁷ und R¹⁸: gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
   oder
   - R¹⁵ und R¹⁶: gemeinsam für ein Sauerstoffatom stehen.

Insbesondere bevorzugt stehen
- R¹³, R¹⁷: für Wasserstoff oder C₁-C₄-Alkyl;
- R¹⁴, R¹⁶, R¹⁸: für Wasserstoff oder Methyl;
- R¹⁵: für Wasserstoff, C₁-C₆-Alkyl oder Di(C₁-C₆-alkoxy)methyl;
außerordentlich bevorzugt für Wasserstoff oder C₁-C₆-Alkyl;
oder
- R¹⁴ und R¹⁸: gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
- R¹⁵ und R¹⁶: stehen gemeinsam für ein Sauerstoffatom.

Insbesonders außerordentlich bevorzugt sind die 3-(4,5-Dihydroisoxazol-5-yl)benzoylcyclohexenone der Formel Ia1 (≡ I mit R¹ = Cl; R² = SO₂CH₃; R³, R⁴, R¹⁵ bis R¹⁸ = H; R¹² = OH), besonders die Verbindungen Ia1.1 bis Ia1.64, wobei die Restedefinitionen R¹ bis R¹⁴ nicht nur in Kombination miteinander, sondern auch jeweils für sich allein betrachtet für die erfindungsgemäßen Verbindungen eine besondere Bedeutung haben.

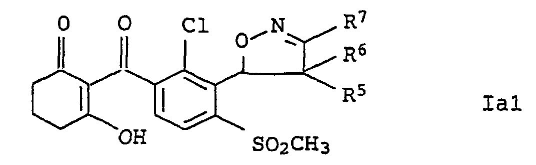

**Tabelle 1:**

| Nr. | R⁵ | R⁶ | R⁷ |
|---|---|---|---|
| Ia1.1 | H | H | CH₃ |
| Ia1.2 | Cl | H | CH₃ |
| Ia1.3 | Br | H | CH₃ |
| Ia1.4 | CN | H | CH₃ |
| Ia1.5 | NO₂ | H | CH₃ |
| Ia1.6 | CH₃ | H | CH₃ |
| Ia1.7 | CH₂CH₃ | H | CH₃ |
| Ia1. 8 | CH(CH₃)₂ | H | CH₃ |
| Ia1.9 | C(CH₃)₃ | H | CH₃ |
| Ia1.10 | CHO | H | CH₃ |
| Ia1.11 | CH=NOH | H | CH₃ |
| Ia1.12 | CH=NOCH₃ | H | CH₃ |
| Ia1.13 | CH=NOCH₂CH₃ | H | CH₃ |
| Ia1.14 | COCH₃ | H | CH₃ |
| Ia1.15 | C(=NOH)CH₃ | H | CH₃ |
| Ia1.16 | C (=NOCH₃) CH₃ | H | CH₃ |
| Ia1.17 | C(=NOCH₂CH₃)CH₃ | H | CH₃ |
| Ia1.18 | CH=NN(CH₃)₂ | H | CH₃ |
| Ia1.19 | C [=NN(CH₃)₂]CH₃ | H | CH₃ |
| Ia1.20 | COOH | H | CH₃ |
| Ia1.21 | COOCH₃ | H | CH₃ |
| Ia1.22 | COOCH₂CH₃ | H | CH₃ |
| Ia1.23 | OCH₃ | H | CH₃ |
| Ia1.24 | OCH₂CH₃ | H | CH₃ |
| Ia1.25 | SCH₃ | H | CH₃ |
| Ia1.26 | CH₂F | H | CH₃ |
| Ia1.27 | CHF₂ | H | CH₃ |
| Ia1.28 | CF₃ | H | CH₃ |
| Ia1.29 | CF₂CF₃ | H | CH₃ |
| Ia1.30 | CH₂Cl | H | CH₃ |
| Ia1.31 | CH₂CN | H | CH₃ |
| Ia1.32 | CH₂-CH₂ | | CH₃ |
| Ia1.33 | CH₂-CH₂-CH₂ | | CH₃ |
| Ia1.34 | CH₂-CH₂-CH₂-CH₂ | | CH₃ |
| Ia1.35 | CH₂-CH₂-CH₂-CH₂-CH₂ | | CH₃ |
| Ia1.36 | CH₃ | CH₃ | CH₃ |
| Ia1.37 | CH₃ | CH₂CH₃ | CH₃ |
| Ia1.38 | CF₃ | COCH₃ | CH₃ |
| Ia1.39 | CF₃ | COOCH₃ | CH₃ |
| Ia1.40 | CN | COCH₃ | CH₃ |
| Ia1.41 | CN | COOCH₃ | CH₃ |
| Ia1.42 | CN | CN | CH₃ |
| Ia1.43 | COCH₃ | COCH₃ | CH₃ |
| Ia1.44 | COCH₃ | COOCH₃ | CH₃ |
| Ia1.45 | H | H | CH₂CH₃ |
| Ia1.46 | H | H | CH(CH₃)₂ |
| Ia1.47 | H | H | C(CH₃)₃ |
| Ia1.48 | H | H | Cl |
| Ia1.49 | H | H | OCH₃ |
| Ia1.50 | H | H | SCH₃ |
| Ia1.51 | H | H | SOCH₃ |
| Ia1.52 | H | H | SO₂CH₃ |
| Ia1.53 | H | H | CN |
| Ia1.54 | H | H | CHO |
| Ia1.55 | H | H | CH=NOH |
| Ia1.56 | H | H | CH=NOCH₃ |
| Ia1.57 | H | H | CH=NOCH₂CH₃ |
| Ia1.58 | H | H | COCH₃ |
| Ia1.59 | H | H | C(=NOH)CH₃ |
| Ia1.60 | H | H | C(=NOCH₃)CH₃ |
| Ia1.61 | H | H | COOH |
| Ia1.62 | H | H | COOCH₃ |
| Ia1.63 | H | H | COOCH₂CH₃ |
| Ia1.64 | H | H | CH(OCH₂CH₃)₂ |

Ebenso insbesondere bevorzugt sind die Verbindungen Ia2, besonders die Verbindungen Ia2.1 - Ia2.64, die sich von den Verbindungen Ia1.1 - Ia1.64 dadurch unterscheiden, daß R¹⁵ für Methyl steht.

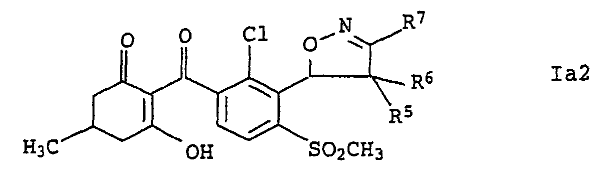

Ebenso insbesondere bevorzugt sind die Verbindungen Ia3., besonders die Verbindungen Ia3.1 - Ia3.64, die sich von den Verbindungen Ia1.1 - Ia1.64 dadurch unterscheiden, daß R¹⁵ und R¹⁶ für Methyl stehen.

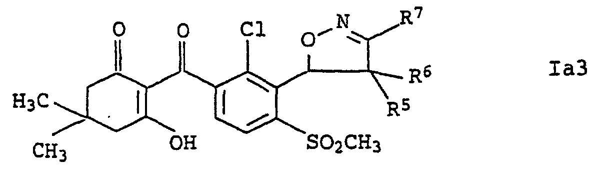

Ebenso insbesondere bevorzugt sind die Verbindungen Ia4., besonders die Verbindungen Ia4.1 - Ia4.64, die sich von den Verbindungen Ia1.1 - Ia1.64 dadurch unterscheiden, daß R¹³ und R¹⁷ für Methyl stehen.

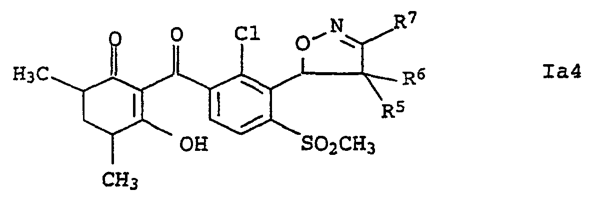

Ebenso insbesondere bevorzugt sind die Verbindungen Ia5., besonders die Verbindungen Ia5.1 - Ia5.64, die sich von den Verbindungen Ia1.1 - Ia1.64 dadurch unterscheiden, daß R¹³ für Methylthio und R¹⁴ für Methyl stehen.

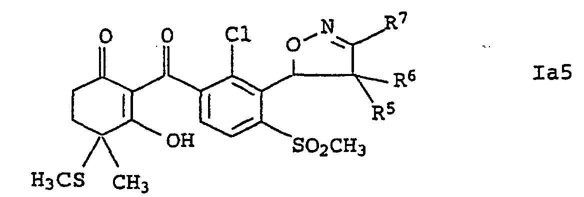

Ebenso insbesondere bevorzugt sind die Verbindungen Ia6., besonders die Verbindungen Ia6.1 - Ia6.64, die sich von den Verbindungen Ia1.1 - Ia1.64 dadurch unterscheiden, daß R¹⁴ und R¹⁸ gemeinsam für Ethan-1,2-diyl stehen.

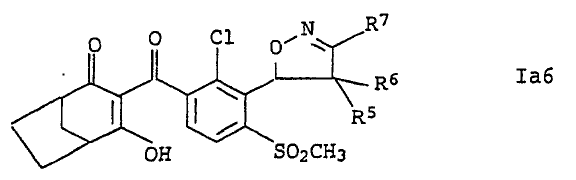

Ebenso insbesondere bevorzugt sind die Verbindungen Ia7., besonders die Verbindungen Ia7.1 - Ia7.64, die sich von den Verbindungen Ia1.1 - Ia1.64 dadurch unterscheiden, daß R¹³, R¹⁴, R¹⁷ und R¹⁸ für Methyl und R¹⁵ und R¹⁶ für Sauerstoff stehen.

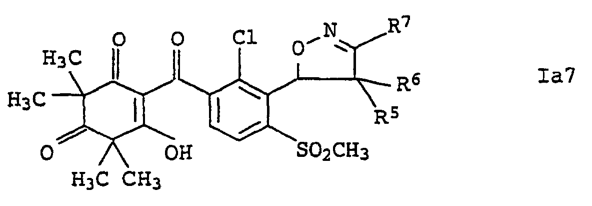

Ebenso insbesondere bevorzugt sind die Verbindungen Ia8., besonders die Verbindungen Ia8.1 - Ia8.64, die sich von den Verbindungen Ia1.1 - Ia1.64 dadurch unterscheiden, daß R¹⁵ für Hydroxy steht.

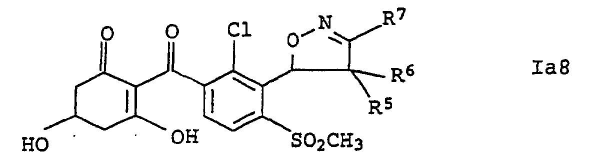

Ebenso insbesondere bevorzugt sind die Verbindungen Ia9., besonders die Verbindungen Ia9.1 - Ia9.64, die sich von den Verbindungen Ia1.1 - Ia1.64 dadurch unterscheiden, daß R¹ für Methyl steht.

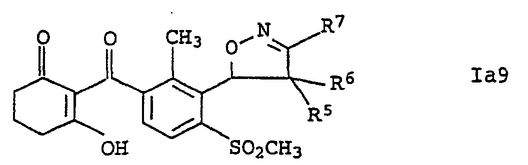

Ebenso insbesondere bevorzugt sind die Verbindungen Ia10., besonders die Verbindungen Ia10.1 - Ia10.64, die sich von den Verbindungen Ia1.1 - Ia1.64 dadurch unterscheiden, daß R¹ und R¹⁵ für Methyl stehen.

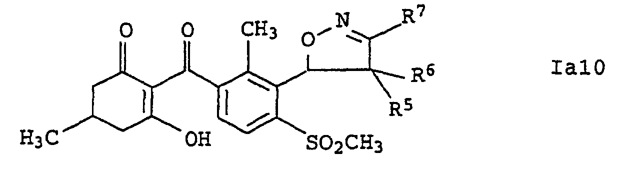

Ebenso insbesondere bevorzugt sind die Verbindungen Ia11., besonders die Verbindungen Ia11.1 - Ia11.64, die sich von den Verbindungen Ia1.1 - Ia1.64 dadurch unterscheiden, daß R¹, R¹⁵ und R¹⁶ für Methyl stehen.

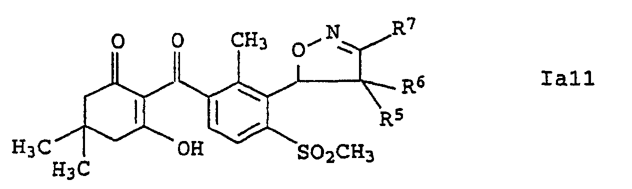

Ebenso insbesondere bevorzugt sind die Verbindungen Ia12., besonders die Verbindungen Ia22.1 - Ia12.64, die sich von den Verbindungen Ia1.1 - Ia1.64 dadurch unterscheiden, daß R¹, R¹³ und R¹⁷ für Methyl stehen.

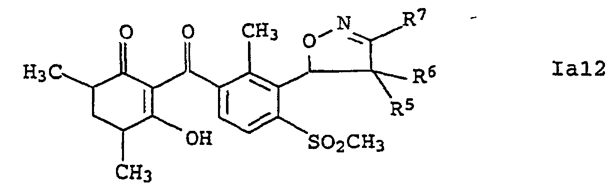

Ebenso insbesondere bevorzugt sind die Verbindungen Ia13., besonders die Verbindungen Ia13.1 - Ia13.64, die sich von den Verbindungen Ia1.1 - Ia1.64 dadurch unterscheiden, daß R¹ und R¹⁴ für Methyl und R¹³ für Methylthio stehen.

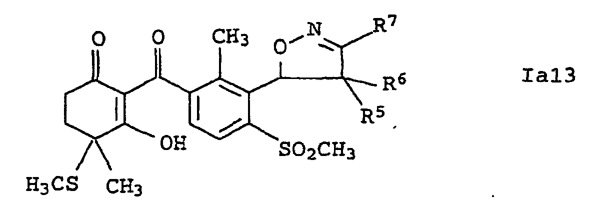

Ebenso insbesondere bevorzugt sind die Verbindungen Ia14., besonders die Verbindungen Ia14.1 - Ia14.64, die sich von den Verbindungen Ia1.1 - Ia1.64 dadurch unterscheiden, daß R¹ für Methyl und R¹⁴ und R¹⁸ gemeinsam für Ethan-1,2-diyl stehen.

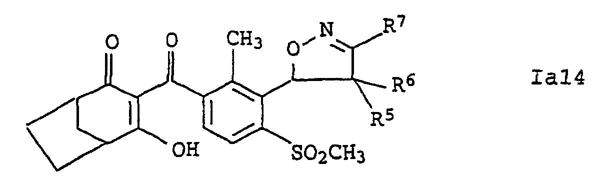

Ebenso insbesondere bevorzugt sind die Verbindungen Ia15., besonders die Verbindungen Ia15.1 - Ia15.64, die sich von den Verbindungen Ia1.1 - Ia1.64 dadurch unterscheiden, daß R¹, R¹³, R¹⁴, R¹⁷ und R¹⁸ für Methyl und R¹⁵ und R¹⁶ für Sauerstoff stehen.

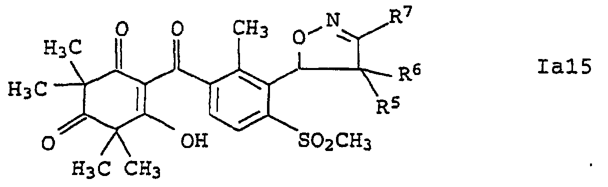

Ebenso insbesondere bevorzugt sind die Verbindungen Ia16., besonders die Verbindungen Ia16.1 - Ia16.64, die sich von den Verbindungen Ia1.1 - Ia1.64 dadurch unterscheiden, daß R¹ für Methyl und R¹⁵ für Hydroxy stehen.

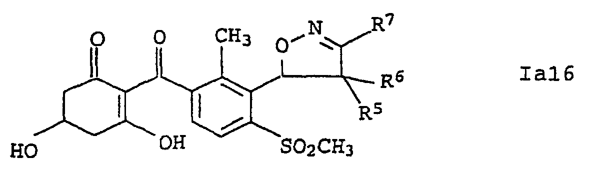

Ebenso insbesondere bevorzugt sind die Verbindungen Ia17., besonders die Verbindungen Ia17.1 - Ia17.64, die sich von den Verbindungen Ia1.1 - Ia1.64 dadurch unterscheiden, daß R¹ für Trifluormethyl steht.

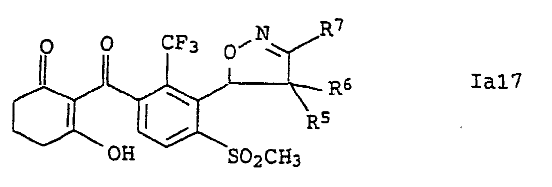

Ebenso insbesondere bevorzugt sind die Verbindungen Ia18., besonders die Verbindungen Ia18.1 - Ia18.64, die sich von den Verbindungen Ia1.1 - Ia1.64 dadurch unterscheiden, daß R¹ für Trifluormethyl und R¹⁵ für Methyl stehen.

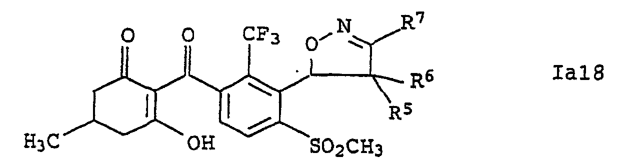

Ebenso insbesondere bevorzugt sind die Verbindungen Ia19., besonders die Verbindungen Ia19.1 - Ia19.64, die sich von den Verbindungen Ia1.1 - Ia1.64 dadurch unterscheiden, daß R¹ für Trifluormethyl und R¹⁵ und R¹⁶ für Methyl stehen.

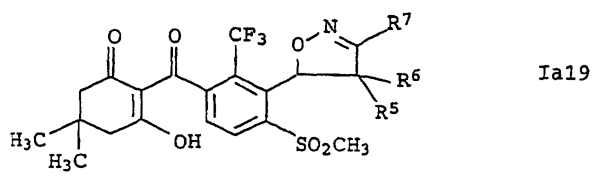

Ebenso insbesondere bevorzugt sind die Verbindungen Ia20., besonders die Verbindungen Ia20.1 - Ia20.64, die sich von den Verbindungen Ia1.1 - Ia1.64 dadurch unterscheiden, daß R¹ für Trifluormethyl und R¹³ und R¹⁷ für Methyl stehen.

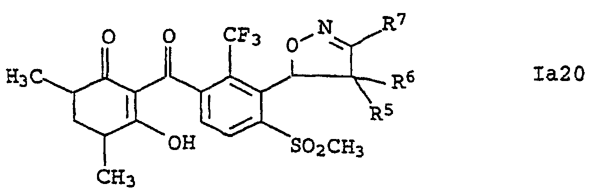

Ebenso insbesondere bevorzugt sind die Verbindungen Ia21., besonders die Verbindungen Ia21.1 - Ia21.64, die sich von den Verbindungen Ia1.1 - Ia1.64 dadurch unterscheiden, daß R¹ für Trifluormethyl, R¹³ für Methylthio und R¹⁴ für Methyl stehen.

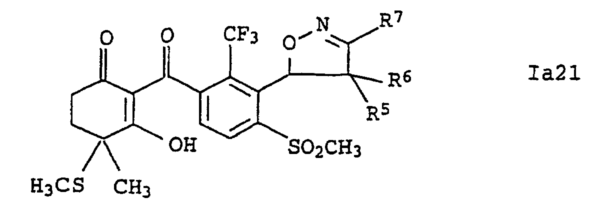

Ebenso insbesondere bevorzugt sind die Verbindungen Ia22., besonders die Verbindungen Ia22.1 - Ia22.64, die sich von den Verbindungen Ia1.1 - Ia1.64 dadurch unterscheiden, daß R¹ für Trifluormethyl, R¹⁴ und R¹⁸ gemeinsam für Ethan-1,2-diyl stehen.

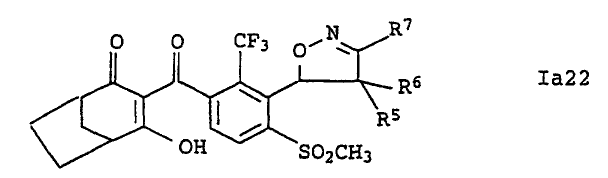

Ebenso insbesondere bevorzugt sind die Verbindungen Ia23., besonders die Verbindungen Ia23.1 - Ia23.64, die sich von den Verbindungen Ia1.1 - Ia1.64 dadurch unterscheiden, daß R¹ für Trifluormethyl, R¹³, R¹⁴, R¹⁷ und R¹⁸ für Methyl und R¹⁶ für Sauerstoff stehen.

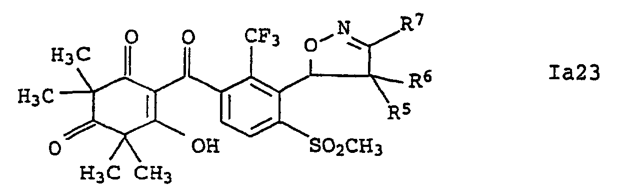

Ebenso insbesondere bevorzugt sind die Verbindungen Ia24., besonders die Verbindungen Ia24.1 - Ia24.64, die sich von den Verbindungen Ia1.1 - Ia1.64 dadurch unterscheiden, daß R¹ für Trifluormethyl und R¹⁵ für Hydroxy stehen.

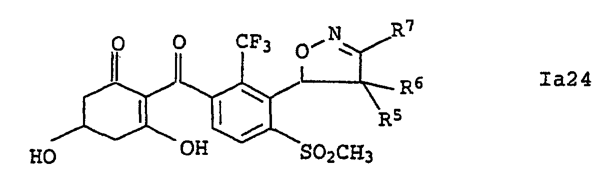

Ebenso insbesondere bevorzugt sind die Verbindungen Ia25., besonders die Verbindungen Ia25.1 - Ia25.64, die sich von den Verbindungen Ia1.1 - Ia1.64 dadurch unterscheiden, daß R¹ für Methoxy steht.

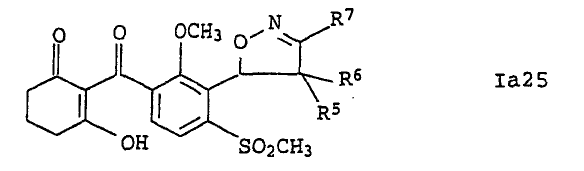

Ebenso insbesondere bevorzugt sind die Verbindungen Ia26., besonders die Verbindungen Ia26.1 - Ia26.64, die sich von den Verbindungen Ia1.1 - Ia1.64 dadurch unterscheiden, daß R¹ für Methoxy und R¹⁵ für Methyl stehen.

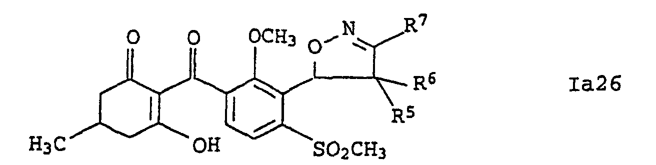

Ebenso insbesondere bevorzugt sind die Verbindungen Ia27., besonders die Verbindungen Ia27.1 - Ia27.64, die sich von den Verbindungen Ia1.1 - Ia1.64 dadurch unterscheiden, daß R¹ für Methoxy und R¹⁵ und R¹⁶ für Methyl stehen.

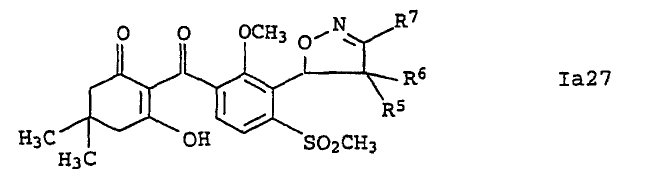

Ebenso insbesondere bevorzugt sind die Verbindungen Ia28., besonders die Verbindungen Ia28.1 - Ia28.64, die sich von den Verbindungen Ia1.1 - Ia1.64 dadurch unterscheiden, daß R¹ für Methoxy und R¹³ und R¹⁷ für Methyl stehen.

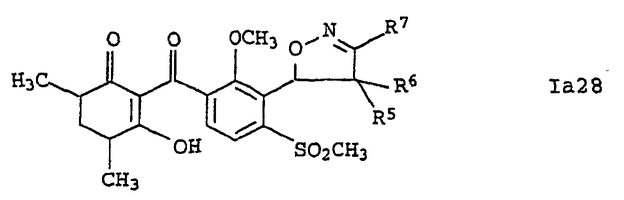

Ebenso insbesondere bevorzugt sind die Verbindungen Ia29., besonders die Verbindungen Ia29.1 - Ia29.64, die sich von den Verbindungen Ia1.1 - Ia1.64 dadurch unterscheiden, daß R¹ für Methoxy, R¹³ für Methylthio und R¹⁴ für Methyl stehen.

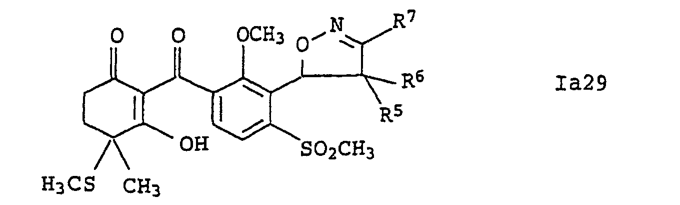

Ebenso insbesondere bevorzugt sind die Verbindungen Ia30., besonders die Verbindungen Ia30.1 - Ia30.64, die sich von den Verbindungen Ia1.1 - Ia1.64 dadurch unterscheiden, daß R¹ für Methoxy, R¹⁴ und R¹⁸ gemeinsam für Ethan-1,2-diyl stehen.

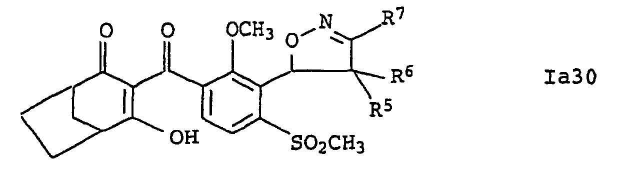

Ebenso insbesondere bevorzugt sind die Verbindungen Ia31., besonders die Verbindungen Ia31.1 - Ia31.64, die sich von den Verbindungen Ia1.1 - Ia1.64 dadurch unterscheiden, daß R¹ für Methoxy, R¹³, R¹⁴, R¹⁷ und R¹⁸ für Methyl und R¹⁵ und R¹⁶ gemeinsam für Sauerstoff stehen.

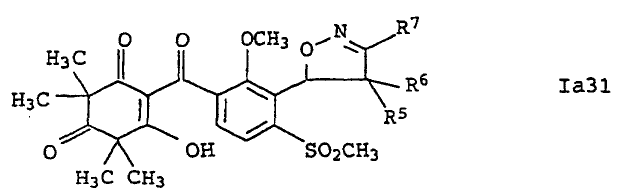

Ebenso insbesondere bevorzugt sind die Verbindungen Ia32., besonders die Verbindungen Ia32.1 - Ia32.64, die sich von den Verbindungen Ia1.1 - Ia1.64 dadurch unterscheiden, daß R¹ für Methoxy R¹⁵ für Hydroxy stehen.

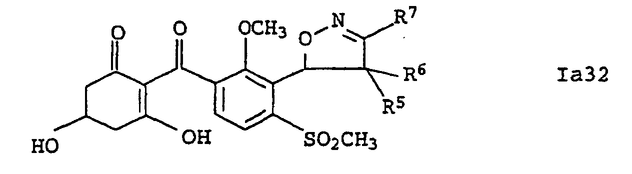

Ebenso insbesondere bevorzugt sind die Verbindungen Ia33., besonders die Verbindungen Ia33.1 - Ia33.64, die sich von den Verbindungen Ia1.1 - Ia1.64 dadurch unterscheiden, daß R² für Ethylsulfonyl steht.

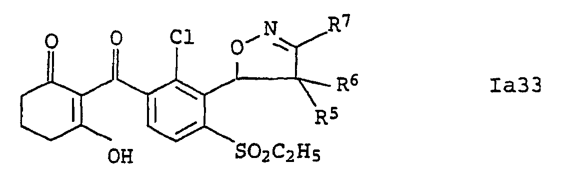

Ebenso insbesondere bevorzugt sind die Verbindungen Ia34., besonders die Verbindungen Ia34.1 - Ia34.64, die sich von den Verbindungen Ia1.1 - Ia1.64 dadurch unterscheiden, daß R² für Ethylsulfonyl und R¹⁵ für Methyl stehen.

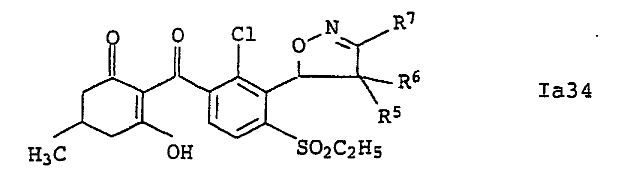

Ebenso insbesondere bevorzugt sind die Verbindungen Ia35., besonders die Verbindungen Ia35.1 - Ia35.64, die sich von den Verbindungen Ia1.1 - Ia1.64 dadurch unterscheiden, daß R² für Ethylsulfonyl und R¹⁵ und R¹⁶ für Methyl stehen.

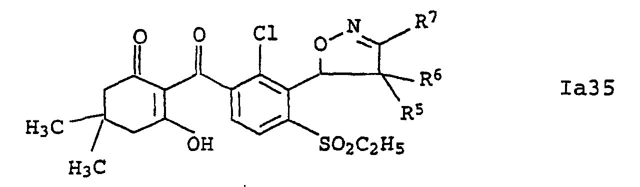

Ebenso insbesondere bevorzugt sind die Verbindungen Ia36., besonders die Verbindungen Ia36.1 - Ia36.64, die sich von den Verbindungen Ia1.1 - Ia1.64 dadurch unterscheiden, daß R² für Ethylsulfonyl und R¹³ und R¹⁷ für Methyl stehen.

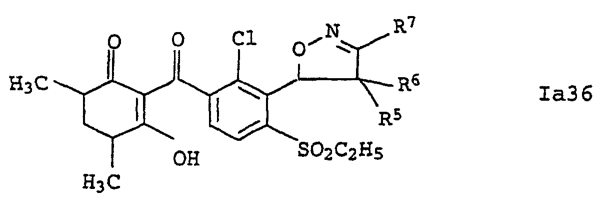

Ebenso insbesondere bevorzugt sind die Verbindungen Ia37., besonders die Verbindungen Ia37.1 - Ia37.64, die sich von den Verbindungen Ia1.1 - Ia1.64 dadurch unterscheiden, daß R² für Ethylsulfonyl, R¹³ für Methylthio und R¹⁴ für Methyl stehen.

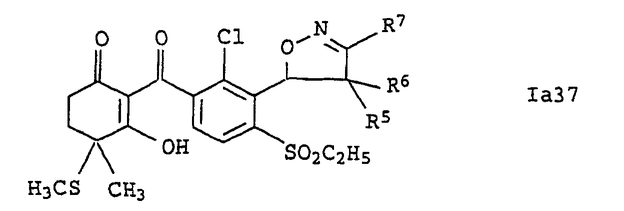

Ebenso insbesondere bevorzugt sind die Verbindungen Ia38., besonders die Verbindungen Ia38.1 - Ia38.64, die sich von den Verbindungen Ia1.1 - Ia1.64 dadurch unterscheiden, daß R² für Ethylsulfonyl, R¹⁴ und R¹⁸ gemeinsam für Ethan-1,2-diyl stehen.

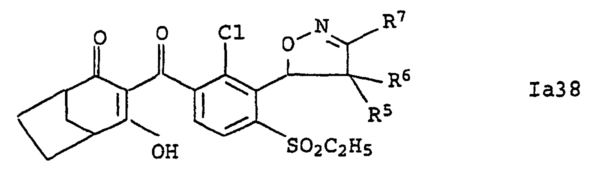

Ebenso insbesondere bevorzugt sind die Verbindungen Ia39., besonders die Verbindungen Ia39.1 - Ia39.64, die sich von den Verbindungen Ia1.1 - Ia1.64 dadurch unterscheiden, daß R² für Ethylsulfonyl, R^{13,} R¹⁴, R¹⁷ und R¹⁸ für Methyl und R¹⁵ und R¹⁶ gemeinsam für Sauerstoff stehen.

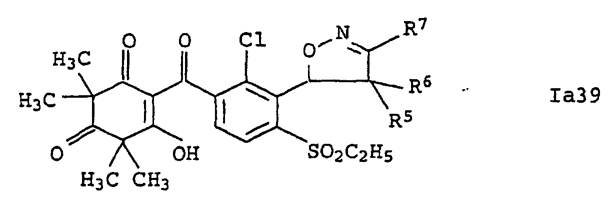

Ebenso insbesondere bevorzugt sind die Verbindungen Ia40., besonders die Verbindungen Ia40.2 - Ia40.64, die sich von den Verbindungen Ia1.1 - Ia1.64 dadurch unterscheiden, daß R² für Ethylsulfonyl, R¹⁵ für Hydroxy steht.

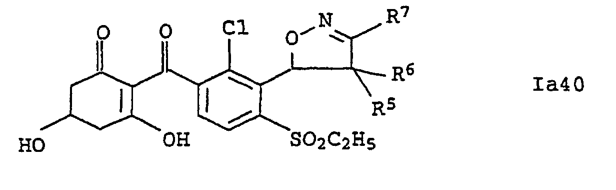

Ebenso insbesondere bevorzugt sind die Verbindungen Ia41., besonders die Verbindungen Ia41.1 - Ia41.64, die sich von den Verbindungen Ia1.1 - Ia1.64 dadurch unterscheiden, daß R¹ für Methyl und R² für Ethylsulfonyl stehen.

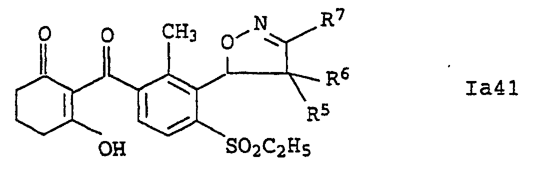

Ebenso insbesondere bevorzugt sind die Verbindungen Ia42., besonders die Verbindungen Ia42.1 - Ia42.64, die sich von den Verbindungen Ia1.1 - Ia1.64 dadurch unterscheiden, daß R¹ und R¹⁵ für Methyl und R² für Ethylsulfonyl stehen.

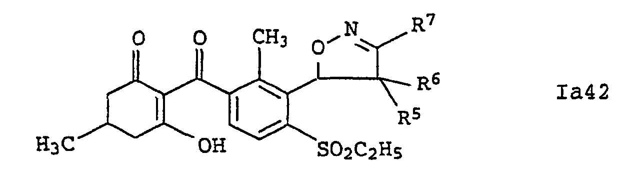

Ebenso insbesondere bevorzugt sind die Verbindungen Ia43., besonders die Verbindungen Ia43.1 - Ia43.64, die sich von den Verbindungen Ia1.1 - Ia1.64 dadurch unterscheiden, daß R¹, R¹⁵ und R¹⁶ für Methyl und R² für Ethylsulfonyl stehen.

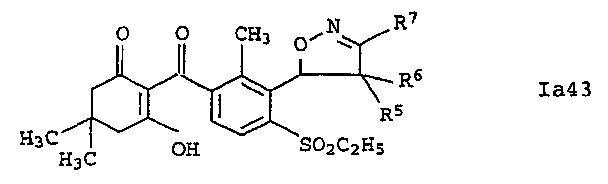

Ebenso insbesondere bevorzugt sind die Verbindungen Ia44., besonders die Verbindungen Ia44.1 - Ia44.64, die sich von den Verbindungen Ia1.1 - Ia1.64 dadurch unterscheiden, daß R¹, R¹³ und R¹⁷ für Methyl und R² für Ethylsulfonyl stehen.

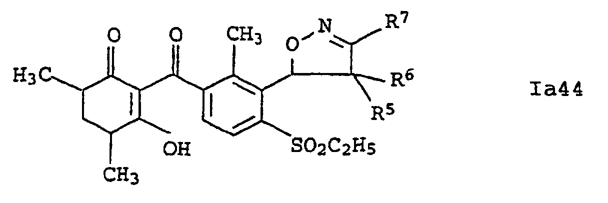

Ebenso insbesondere bevorzugt sind die Verbindungen Ia45., besonders die Verbindungen Ia45.1 - Ia45.64, die sich von den verbindungen Ia1.1 - Ia1.64 dadurch unterscheiden, daß R¹ und R¹⁴ für Methyl und R¹³ für Methylthio stehen.

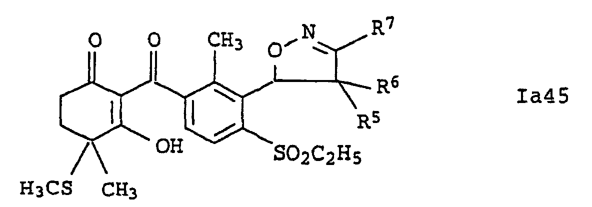

Ebenso insbesondere bevorzugt sind die Verbindungen Ia46., besonders die Verbindungen Ia46.1 - Ia46.64, die sich von den Verbindungen Ia1.1 - Ia1.64 dadurch unterscheiden, daß R¹ für Methyl, R² für Ethylsulfonyl und R¹⁴ und R¹⁸ gemeinsam für Ethan-1,2-diyl stehen.

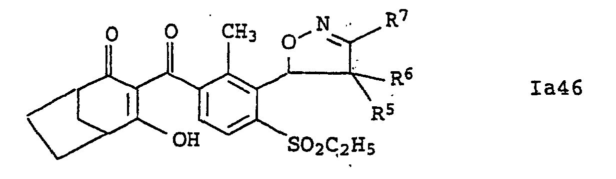

Ebenso insbesondere bevorzugt sind die Verbindungen Ia47., besonders die Verbindungen Ia47.1 - Ia47.64, die sich von den Verbindungen Ia1.1 - Ia1.64 dadurch unterscheiden, daß R¹, R¹³, R¹⁴, R¹⁷ und R¹⁸ für Methyl R² für Ethylsulfonyl und R¹⁵ und R¹⁶ gemeinsam für Sauerstoff stehen.

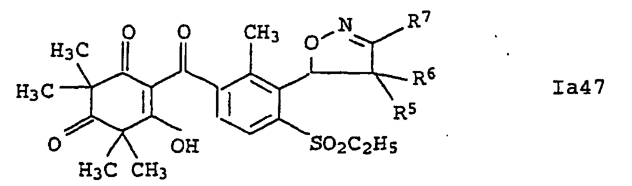

Ebenso insbesondere bevorzugt sind die Verbindungen Ia48., besonders die Verbindungen Ia48.1 - Ia48.64, die sich von den verbindungen Ia1.1 - Ia1.64 dadurch unterscheiden, daß R¹ für Methyl, R² für Ethylsulfonyl und R¹⁵ für Hydroxy stehen.

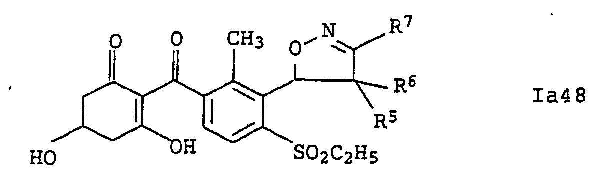

Ebenso insbesondere bevorzugt sind die Verbindungen Ia49., besonders die Verbindungen Ia49.1 - Ia49.64, die sich von den Verbindungen Ia1.1 - Ia1.64 dadurch unterscheiden, daß R¹ für Trifluormethyl und R² für Ethylsulfonyl stehen.

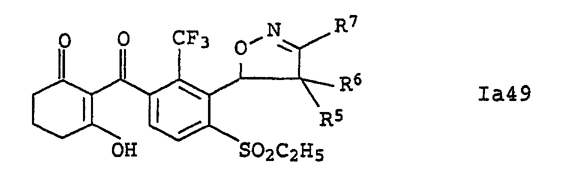

Ebenso insbesondere bevorzugt sind die Verbindungen Ia50., besonders die Verbindungen Ia50.1 - Ia50.64, die sich von den Verbindungen Ia1.1 - Ia1.64 dadurch unterscheiden, daß R¹ für Trifluormethyl, R² für Ethylsulfonyl und R¹⁵ für Methyl stehen.

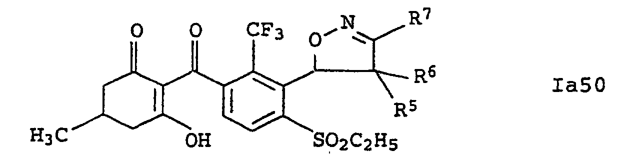

Ebenso insbesondere bevorzugt sind die Verbindungen Ia51., besonders die Verbindungen Ia51.1 - Ia51.64, die sich von den Verbindungen Ia1.1 - Ia1.64 dadurch unterscheiden, daß R¹ für Trifluormethyl, R² für Ethylsulfonyl und R¹⁵ und R¹⁶ für Methyl stehen.

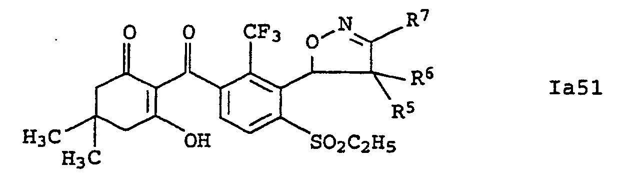

Ebenso insbesondere bevorzugt sind die Verbindungen Ia52., besonders die Verbindungen Ia52.1 - Ia52.64, die sich von den Verbindungen Ia1.1 - Ia1.64 dadurch unterscheiden, daß R¹ für Trifluormethyl, R² für Ethylsulfonyl und R¹³ und R¹⁷ für Methyl stehen.

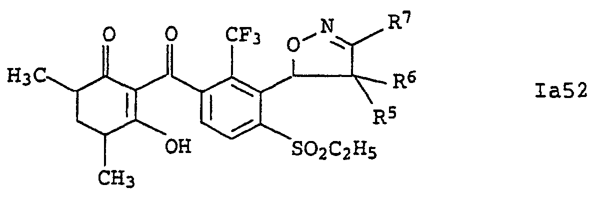

Ebenso insbesondere bevorzugt sind die Verbindungen Ia53., besonders die Verbindungen Ia53.1 - Ia53.64, die sich von den Verbindungen Ia1.1 - Ia1.64 dadurch unterscheiden, daß R¹ für Trifluormethyl, R² für Ethylsulfonyl, R¹³ für Methylthio und R¹⁴ für Methyl stehen.

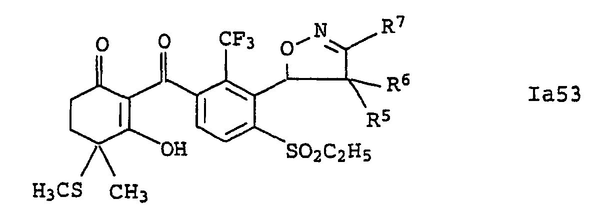

Ebenso insbesondere bevorzugt sind die Verbindungen Ia54., besonders die Verbindungen Ia54.1 - Ia54.64, die sich von den Verbindungen Ia1.1 - Ia1.64 dadurch unterscheiden, daß R¹ für Trifluormethyl, R² für Ethylsulfonyl, R¹⁴ und R¹⁸ gemeinsam für Ethan-1,2-diyl stehen.

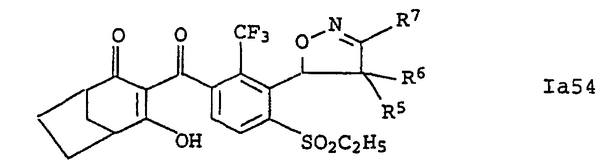

Ebenso insbesondere bevorzugt sind die Verbindungen Ia55., besonders die Verbindungen Ia55.1 - Ia55.64, die sich von den Verbindungen Ia1.1 - Ia1.64 dadurch unterscheiden, daß R¹ für Trifluormethyl, R² für Ethylsulfonyl, R¹³, R¹⁴, R¹⁷ und R¹⁸ Methyl und R¹⁵ und R¹⁶ gemeinsam für Sauerstoff stehen.

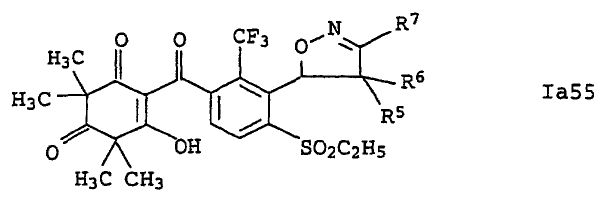

Ebenso insbesondere bevorzugt sind die Verbindungen Ia56., besonders die Verbindungen Ia56.1 - Ia56.64, die sich von den Verbindungen Ia1.1 - Ia1.64 dadurch unterscheiden, daß R¹ für Trifluormethyl, R² für Ethylsulfonyl und R¹⁵ für Hydroxy stehen.

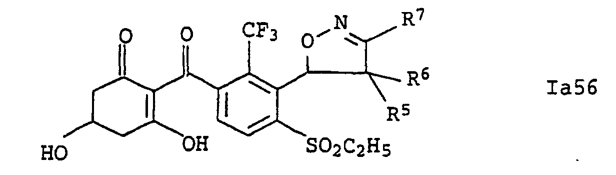

Ebenso insbesondere bevorzugt sind die Verbindungen Ia57., besonders die Verbindungen Ia57.1 - Ia57.64, die sich von den Verbindungen Ia1.1 - Ia1.64 dadurch unterscheiden, daß R¹ für Methoxy und R² für Ethylsulfonyl stehen.

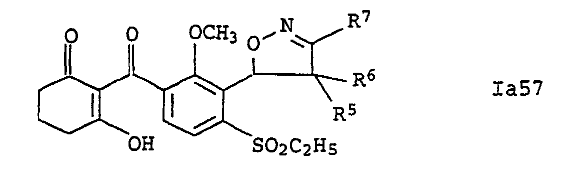

Ebenso insbesondere bevorzugt sind die Verbindungen Ia58., besonders die Verbindungen Ia58.1 - Ia58.64, die sich von den Verbindungen Ia1.1 - Ia1.64 dadurch unterscheiden, daß R¹ für Methoxy, R² für Ethylsulfonyl und R¹⁵ für Methyl stehen.

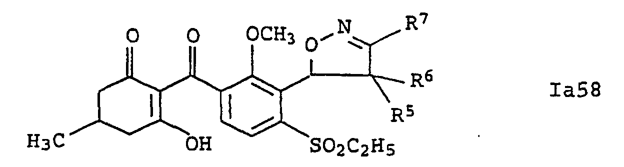

Ebenso insbesondere bevorzugt sind die Verbindungen Ia59., besonders die Verbindungen Ia59.1 - Ia59.64, die sich von den Verbindungen Ia1.1 - Ia1.64 dadurch unterscheiden, daß R¹ für Methoxy, R² für Ethylsulfonyl und R¹⁵ und R¹⁶ für Methyl stehen.

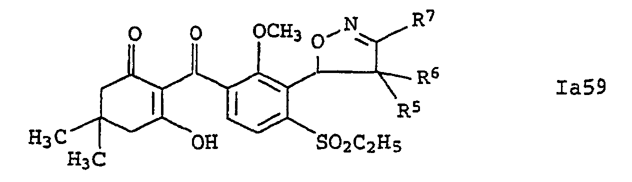

Ebenso insbesondere bevorzugt sind die Verbindungen Ia60., besonders die Verbindungen Ia60.1 - Ia60.64, die sich von den Verbindungen Ia1.1 - Ia1.64 dadurch unterscheiden, daß R¹ für Methoxy, R² für Ethylsulfonyl und R¹³ und R¹⁷ für Methyl stehen.

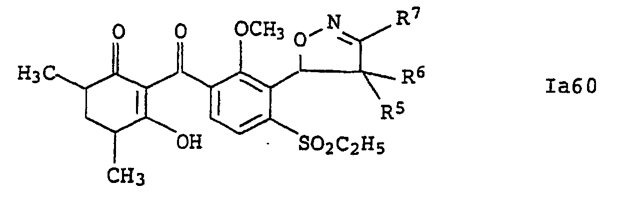

Ebenso insbesondere bevorzugt sind die Verbindungen Ia61., besonders die Verbindungen Ia61.1 - Ia61.64, die sich von den Verbindungen Ia1.1 - Ia1.64 dadurch unterscheiden, daß R¹ für Methoxy, R² für Ethylsulfonyl, R¹³ für Methylthio und R¹⁴ für Methyl stehen.

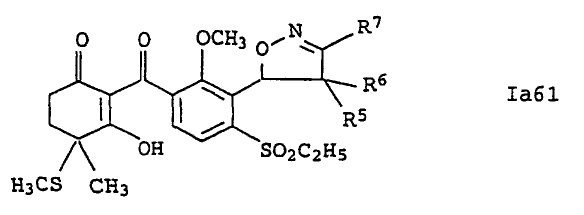

Ebenso insbesondere bevorzugt sind die Verbindungen Ia62., besonders die Verbindungen Ia62.1 - Ia62.64, die sich von den Verbindungen Ia1.1 - Ia1.64 dadurch unterscheiden, daß R¹ für Methoxy, R² für Ethylsulfonyl, R¹⁴, R¹⁸ gemeinsam für Ethan-1,2-diyl stehen.

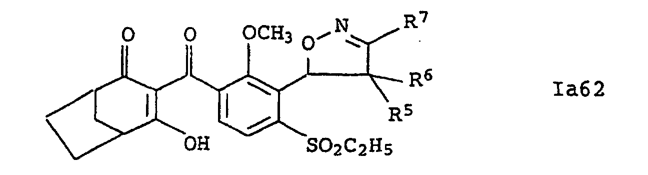

Ebenso insbesondere bevorzugt sind die Verbindungen Ia63., besonders die Verbindungen Ia63.1 - Ia63.64, die sich von den Verbindungen Ia1.1 - Ia1.64 dadurch unterscheiden, daß R¹ für Methoxy, R² für Ethylsulfonyl, R¹³, R¹⁴, R¹⁷ und R¹⁸ für Methyl und R¹⁵ und R¹⁶ gemeinsam für Sauerstoff stehen.

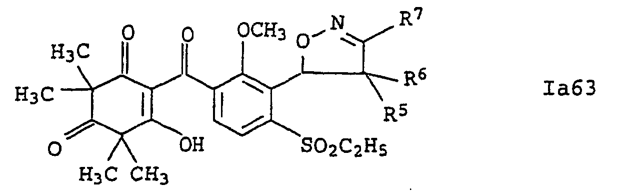

Ebenso insbesondere bevorzugt sind die Verbindungen Ia64., besonders die Verbindungen Ia64.1 - Ia64.64, die sich von den Verbindungen Ta1.1 - Ia1.64 dadurch unterscheiden, daß R¹ für Methoxy, R² für Ethylsulfonyl und R¹⁵ für Hydroxy stehen.

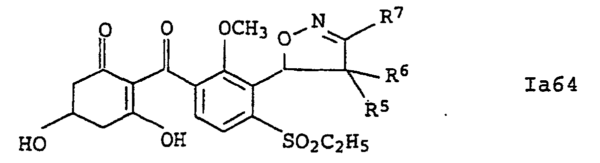

Ebenso insbesondere bevorzugt sind die Verbindungen Ia65., besonders die Verbindungen Ia65.1 - Ia65.64, die sich von den Verbindungen Ia1.1 - Ia1.64 dadurch unterscheiden, daß R² für Chlor steht.

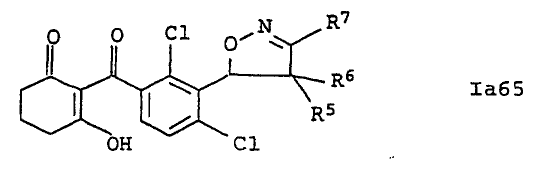

Ebenso insbesondere bevorzugt sind die Verbindungen Ia66., besonders die Verbindungen Ia66.1 - Ia66.64, die sich von den Verbindungen Ia1.1 - Ia1.64 dadurch unterscheiden, daß R² für Chlor und R¹⁵ für Methyl stehen.

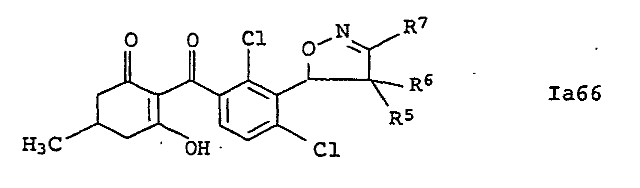

Ebenso insbesondere bevorzugt sind die Verbindungen Ia67., besonders die Verbindungen Ia67.1 - Ia67.64, die sich von den Verbindungen Ia1.1 - Ia1.64 dadurch unterscheiden, daß R² für Chlor und R¹⁵ und R¹⁶ für Methyl stehen.

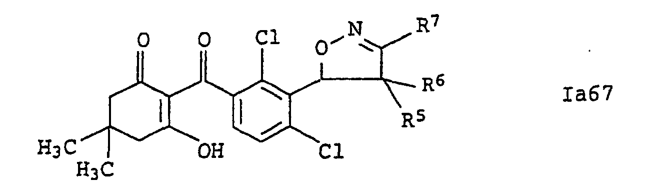

Ebenso insbesondere bevorzugt sind die Verbindungen Ia68., besonders die Verbindungen Ia68.1 - Ia68.64, die sich von den Verbindungen Ia1.1 - Ia1.64 dadurch unterscheiden, daß R² für Chlor und R¹³ und R¹⁷ für Methyl stehen.

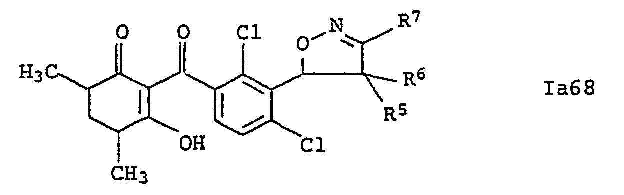

Ebenso insbesondere bevorzugt sind die Verbindungen Ia69., besonders die Verbindungen Ia69.1 - Ia69.64, die sich von den Verbindungen Ia1.1 - Ia1.64 dadurch unterscheiden, daß R² für Chlor, R¹³ für Methylthio und R¹⁴ für Methyl stehen.

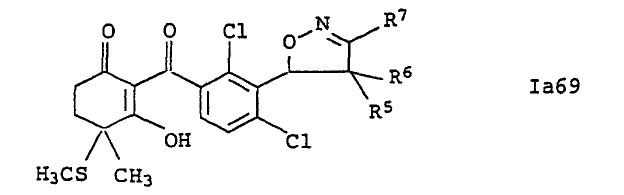

Ebenso insbesondere bevorzugt sind die Verbindungen Ia70., besonders die Verbindungen Ia70.1 - Ia70.64, die sich von den Verbindungen Ia1.1 - Ia1.64 dadurch unterscheiden, daß R² für Chlor und R¹⁴ und R¹⁸ gemeinsam für Ethan-1,2-diyl stehen.

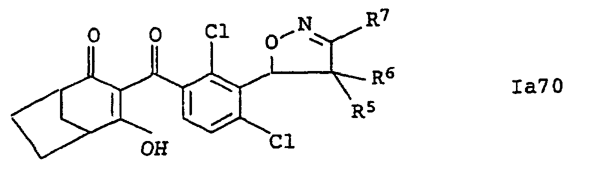

Ebenso insbesondere bevorzugt sind die Verbindungen Ia71., besonders die Verbindungen Ia71.1 - Ia71.64, die sich von den Verbindungen Ia1.1 - Ia1.64 dadurch unterscheiden, daß R² für Chlor, R¹³, R¹⁴, R¹⁷ und R¹⁸ für Methyl und R¹⁵ und R¹⁶ gemeinsam für Sauerstoff stehen.

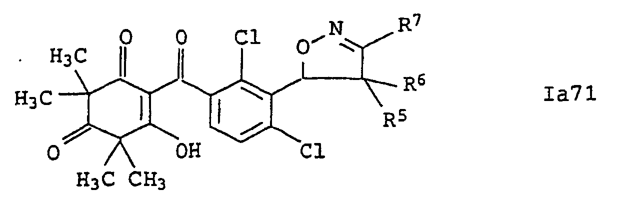

Ebenso insbesondere bevorzugt sind die Verbindungen Ia72., besonders die Verbindungen Ia72.1 - Ia72.64, die sich von den Verbindungen Ia1.1 - Ia1.64 dadurch unterscheiden, daß R² für Chlor und R¹⁵ für Hydroxy stehen.

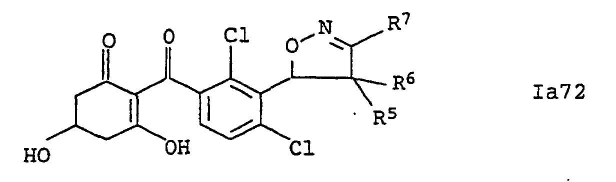

Ebenso insbesondere bevorzugt sind die Verbindungen Ia73., besonders die Verbindungen Ia73.1 - Ia73.64, die sich von den Verbindungen Ia1.1 - Ia1.64 dadurch unterscheiden, daß R¹ für Methyl und R² für Chlor stehen.

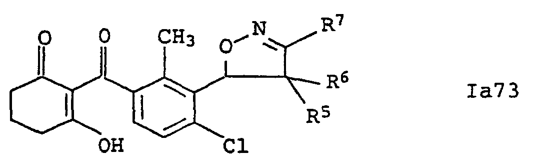

Ebenso insbesondere bevorzugt sind die Verbindungen Ia74., besonders die Verbindungen Ia74.1 - Ia74.64, die sich von den Verbindungen Ia1.1 - Ia1.64 dadurch unterscheiden, daß R¹ und R¹⁵ für Methyl und R² für Chlor stehen.

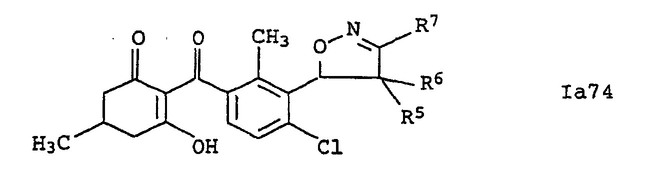

Ebenso insbesondere bevorzugt sind die Verbindungen Ia75., besonders die Verbindungen Ia75.1 - Ia75.64, die sich von den Verbindungen Ia1.1 - Ia1.64 dadurch unterscheiden, daß R¹, R¹⁵ und R¹⁸ für Methyl und R² für Chlor stehen.

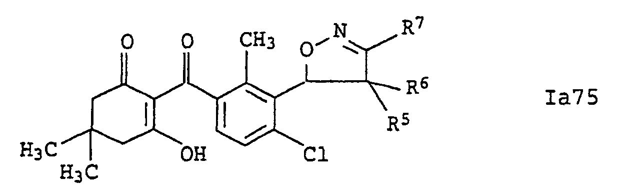

Ebenso insbesondere bevorzugt sind die Verbindungen Ia76., besonders die Verbindungen Ia76.1 - Ia76.64, die sich von den Verbindungen Ia1.1 - Ia1.64 dadurch unterscheiden, daß R¹, R¹³ und R¹⁷ für Methyl und R² für Chlor stehen.

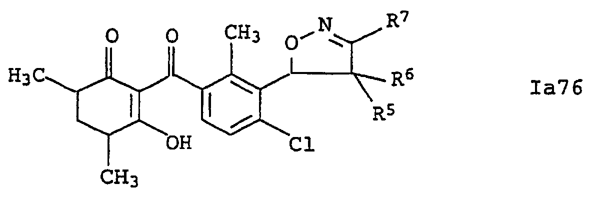

Ebenso insbesondere bevorzugt sind die Verbindungen Ia77., besonders die Verbindungen Ia77.1 - Ia77.64, die sich von den Verbindungen Ia1.1 - Ia1.64 dadurch unterscheiden, daß R¹ und R¹⁴ für Methyl, R² für Chlor und R¹³ für Methylthio stehen.

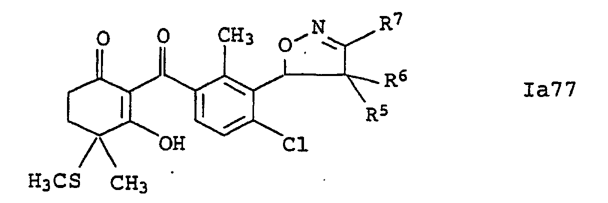

Ebenso insbesondere bevorzugt sind die Verbindungen Ia78., besonders die Verbindungen Ia78.1 - Ia78.64, die sich von den Verbindungen Ia1.1 - Ia1.64 dadurch unterscheiden, daß R¹ für Methyl, R² für Chlor und R¹⁴ und R¹⁸ gemeinsam für Ehtan-1,2-diyl stehen.

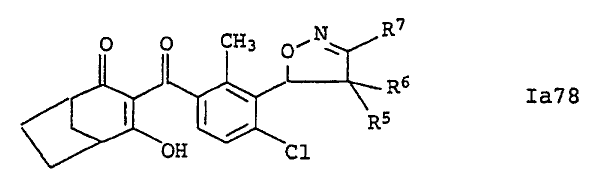

Ebenso insbesondere bevorzugt sind die Verbindungen Ia79., besonders die Verbindungen Ia79.1 - Ia79.64, die sich von den Verbindungen Ia1.1 - Ia1.64 dadurch unterscheiden, daß R¹, R¹³, R¹⁴, R¹⁷ und R¹⁸ für Methyl, R² für Chlor und R¹⁵ und R¹⁶ gemeinsam für Sauerstoff stehen.

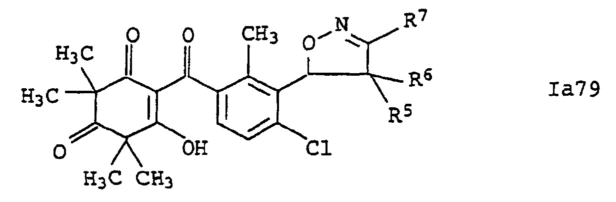

Ebenso insbesondere bevorzugt sind die Verbindungen Ia80., besonders die Verbindungen Ia80.1 - Ia80.64, die sich von den Verbindungen Ia1.1 - Ia1.64 dadurch unterscheiden, daß R¹ für Methyl, R² für Chlor und R¹⁵ für Hydroxy stehen.

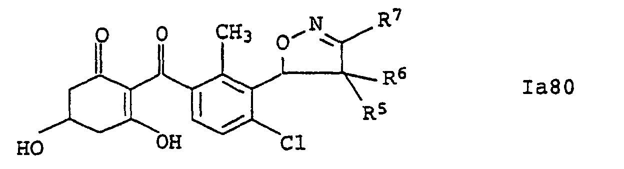

Ebenso insbesondere bevorzugt sind die Verbindungen Ia81., besonders die Verbindungen Ia81.1 - Ia81.64, die sich von den Verbindungen Ia1.1 - Ia1.64 dadurch unterscheiden, daß R¹ für Trifluormethyl und R² für Chlor stehen.

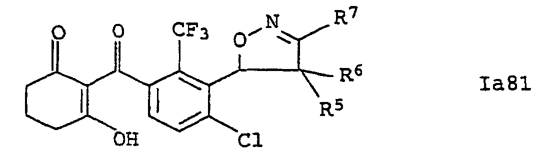

Ebenso insbesondere bevorzugt sind die Verbindungen Ia82., besonders die Verbindungen Ia82.1 - Ia82.64, die sich von den Verbindungen Ia1.1 - Ia1.64 dadurch unterscheiden, daß R¹ für Trifluormethyl, R² für Chlor und R¹⁵ für Methyl stehen.

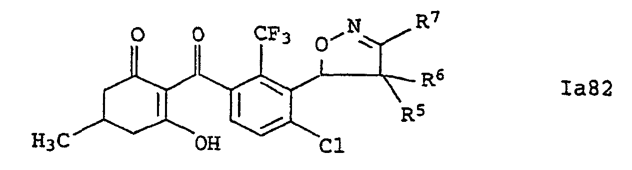

Ebenso insbesondere bevorzugt sind die Verbindungen Ia83., besonders die Verbindungen Ia83.1 - Ia83.64, die sich von den Verbindungen Ia1.1 - Ia1.64 dadurch unterscheiden, daß R¹ für Trifluormethyl, R² für Chlor und R¹⁵ und R¹⁶ für Methyl stehen.

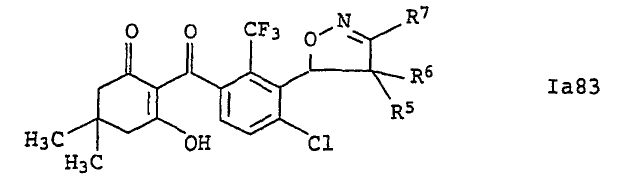

Ebenso insbesondere bevorzugt sind die Verbindungen Ia84., besonders die Verbindungen Ia84.1 - Ia84.64, die sich von den verbindungen Ia1.1 - Ia1.64 dadurch unterscheiden, daß R¹ für Trifluormethyl, R² Chlor und R¹³ und R¹⁷ Methyl stehen.

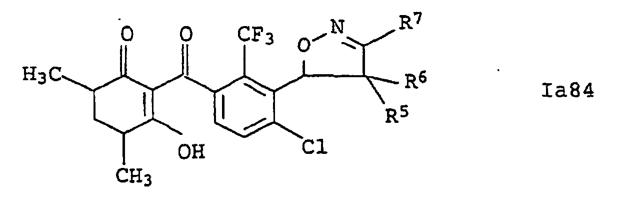

Ebenso insbesondere bevorzugt sind die Verbindungen Ia85., besonders die Verbindungen Ia85.1 - Ia85.64, die sich von den Verbindungen Ia1.1 - Ia1.64 dadurch unterscheiden, daß R¹ für Trifluormethyl, R² für Chlor, R¹³ für Methylthio und R¹⁴ für Methyl stehen.

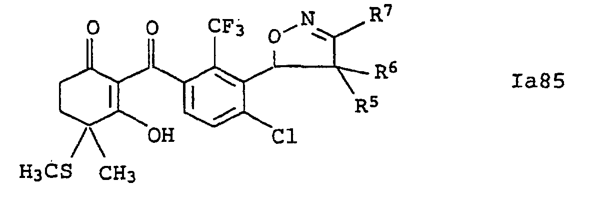

Ebenso insbesondere bevorzugt sind die Verbindungen Ia86., besonders die Verbindungen Ia86.1 - Ia86.64, die sich von den Verbindungen Ia1.1 - Ia1.64 dadurch unterscheiden, daß R¹ für Trifluormethyl, R² für Chlor und R¹⁴ und R¹⁸ gemeinsam für Ethan-1,2-diyl stehen.

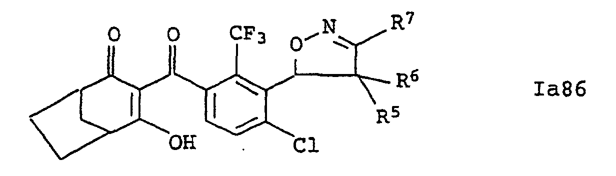

Ebenso insbesondere bevorzugt sind die Verbindungen Ia87., besonders die Verbindungen Ia87.1 - Ia87.64, die sich von den Verbindungen Ia1.1 - Ia1.64 dadurch unterscheiden, daß R¹ für Trifluormethyl, R² für Chlor, R¹³, R¹⁴, R¹⁷ und R¹⁸ für Methyl und R¹⁵ und R¹⁶ gemeinsam für Sauerstoff stehen.

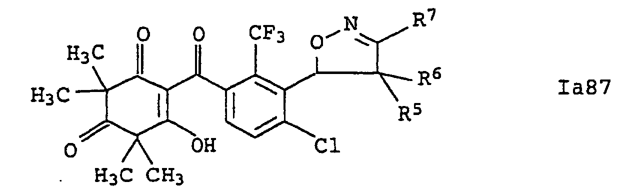

Ebenso insbesondere bevorzugt sind die Verbindungen Ia88., besonders die Verbindungen Ia88.1 - Ia88.64, die.sich von den Verbindungen Ia1.1 - Ia1.64 dadurch unterscheiden, daß R¹ für Trifluormethyl, R² für Chlor und R¹⁵ für Hydroxy stehen.

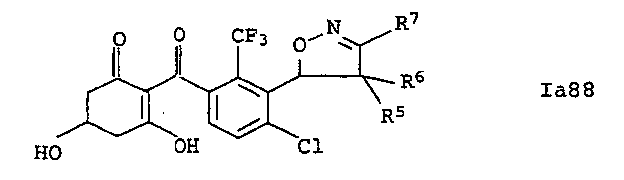

Ebenso insbesondere bevorzugt sind die Verbindungen Ia89., besonders die Verbindungen Ia89.1 - Ia89.64, die sich von den Verbindungen Ia1.1 - Ia1.64 dadurch unterscheiden, daß R¹ für Methoxy und R² für Chlor stehen.

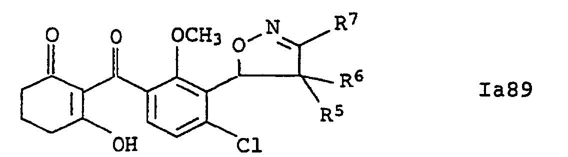

Ebenso insbesondere bevorzugt sind die Verbindungen Ia90., besonders die Verbindungen Ia90.1 - Ia90.64, die sich von den Verbindungen Ia1.1 - Ia1.64 dadurch unterscheiden, daß R¹ für Methoxy, R² für Chlor und R¹⁵ für Methyl stehen.

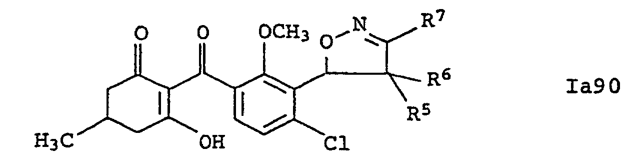

Ebenso insbesondere bevorzugt sind die Verbindungen Ia91., besonders die Verbindungen Ia91.1 - Ia91.64, die sich von den Verbindungen Ia1.1 - Ia1.64 dadurch unterscheiden, daß R¹ für Methoxy, R² für Chlor und R¹⁵ und R¹⁶ für Methyl stehen.

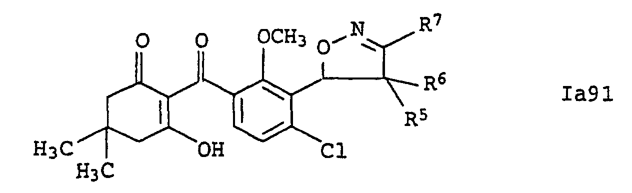

Ebenso insbesondere bevorzugt sind die Verbindungen Ia92., besonders die Verbindungen Ia92.1 - Ia92.64, die sich von den Verbindungen Ia1.1 - Ia1.64 dadurch unterscheiden, daß R¹ für Methoxy, R² für Chlor und R¹³ und R¹⁷ für Methyl stehen.

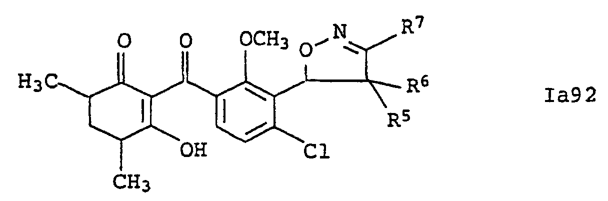

Ebenso insbesondere bevorzugt sind die Verbindungen Ia93., besonders die Verbindungen Ia93.1 - Ia93.64, die sich von den Verbindungen Ia1.1 - Ia1.64 dadurch unterscheiden, daß R¹ für Methoxy, R² für Chlor, R¹³ für Methylthio und R¹⁴ für Methyl stehen.

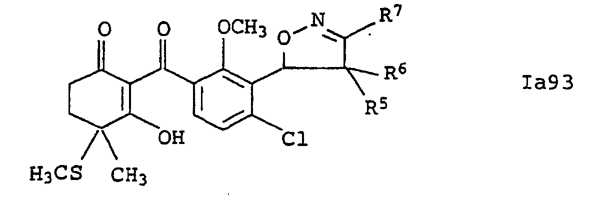

Ebenso insbesondere bevorzugt sind die Verbindungen Ia94., besonders die Verbindungen Ia94.1 - Ia94.64, die sich von den Verbindungen Ia1.1 - Ia1.64 dadurch unterscheiden, daß R¹ für Methoxy, R² für Chlor und R¹⁴ und R¹⁸ gemeinsam für Ethan-1,2-diyl stehen.

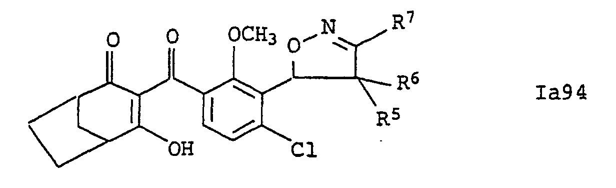

Ebenso insbesondere bevorzugt sind die Verbindungen Ia95., besonders die Verbindungen Ia95.1 - Ia95.64, die sich von den Verbindungen Ia1.1 - Ia1.64 dadurch unterscheiden, daß R¹ für Methoxy, R² für Chlor, R¹³, R¹⁴, R¹² und R¹⁸ für Methyl und R¹⁵ und R¹⁶ gemeinsam für Sauerstoff stehen.

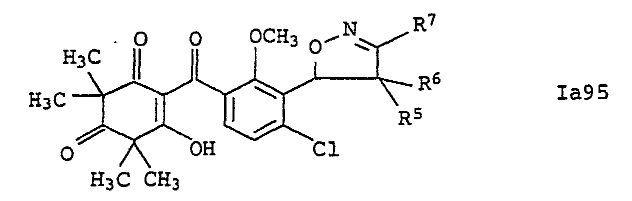

Ebenso insbesondere bevorzugt sind die Verbindungen Ia96., besonders die Verbindungen Ia96.1 - Ia96.64, die sich von den Verbindungen Ia1.1 - Ia1.64 dadurch unterscheiden, daß R¹ für Methoxy, R² für Chlor und R¹⁵ für Hydroxy stehen.

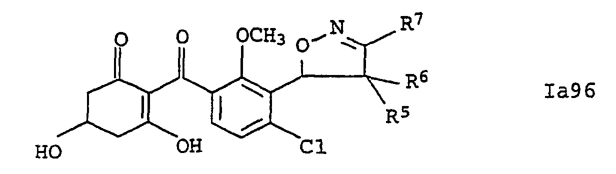

Ebenso insbesondere bevorzugt sind die Verbindungen Ia97., besonders die Verbindungen Ia97.1 - Ia97.64, die sich von den Verbindungen Ia1.1 - Ia1.64 dadurch unterscheiden, daß R² für Trifluormethyl steht.

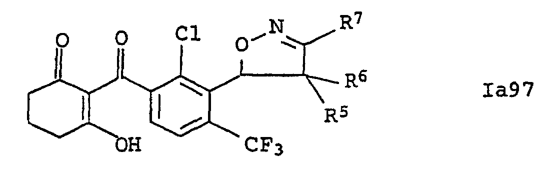

Ebenso insbesondere bevorzugt sind die Verbindungen Ia98., besonders die Verbindungen Ia98.1 - Ia98.64, die sich von den Verbindungen Ia1.1 - Ia1.64 dadurch unterscheiden, daß R² für Trifluormethyl und R¹⁵ für Methyl stehen.

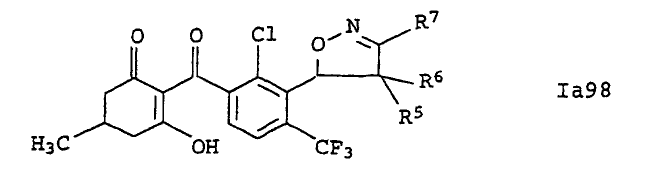

Ebenso insbesondere bevorzugt sind die Verbindungen Ia99., besonders die Verbindungen Ia99.1 - Ia99.64, die sich von den Verbindungen Ia1.1 - Ia1.64 dadurch unterscheiden, daß R² für Trifluormethyl und R¹⁵ und R¹⁶ für Methyl stehen.

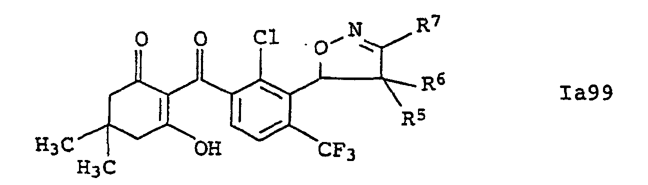

Ebenso insbesondere bevorzugt sind die Verbindungen Ia100., besonders die Verbindungen Ia100.1 - Ia100.64, die sich von den Verbindungen Ia1.1 - Ia1.64 dadurch unterscheiden, daß R² für Trifluormethyl und R¹³ und R¹⁷ für Methyl stehen.

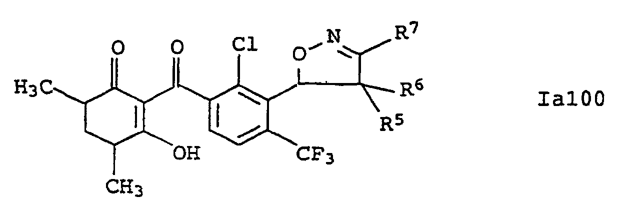

Ebenso insbesondere bevorzugt sind die Verbindungen Ia101., besonders die Verbindungen Ia101.1 - Ia101.64, die sich von den Verbindungen Ia1.1 - Ia1.64 dadurch unterscheiden, daß R² für Trifluormethyl, R¹³ für Methylthio und R¹⁴ für Methyl stehen.

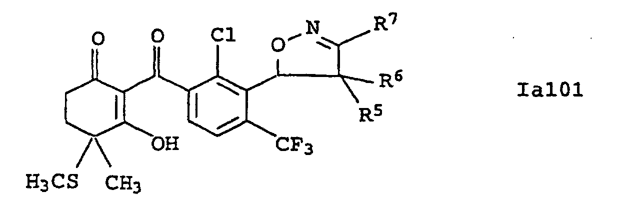

Ebenso insbesondere bevorzugt sind die Verbindungen Ia102., besonders die Verbindungen Ia102.1 - Ia102.64, die sich von den Verbindungen Ia1.1 - Ia1.64 dadurch unterscheiden, daß R² für Trifluormethyl und R¹⁴ und R¹⁸ gemeinsam für Ethan-1,2-diyl stehen.

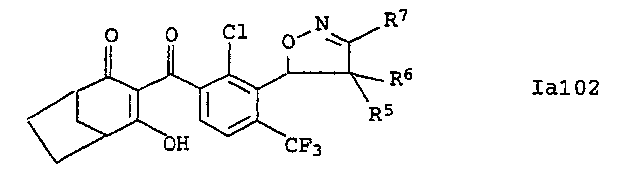

Ebenso insbesondere bevorzugt sind die Verbindungen Ia103., besonders die Verbindungen Ia103.1 - Ia203.64, die sich von den Verbindungen Ia1.1 - Ia1.64 dadurch unterscheiden, daß R² für Trifluormethyl, R¹³, R¹⁴, R¹⁷ und R¹⁸ für Methyl und R¹⁵ und R¹⁶ gemeinsam für Sauerstoff stehen.

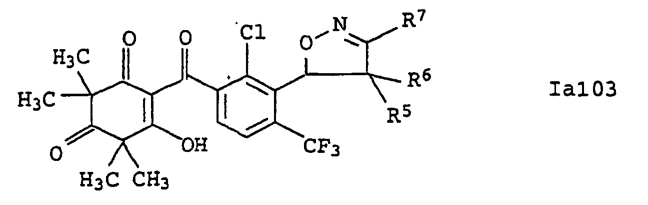

Ebenso insbesondere bevorzugt sind die Verbindungen Ia104., besonders die Verbindungen Ia104.1 - Ia104.64, die sich von den Verbindungen Ia1.1 - Ia1.64 dadurch unterscheiden, daß R² für Trifluormethyl und R¹⁵ für Hydroxy stehen.

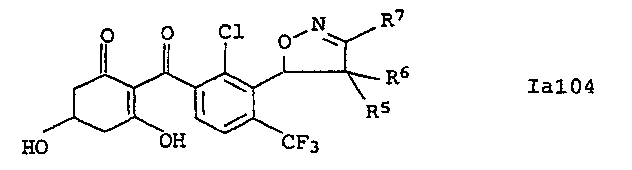

Ebenso insbesondere bevorzugt sind die Verbindungen Ia105., besonders die Verbindungen Ia105.1 - Ia105.64, die sich von den Verbindungen Ia1.1 - Ia1.64 dadurch unterscheiden, daß R¹ für Methyl und R² für Trifluormethyl stehen.

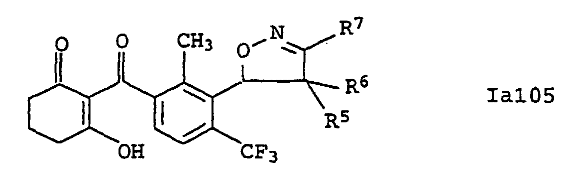

Ebenso insbesondere bevorzugt sind die Verbindungen Ia106., besonders die Verbindungen Ia106.1 - Ia106.64, die sich von den Verbindungen Ia1.1 - Ia1.64 dadurch unterscheiden, daß R¹ und R¹⁵ für Methyl und R² für Trifluormethyl stehen.

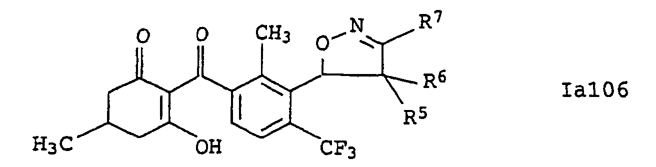

Ebenso insbesondere bevorzugt sind die Verbindungen Ia107., besonders die Verbindungen Ia107.1 - Ia107.64, die sich von den Verbindungen Ia1.1 - Ia1.64 dadurch unterscheiden, daß R¹, R¹⁵ und R¹⁶ für Methyl und R² für Trifluormethyl stehen.

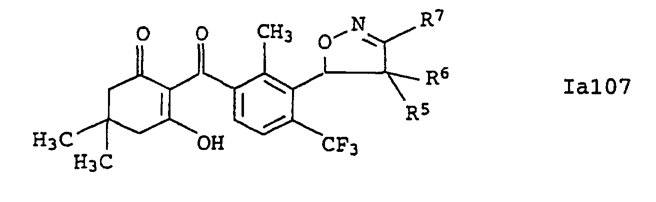

Ebenso insbesondere bevorzugt sind die Verbindungen Ia108., besonders die Verbindungen Ia108.1 - Ia108.64, die sich von den Verbindungen Ia1.1 - Ia1.64 dadurch unterscheiden, daß R¹, R¹³ und R¹⁷ für Methyl und R² für Trifluormethyl stehen.

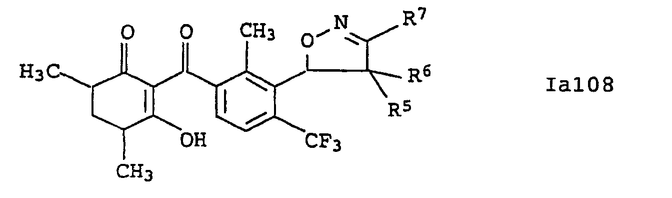

Ebenso insbesondere bevorzugt sind die Verbindungen Ia109., besonders die Verbindungen Ia109.1 - Ia109.64, die sich von den Verbindungen Ia1.1 - Ia1.64 dadurch unterscheiden, daß R¹ und R¹⁴ für Methyl, R² für Trifluormethyl und R¹³ für Methylthio stehen.

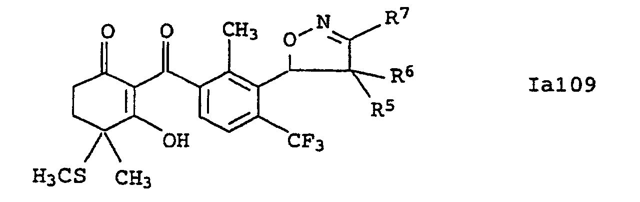

Ebenso insbesondere bevorzugt sind die Verbindungen Ia110., besonders die Verbindungen Ia110.1 - Ia110.64, die sich von den Verbindungen Ia1.1 - Ia1.64 dadurch unterscheiden, daß R¹ für Methyl, R² für Trifluormethyl und R¹⁴ und R¹⁸ gemeinsam für Ethan-1,2-diyl stehen.

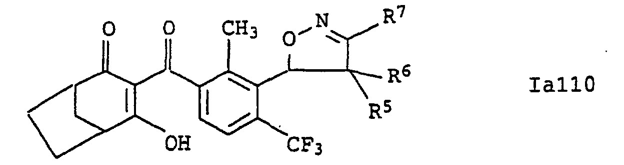

Ebenso insbesondere bevorzugt sind die Verbindungen Ia111., besonders die Verbindungen Ia111.1 - Ia111.64, die sich von den Verbindungen Ia1.1 - Ia1.64 dadurch unterscheiden, daß R¹, R¹³, R¹⁴, R¹⁷ und R¹⁸ für Methyl, R² für Trifluormethyl und R¹⁵ und R¹⁶ gemeinsam für Sauerstoff stehen.

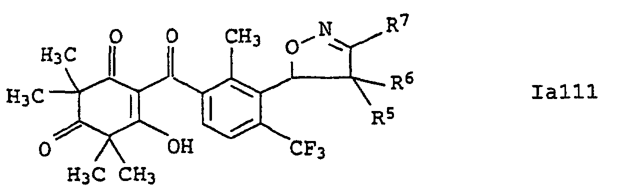

Ebenso insbesondere bevorzugt sind die Verbindungen Ia112., besonders die Verbindungen Ia112.1 - Ia112.64, die sich von den Verbindungen Ia1.1 - Ia1.64 dadurch unterscheiden, daß R¹ für Methyl, R² für Trifluormethyl und R¹⁵ für Hydroxy stehen.

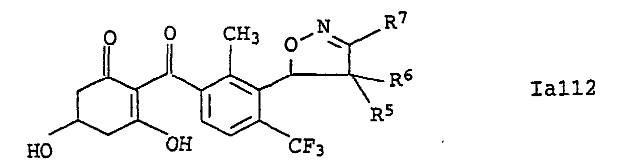

Ebenso insbesondere bevorzugt sind die Verbindungen Ia113., besonders die Verbindungen Ia113.1 - Ia113.64, die sich von den Verbindungen Ia1.1 - Ia1.64 dadurch unterscheiden, daß R¹ und R² für Trifluormethyl stehen.

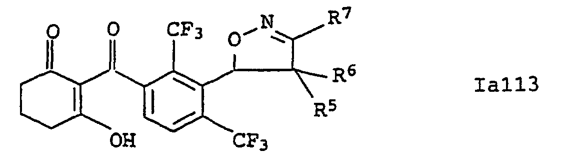

Ebenso insbesondere bevorzugt sind die Verbindungen Ia114., besonders die Verbindungen Ia114.1 - Ia114.64, die sich von den Verbindungen Ia1.1 - Ia1.64 dadurch unterscheiden, daß R¹ und R² für Trifluormethyl und R¹⁵ für Methyl stehen.

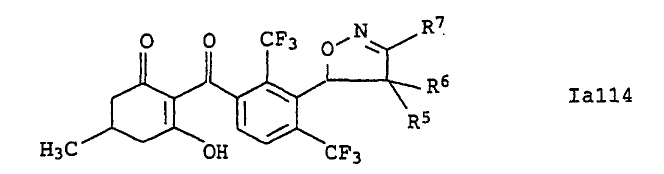

Ebenso insbesondere bevorzugt sind die Verbindungen Ia115., besonders die Verbindungen Ia115.1 - Ia115.64, die sich von den Verbindungen Ia1.1 - Ia1.64 dadurch unterscheiden, daß R¹ und R² für Trifluormethyl und R¹⁵ und R¹⁶ für Methyl stehen.

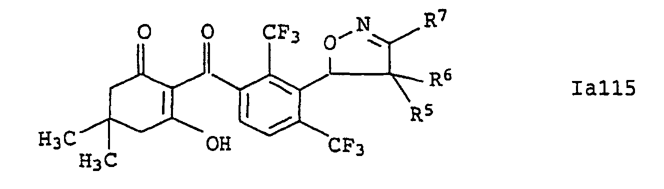

Ebenso insbesondere bevorzugt sind die Verbindungen Ia116., besonders die Verbindungen Ia116.1 - Ia116.64, die sich von den Verbindungen Ia1.1 - Ia1.64 dadurch unterscheiden, daß R¹ und R² für Trifluormethyl und R¹³ und R¹⁷ für Methyl stehen.

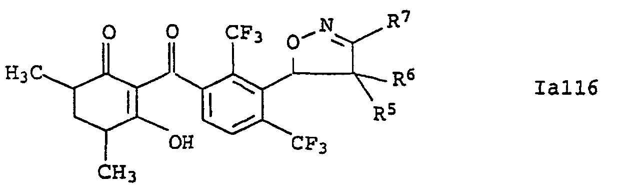

Ebenso insbesondere bevorzugt sind die Verbindungen Ia117., besonders die Verbindungen Ia117.1 - Ia117.64, die sich von den Verbindungen Ia1.1 - Ia1.64 dadurch unterscheiden, daß R¹ und R² für Trifluormethyl, R¹³ für Methylthio und R¹⁴ für Methyl stehen.

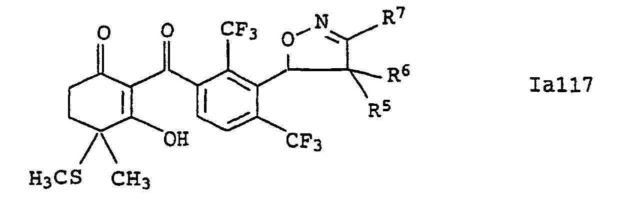

Ebenso insbesondere bevorzugt sind die Verbindungen Ia118., besonders die Verbindungen Ia118.1 - Ia118.64, die sich von den Verbindungen Ia1.1 - Ia1.64 dadurch unterscheiden, daß R¹ und R² für Trifluormethyl und R¹⁴ und R¹⁸ gemeinsam für Ethan-1,2-diyl stehen.

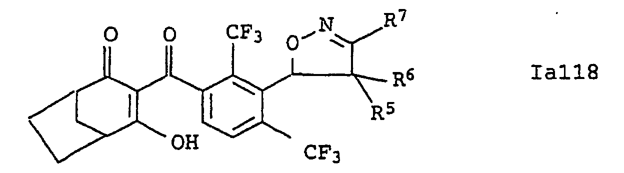

Ebenso insbesondere bevorzugt sind die Verbindungen Ia119., besonders die Verbindungen Ia119.1 - Ia119.64, die sich von den Verbindungen Ia1.1 - Ia1.64 dadurch unterscheiden, daß R¹ und R² für Trifluormethyl, R¹³, R¹⁴, R¹⁷ und R¹⁸ für Methyl und R¹⁵ und R¹⁶ gemeinsam für Sauerstoff stehen.

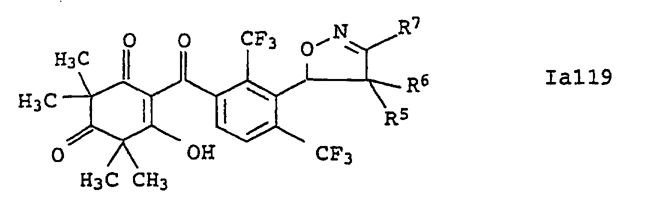

Ebenso insbesondere bevorzugt sind die Verbindungen Ia120., besonders die Verbindungen Ia120.1 - Ia120.64, die sich von den Verbindungen Ia1.1 - Ia1.64 dadurch unterscheiden, daß R¹ und R² für Trifluormethyl und R¹⁵ für Hydroxy stehen.

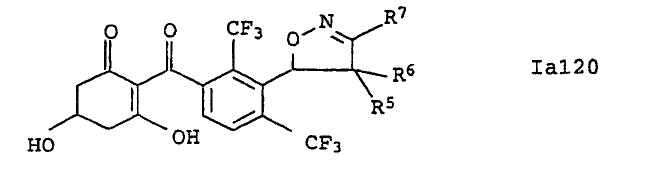

Ebenso insbesondere bevorzugt sind die Verbindungen Ia121., besonders die Verbindungen Ia121.1 - Ia121.64, die sich von den Verbindungen Ia1.1 - Ia1.64 dadurch unterscheiden, daß R¹ für Methoxy und R² für Trifluormethyl stehen.

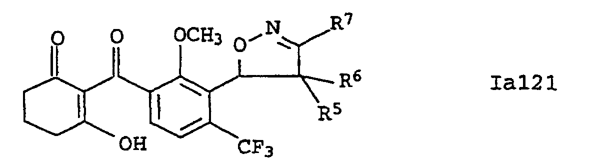

Ebenso insbesondere bevorzugt sind die Verbindungen Ia122., besonders die Verbindungen Ia122.1 - Ia122.64, die sich von den Verbindungen Ia1.1 - Ia1.64 dadurch unterscheiden, daß R¹ für Methoxy, R² für Trifluormethyl und R¹⁵ für Methyl stehen.

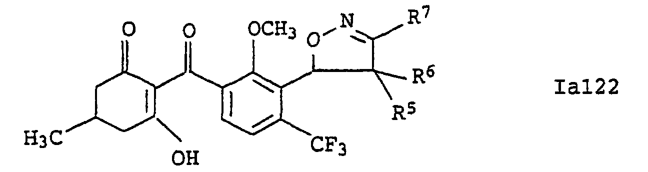

Ebenso insbesondere bevorzugt sind die Verbindungen Ia123., besonders die Verbindungen Ia123.1 - Ia123.64, die sich von den Verbindungen Ia1.1 - Ia1.64 dadurch unterscheiden, daß R¹ für Methoxy, R² für Trifluormethyl und R¹⁵ und R¹⁶ für Methyl stehen.

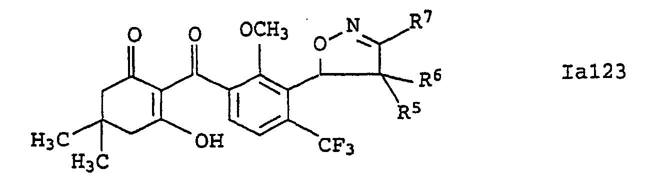

Ebenso insbesondere bevorzugt sind die Verbindungen Ia124., besonders die Verbindungen Ia124.1 - Ia124.64, die sich von den Verbindungen Ia1.1 - Ia1.64 dadurch unterscheiden, daß R¹ für Methoxy, R² für Trifluormethyl und R¹³ und R¹⁷ für Methyl stehen.

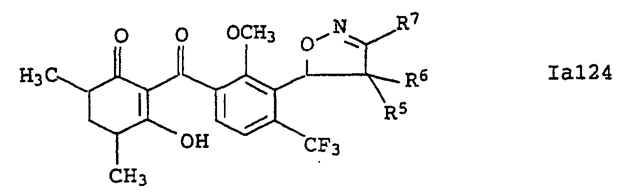

Ebenso insbesondere bevorzugt sind die Verbindungen Ia125., besonders die Verbindungen Ia125.1 - Ia125.64, die sich von den Verbindungen Ia1.1 - Ia1.64 dadurch unterscheiden, daß R¹ für Methoxy, R² für Trifluormethyl, R¹³ für Methylthio und R¹⁴ für Methyl stehen.

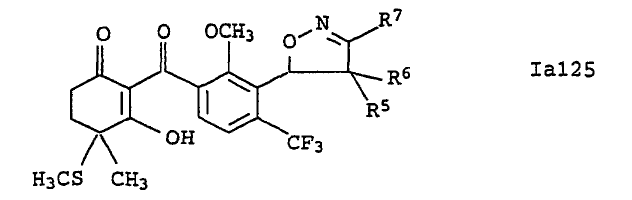

Ebenso insbesondere bevorzugt sind die Verbindungen Ia126., besonders die Verbindungen Ia126.1 - Ia126.64, die sich von den Verbindungen Ia1.1 - Ia1.64 dadurch unterscheiden, daß R¹ für Methoxy, R² für Trifluormethyl und R¹⁴ und R¹⁸ gemeinsam für Ethan-1,2-diyl stehen.

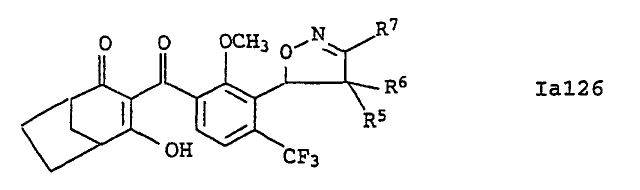

Ebenso insbesondere bevorzugt sind die Verbindungen Ia127., besonders die Verbindungen Ia127.1 - Ia127.64, die sich von den Verbindungen Ia1.1 - Ia1.64 dadurch unterscheiden, daß R¹ für Methoxy, R² für Trifluormethyl, R¹³, R¹⁴, R¹⁷ und R¹⁸ für Methyl und R¹⁵ und R¹⁶ gemeinsam für Sauerstoff stehen.

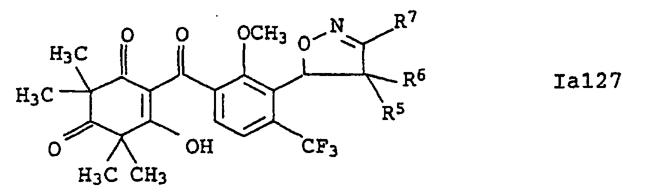

Ebenso insbesondere bevorzugt sind die Verbindungen Ia128., besonders die Verbindungen Ia128.1 - Ia128.64, die sich von den Verbindungen Ia1.1 - Ia1.64 dadurch unterscheiden, daß R¹ für Methoxy, R² für Trifluormethyl und R¹⁵ für Hydroxy stehen.

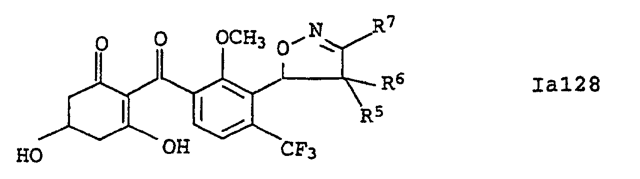

Ebenso insbesondere bevorzugt sind die Verbindungen Ia129., besonders die Verbindungen Ia129.1 - Ia129.64, die sich von den Verbindungen Ia1.1 - Ia1.64 dadurch unterscheiden, daß R³ für Chlor steht.

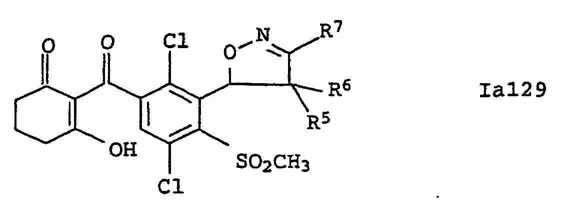

Ebenso insbesondere bevorzugt sind die Verbindungen Ia130., besonders die Verbindungen Ia130.1 - Ia130.64, die sich von den Verbindungen Ia1.1 - Ia1.64 dadurch unterscheiden, daß R³ für Chlor und R¹⁵ für Methyl stehen.

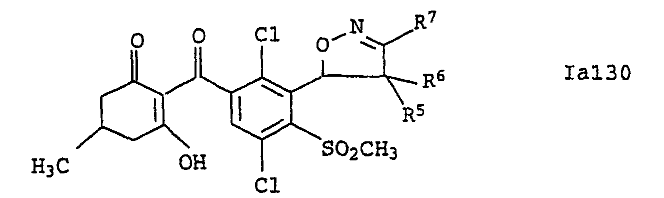

Ebenso insbesondere bevorzugt sind die Verbindungen Ia131., besonders die Verbindungen Ia131.1 - Ia131.64, die sich von den Verbindungen Ia1.1 - Ia1.64 dadurch unterscheiden, daß R³ für Chlor und R¹⁵ und R¹⁶ für Methyl stehen.

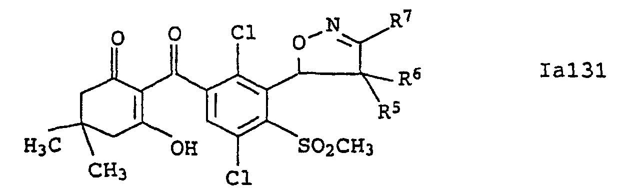

Ebenso insbesondere bevorzugt sind die Verbindungen Ia132., besonders die Verbindungen Ia132.1 - Ia132.64, die sich von den Verbindungen Ia1.1 - Ia1.64 dadurch unterscheiden, daß R³ für Chlor und R¹³ und R¹⁷ für Methyl stehen.

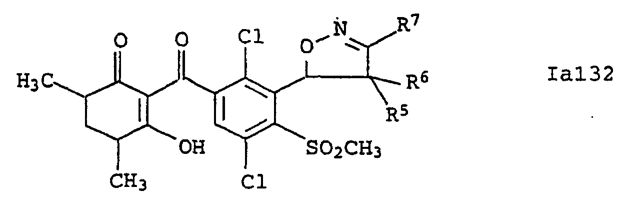

Ebenso insbesondere bevorzugt sind die Verbindungen Ia133., besonders die Verbindungen Ia133.1 - Ia133.64, die sich von den Verbindungen Ia1.1 - Ia1.64 dadurch unterscheiden, daß R³ für Chlor, R¹³ Methylthio und R¹⁴ für Methyl steht.

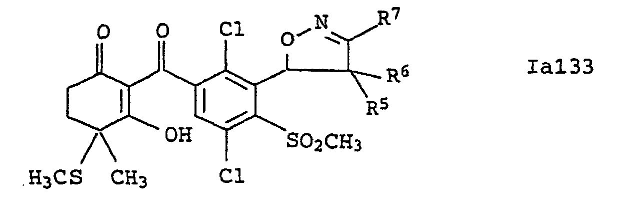

Ebenso insbesondere bevorzugt sind die Verbindungen Ia134., besonders die Verbindungen Ia134.1 - Ia134.64, die sich von den Verbindungen Ia1.1 - Ia1.64 dadurch unterscheiden, daß R³ für Chlor und R¹⁴ und R¹⁸ gemeinsam für Ethan-1,2-diyl stehen.

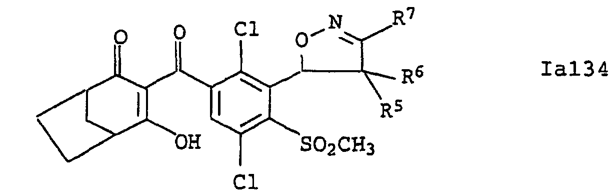

Ebenso insbesondere bevorzugt sind die Verbindungen Ia135., besonders die Verbindungen Ia135.1 - Ia135.64, die sich von den Verbindungen Ia1.1 - Ia1.64 dadurch unterscheiden, daß R³ für Chlor, R¹³, R¹⁴, R¹⁷ und R¹⁸ für Methyl und R¹⁵ und R¹⁶ gemeinsam für Sauerstoff stehen.

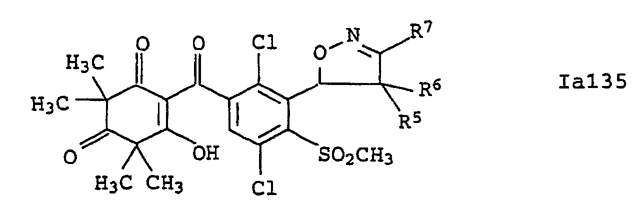

Ebenso insbesondere bevorzugt sind die Verbindungen Ia136., besonders die Verbindungen Ia136.1 - Ia136.64, die sich von den Verbindungen Ia1.1 - Ia1.64 dadurch unterscheiden, daß R³ für Chlor und R¹⁵ für Hydroxy stehen.

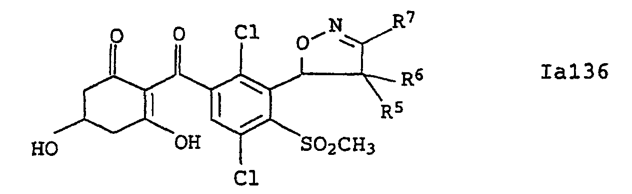

Ebenso insbesondere bevorzugt sind die Verbindungen Ia137., besonders die Verbindungen Ia137.1 - Ia137.64, die sich von den Verbindungen Ia1.1 - Ia1.64 dadurch unterscheiden, daß R² für Ethylsulfonyl und R³ für Chlor stehen.

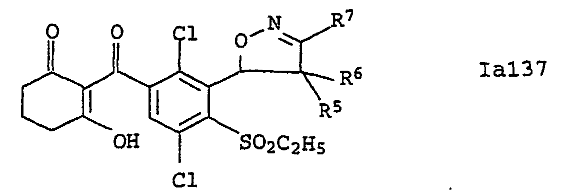

Ebenso insbesondere bevorzugt sind die Verbindungen Ia138., besonders die Verbindungen Ia138.1 - Ia138.64, die sich von den Verbindungen Ia1.1 - Ia1.64 dadurch unterscheiden, daß R² für Ethylsulfonyl, R³ für Chlor und R¹⁵ Methyl stehen.

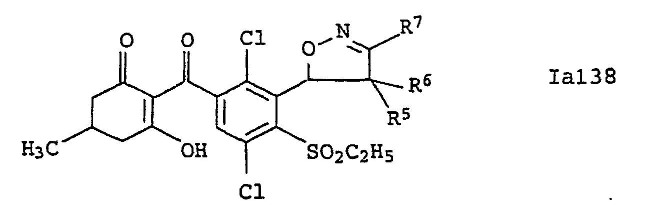

Ebenso insbesondere bevorzugt sind die Verbindungen Ia139., besonders die Verbindungen Ia139.1 - Ia139.64, die sich von den Verbindungen Ia1.1 - Ia1.64 dadurch unterscheiden, daß R² für Ethylsulfonyl, R³ für Chlor und R¹⁵ und R¹⁶ für Methyl stehen.

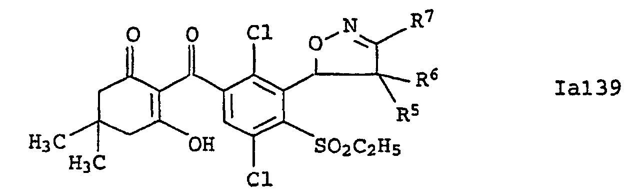

Ebenso insbesondere bevorzugt sind die Verbindungen Ia140., besonders die Verbindungen Ia140.1 - Ia140.64, die sich von den Verbindungen Ia1.1 - Ia1.64 dadurch unterscheiden, daß R² für Ethylsulfonyl, R³ für Chlor und R¹³ und R¹⁷ für Methyl stehen.

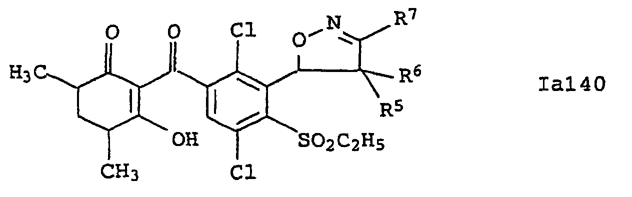

Ebenso insbesondere bevorzugt sind die Verbindungen Ia141., besonders die Verbindungen Ia141.1 - Ia141.64, die sich von den Verbindungen Ia1.1 - Ia1.64 dadurch unterscheiden, daß R² für Ethylsulfonyl, R³ für Chlor, R¹³ für Methylthio und R¹⁴ für Methyl stehen.

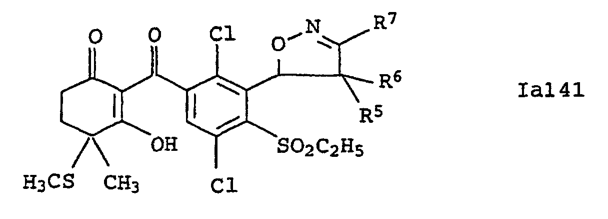

Ebenso insbesondere bevorzugt sind die Verbindungen Ia142., besonders die Verbindungen Ia142.1 - Ia142.64, die sich von den Verbindungen Ia1.1 - Ia1.64 dadurch unterscheiden, daß R² für Ethylsulfonyl, R³ für Chlor, R¹⁴ und R¹⁸ gemeinsam für Ethan-1,2-diyl stehen.

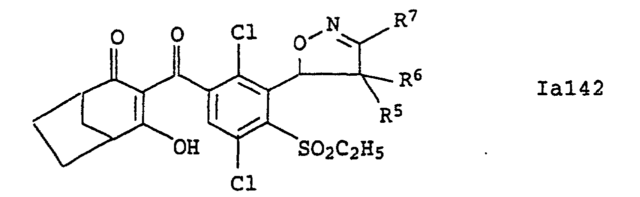

Ebenso insbesondere bevorzugt sind die Verbindungen Ia143., besonders die Verbindungen Ia143.1 - Ia143.64, die sich von den Verbindungen Ia1.1 - Ia1.64 dadurch unterscheiden, daß R² für Ethylsulfonyl, R³ für Chlor, R¹³, R¹⁴, R¹⁷ und R¹⁸ für Methyl und R¹⁵ und R¹⁶ gemeinsam für Sauerstoff stehen.

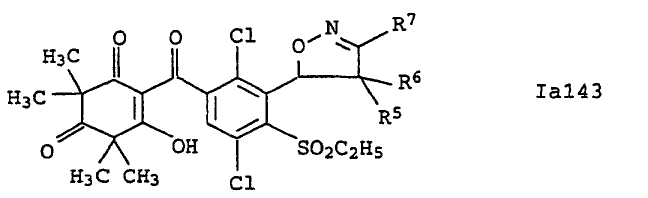

Ebenso insbesondere bevorzugt sind die Verbindungen Ia144., besonders die Verbindungen Ia144.1 - Ia144.64, die sich von den Verbindungen Ia1.1 - Ia1.64 dadurch unterscheiden, daß R² für Ethylsulfonyl, R³ für Chlor, R¹⁵ für Hydroxy stehen.

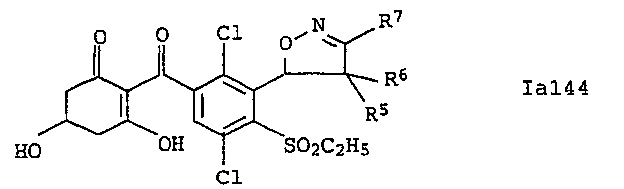

Ebenso insbesondere bevorzugt sind die Verbindungen Ia145., besonders die Verbindungen Ia145.1 - Ia145.64, die sich von den Verbindungen Ia1.1 - Ia1.64 dadurch unterscheiden, daß R¹ und R³ für Methyl stehen.

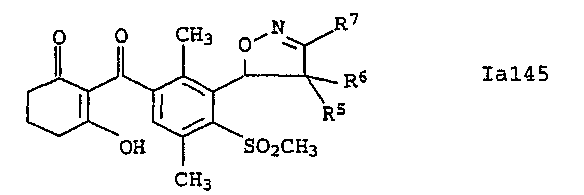

Ebenso insbesondere bevorzugt sind die Verbindungen Ia146., besonders die Verbindungen Ia146.1 - Ia146.64, die sich von den Verbindungen Ia1.1 - Ia1.64 dadurch unterscheiden, daß R¹, R³ und R¹⁵ für Methyl stehen.

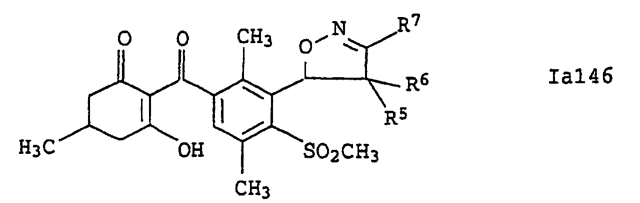

Ebenso insbesondere bevorzugt sind die Verbindungen Ia147., besonders die Verbindungen Ia147.1 - Ia147.64, die sich von den Verbindungen Ia1.1 - Ia1.64 dadurch unterscheiden, daß R¹, R³, R¹⁵ und R¹⁶ für Methyl stehen.

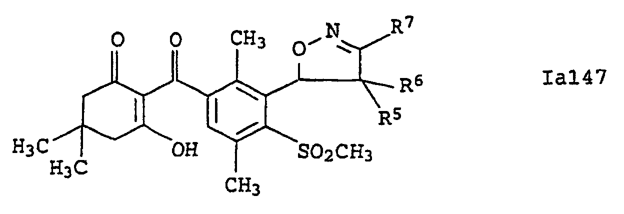

Ebenso insbesondere bevorzugt sind die Verbindungen Ia148., besonders die Verbindungen Ia148.1 - Ia148.64, die sich von den Verbindungen Ia1.1 - Ia1.64 dadurch unterscheiden, daß R¹, R³, R¹³ und R¹⁷ für Methyl stehen.

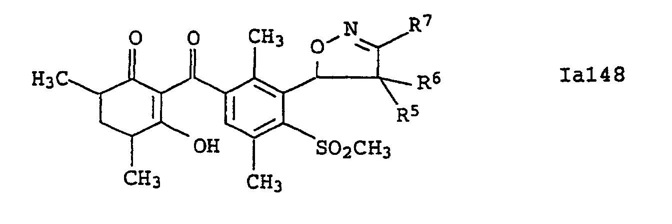

Ebenso insbesondere bevorzugt sind die Verbindungen Ia149., besonders die Verbindungen Ia149.1 - Ia149.64, die sich von den Verbindungen Ia1.1 - Ia1.64 dadurch unterscheiden, daß R¹, R³ und R¹⁴ für Methyl und R¹³ für Methylthio stehen.

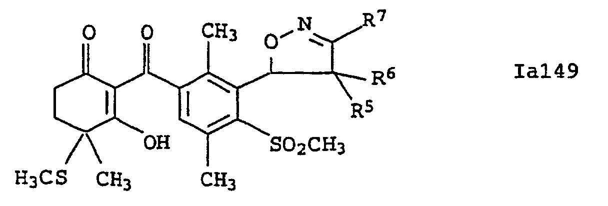

Ebenso insbesondere bevorzugt sind die Verbindungen Ia150., besonders die Verbindungen Ia150.1 - Ia150.64, die sich von den Verbindungen Ia1.1 - Ia1.64 dadurch unterscheiden, daß R¹ und R³ für Methyl und R¹⁴ und R¹⁸ gemeinsam für Ethan-1,2-diyl stehen.

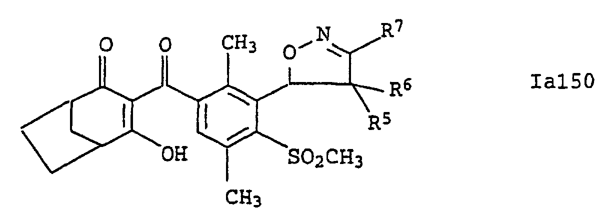

Ebenso insbesondere bevorzugt sind die Verbindungen Ia151., besonders die Verbindungen Ia151.1 - Ia151.64, die sich von den Verbindungen Ia1.1 - Ia1.64 dadurch unterscheiden, daß R¹, R³, R¹³, R¹⁴, R¹⁷ und R¹⁸ für Methyl und R¹⁵ und R¹⁶ gemeinsam für Sauerstoff stehen.

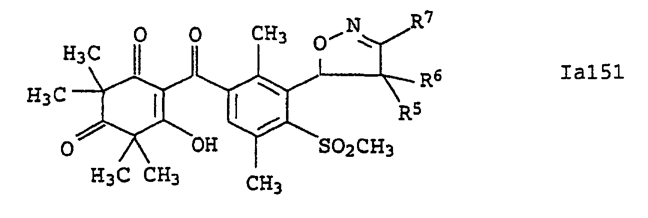

Ebenso insbesondere bevorzugt sind die Verbindungen Ia152., besonders die Verbindungen Ia152.1 - Ia152.64, die sich von den Verbindungen Ia1.1 - Ia1.64 dadurch unterscheiden, daß R¹ und R³ für Methyl und R¹⁵ für Hydroxy stehen.

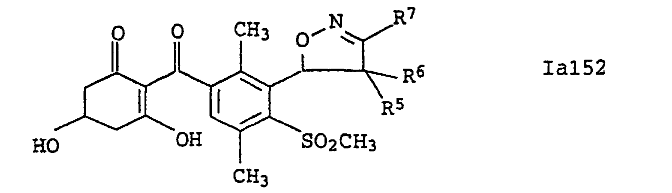

Ebenso insbesondere bevorzugt sind die Verbindungen Ia153., besonders die Verbindungen Ia153.1 - Ia153.64, die sich von den Verbindungen Ia1.1 - Ia1.64 dadurch unterscheiden, daß R¹ und R³ für Methyl und R² für Ethylsulfonyl stehen.

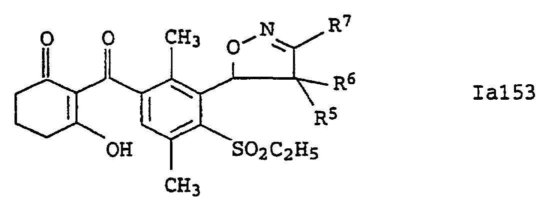

Ebenso insbesondere bevorzugt sind die Verbindungen Ia154., besonders die Verbindungen Ia154.1 - Ia154.64, die sich von den Verbindungen Ia1.1 - Ia1.64 dadurch unterscheiden, daß R¹, R³ und R¹⁵ für Methyl und R² für Ethylsulfonyl stehen.

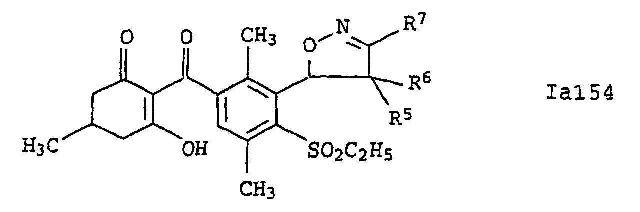

Ebenso insbesondere bevorzugt sind die Verbindungen Ia155., besonders die Verbindungen Ia155.1 - Ia155.64, die sich von den Verbindungen Ia1.1 - Ia1.64 dadurch unterscheiden, daß R¹, R³, R¹⁵ und R¹⁶ für Methyl und R² für Ethylsulfonyl stehen.

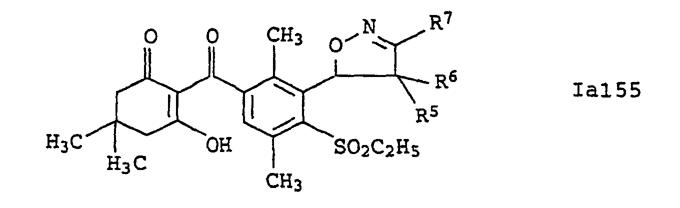

Ebenso insbesondere bevorzugt sind die Verbindungen Ia156., besonders die Verbindungen Ia156.1 - Ia156.64, die sich von den Verbindungen Ia1.1 - Ia1.64 dadurch unterscheiden, daß R¹, R³, R¹³ und R¹⁷ für Methyl und R² für Ethylsulfonyl stehen.

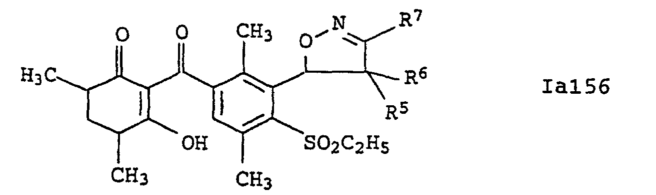

Ebenso insbesondere bevorzugt sind die Verbindungen Ia157., besonders die Verbindungen Ta157.1 - Ia157.64, die sich von den Verbindungen Ia1.1 - Ia1.64 dadurch unterscheiden, daß R¹, R³ und R¹⁴ für Methyl, R² für Ethylsulfonyl und R¹³ für Methylthio stehen.

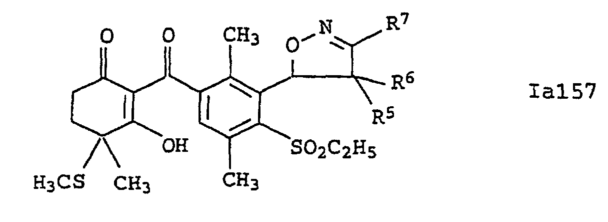

Ebenso insbesondere bevorzugt sind die Verbindungen Ia158., besonders die Verbindungen Ia158.1 - Ia158.64, die sich von den Verbindungen Ia1.1 - Ia1.64 dadurch unterscheiden, daß R¹ und R³ für Methyl, R² für Ethylsulfonyl und R¹⁴ und R¹⁸ gemeinsam für Ethan-1,2-diyl stehen.

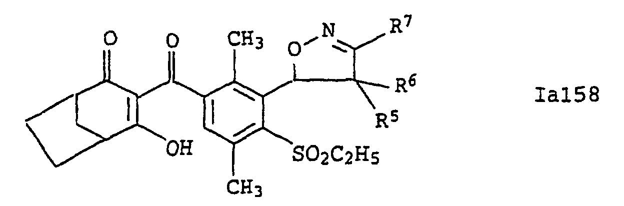

Ebenso insbesondere bevorzugt sind die Verbindungen Ia159., besonders die Verbindungen Ia159.1 - Ia159.64, die sich von den verbindungen Ia1.1 - Ia1.64 dadurch unterscheiden, daß R¹, R³, R¹³, R¹⁴, R¹⁷ und R¹⁸ für Methyl, R² für Ethylsulfonyl und R¹⁵ und R¹⁶ gemeinsam für Sauerstoff stehen.

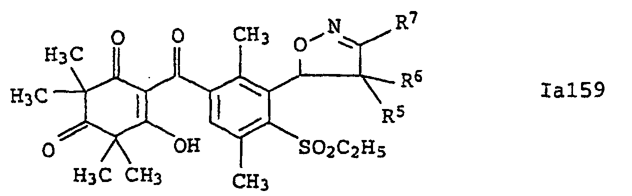

Ebenso insbesondere bevorzugt sind die Verbindungen Ia160., besonders die Verbindungen Ia160.1 - Ia160.64, die sich von den Verbindungen Ia1.1 - Ia1.64 dadurch unterscheiden, daß R¹ und R³ für Methyl, R² für Ethylsulfonyl und R¹⁵ für Hydroxy stehen.

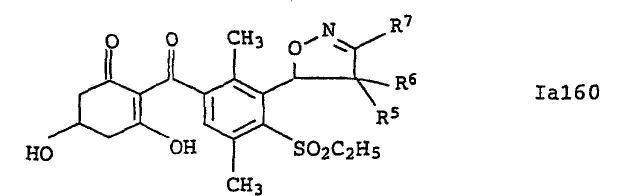

Ebenso insbesondere bevorzugt sind die Verbindungen Ia161., besonders die Verbindungen Ia161.1 - Ia161.64, die sich von den Verbindungen Ia1.1 - Ia1.64 dadurch unterscheiden, daß R¹ und R³ für Methyl und R² für Chlor stehen.

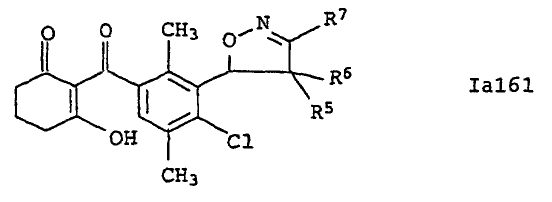

Ebenso insbesondere bevorzugt sind die Verbindungen Ia162., besonders die Verbindungen Ia162.1 - Ia162.64, die sich von den Verbindungen Ia1.1 - Ia1.64 dadurch unterscheiden, daß R¹, R³ und R¹⁵ für Methyl und R² für Chlor stehen.

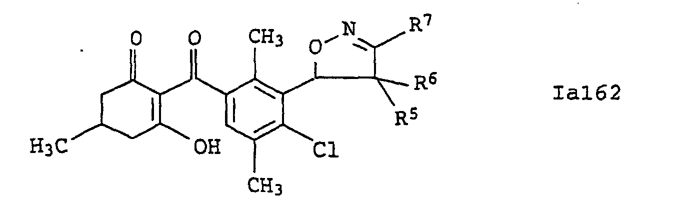

Ebenso insbesondere bevorzugt sind die Verbindungen Ia163., besonders die Verbindungen Ia163.1 - Ia163.64, die sich von den Verbindungen Ia1.1 - Ia1.64 dadurch unterscheiden, daß R¹, R³, R¹⁵ und R¹⁶ für Methyl und R² für Chlor stehen.

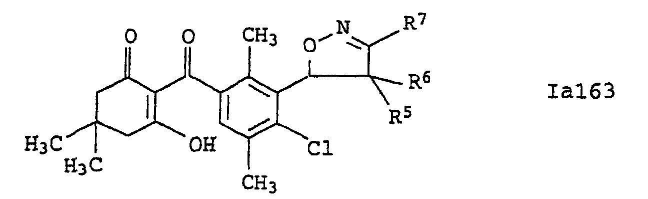

Ebenso insbesondere bevorzugt sind die Verbindungen Ia164., besonders die Verbindungen Ia164.1 - Ia164.64, die sich von den Verbindungen Ia1.1 - Ia1.64 dadurch unterscheiden, daß R¹, R³, R¹³ und R¹⁷ für Methyl und R² für Chlor stehen.

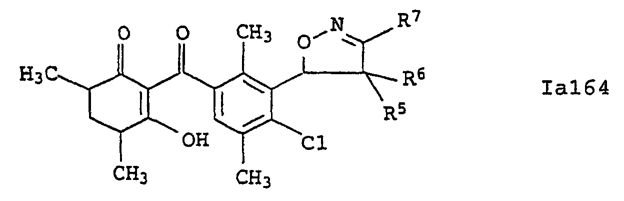

Ebenso insbesondere bevorzugt sind die Verbindungen Ia165., besonders die Verbindungen Ia165.1 - Ia165.64, die sich von den Verbindungen Ia1.1 - Ia1.64 dadurch unterscheiden, daß R¹, R³ und R¹⁴ für Methyl, R² für Chlor und R¹³ für Methylthio stehen. 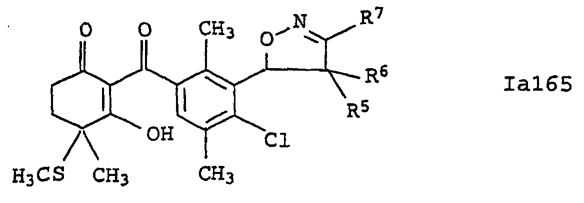 Ebenso insbesondere bevorzugt sind die Verbindungen Ia166., besonders die Verbindungen Ia166.1 - Ia166.64, die sich von den Verbindungen Ia1.1 - Ia1.64 dadurch unterscheiden, daß R¹ und R³ für Methyl, R² für Chlor und R¹⁴ und R¹⁸ gemeinsam für Ethan-1,2-diyl stehen.

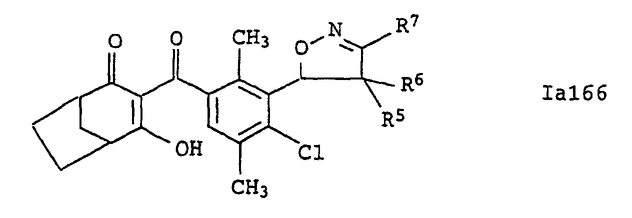

Ebenso insbesondere bevorzugt sind die Verbindungen Ia167., besonders die Verbindungen Ia167.1 - Ia167.64, die sich von den Verbindungen Ia1.1 - Ia1.64 dadurch unterscheiden, daß R¹, R³, R¹³, R¹⁴, R¹⁷ und R¹⁸ für Methyl, R² für Chlor und R¹⁵ und R¹⁶ gemeinsam für Sauerstoff stehen.

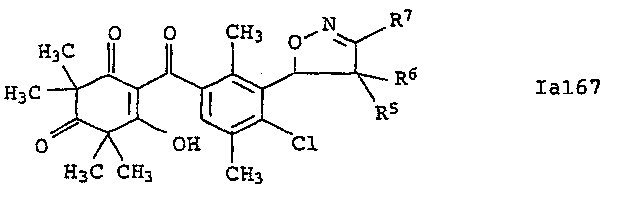

Ebenso insbesondere bevorzugt sind die Verbindungen Ia168., besonders die Verbindungen Ia168.1 - Ia168.64, die sich von den Verbindungen Ia1.1 - Ia1.64 dadurch unterscheiden, daß R¹ und R³ für Methyl und R¹⁵ für Hydroxy stehen.

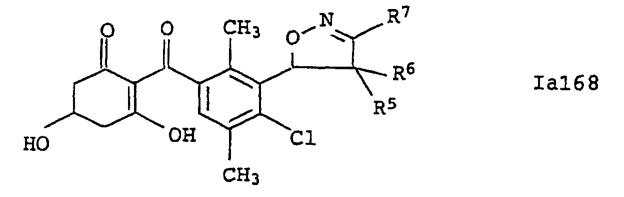

In einer weiteren bevorzugten Ausführungsform der Verbindungen der Formal I haben die Variablen, und zwar für sich allein oder in Kombination, folgende Bedeutung:
- R¹: Halogen, C₁-C₆-Alkyl oder C₁-C₆-Alkoxy; bevorzugt Halogen wie Chlor oder Brom, C₁-C₄-Alkyl wie Methyl, Ethyl der Propyl, C₁-C₄-Alkoxy wie Methoxy oder Ethoxy;
besonders bevorzugt Chlor, Methyl oder Methoxy;
- R²: Halogen oder C₁-C₆-Alkylsulfonyl; bevorzugt Halogen wie Chlor oder Brom oder C₁-C₄-Alkylsulfonyl wie Methylsulfonyl oder Ethylsulfonyl; besonders bevorzugt Chlor oder Methylsulfonyl;
- R³: Wasserstoff;
- R⁴: Wasserstoff;
- R⁵: Wasserstoff;
- R⁶: Wasserstoff;
- R⁷: C₁-C₆-Alkyl, C₁-C₄-Alkylcarbonyl oder C₁-C₄-Alkoxycarbonyl; bevorzugt C₁-C₆-Alkyl wie Methyl, Ethyl, 1-Methylethyl oder 1,1-Dimethylethyl, Methylcarbonyl oder Ethylcarbonyl, Methoxycarbonyl oder Ethoxycarbonyl;
- R¹²: xHydroxy;
- R¹³: Wasserstoff oder C₁-C₄-Alkyl;
- R¹⁴: Wasserstoff oder C₁-C₄-Alkyl;
- R¹⁵: Wasserstoff oder C₁-C₄-Alkyl;
- R¹⁶: Wasserstoff oder C₁-C₄-Alkyl;
- R¹⁷: Wasserstoff oder C₁-C₄-Alkyl;
- R¹⁸: Wasserstoff oder C₁-C₄-Alkyl;
oder
- R¹⁵ und R¹⁶: stehen gemeinsam für ein Sauerstoffatom;
oder
- R¹⁴ und R¹⁸: stehen gemeinsam für C₁-C₅-Alkandiyl, insbesondere für 1,2-Ethandiyl;

Die 3-(4,5-Dihydroisoxazol-5-yl)benzoylcyclohexenone der Formel I sind auf verschiedene Art und Weise erhältlich, beispielsweise nach folgendem Verfahren:

### Verfahren A:

Umsetzung von Cyclohexenonen der Formel II (mit R¹² = OH) mit einer aktivierten Carbonsäure IIIα oder einer Carbonsäure IIIβ, die vorzugsweise in situ aktiviert wird, zu dem Acylierungsprodukt und anschließende Umlagerung.

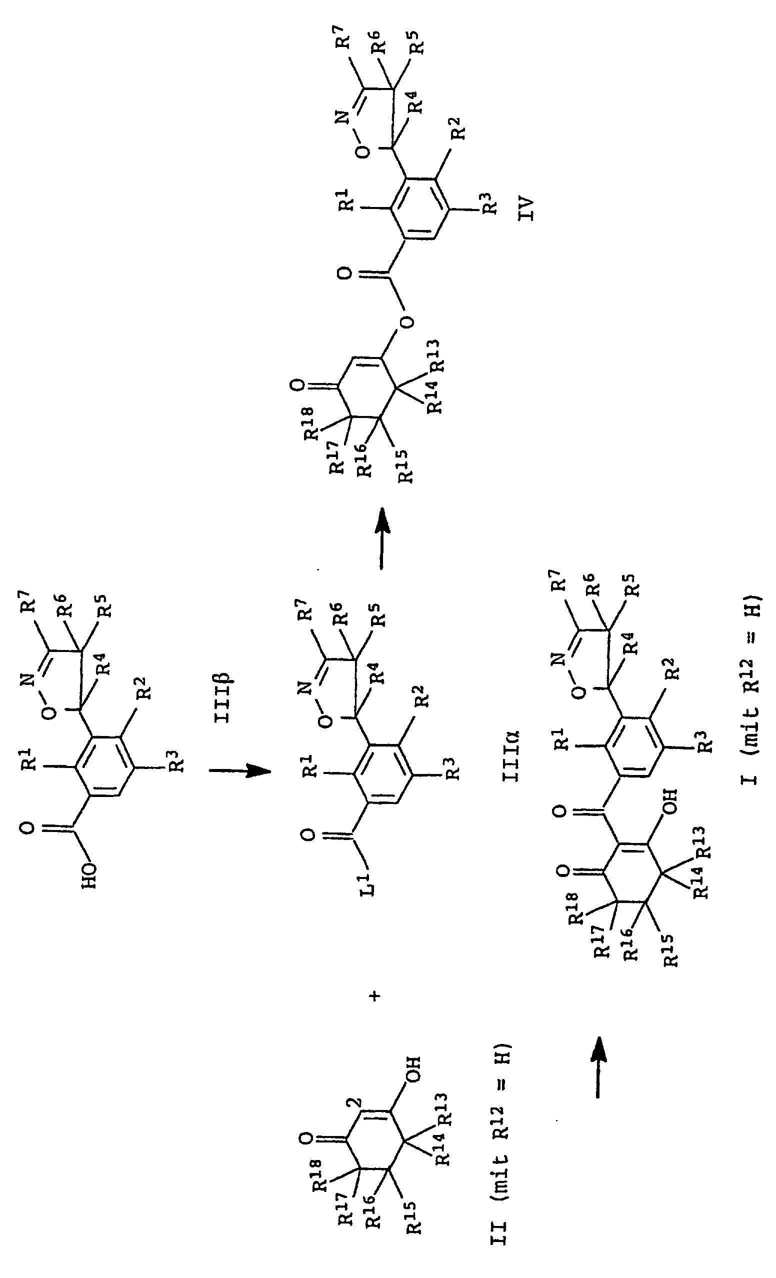

L¹ steht für eine nucleophil verdrängbare Abgangsgruppe, wie Halogen z.B Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclohexylcarbodiimid, Triphenylphosphan/Azodicarbonsäureester, 2-Pyridindisulfid/Triphenylphosphan, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein Überschuß der Hilfsbase z.B. 1,2 bis 2,5 Moläquivalente, insbesondere 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 40°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet, vorzugsweise in doppelt äquimolaren Verhältnis in Bezug auf den Ester.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 25, vorzugsweise 5 bis 15, insbesondere 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder'mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 und US 4 643 757 genannt).

### Verfahren B:

Umsetzung von 3-(4,5-Dihydroisoxazol-5-yl)benzoylcyclohexenone der Formel I (mit R¹² = OH, SH) mit einer Verbindung der Formel V:

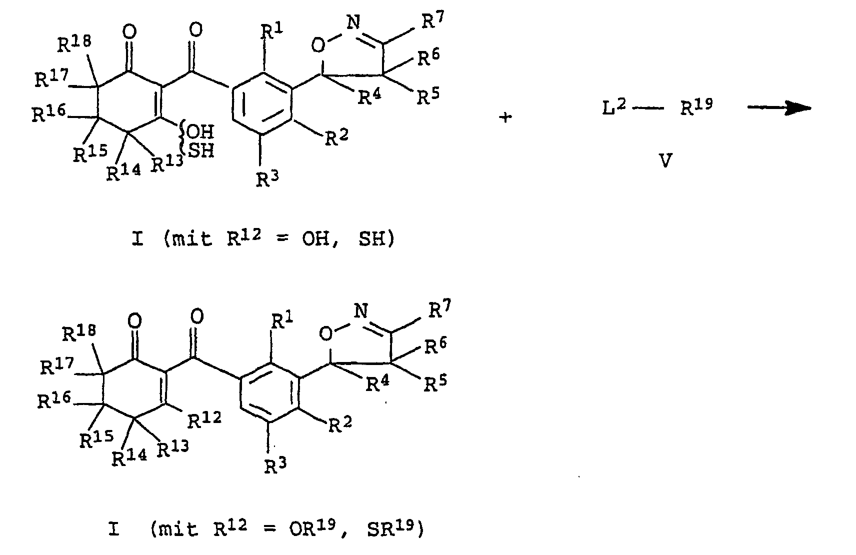

L² steht für eine nucleophil verdrängbare Abgangsgruppe, wie Halogen, z.B. Brom, Chlor, Hetaryl, z.B. Imidazolyl, Pyridyl, Sulfonat.

Die Verbindungen der Formel V können direkt eingesetzt werden, wie z.B. im Fall der Sulfonsäurehalogenide, Sulfonsäureanhydride oder in situ erzeugt werden, z.B. aktivierte Sulfonsäuren (mittels Sulfonsäure und Dicyclohexylcarbonyldiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf II, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin und Pyridin.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

### Verfahren C:

Verbindungen der Formel I mit R¹² = Halogen werden durch Umsetzung von Verbindungen der Formel I mit R¹² = Hydroxy mit einem Halogenierungsmittel erhalten (Hal steht für Halogen).

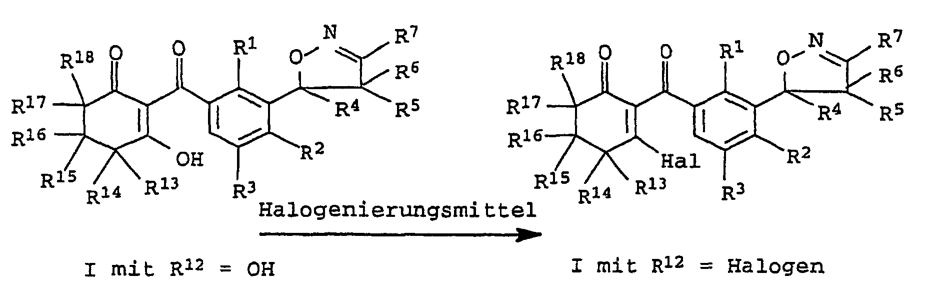

Als Halogenierungsmittel eignen sich beispielsweise Phosgen, Diphosgen, Triphosgen, Thionylchlorid, Oxalylchlorid, Phosphoroxychlorid, Phosphorpentachlorid, Mesylchlorid, Chlormethylen-N,N-dimethylammoniumchlorid, Oxalylbromid, Phosphoroxybromid etc.

Die Ausgangsverbindungen werden in der Regel im äquimolaren Verhältnis eingesetzt. Es kann auch von Vorteil sein, die eine oder andere Komponente im Überschuss einzusetzen.

Als Lösungsmittel kommen z. B. chlorierte Kohlenwasserstoffe, wie Methylenchlorid oder 1,2-Dichlorethan, aromatische Kohlenwasserstoffe, z. B. Toluol, Xylol oder Chlorbenzol, polare aprotische Lösungsmittel, wie Acetonitril, Dimethylformamid oder Dimethylsulfoxid oder Gemische hiervon in Betracht. Es ist auch möglich, die Reaktion in Substanz durchzuführen.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

### Verfahren D:

Verbindungen der Formel I mit R¹² = Mercapto, OR¹⁹ oder SR¹⁹ können durch Umsetzung von Verbindungen der Formel I mit R¹² = Halogen mit Verbindungen VI, gegebenenfalls in Gegenwart einer Base oder unter vorangegangener Salzbildung erhalten werden.

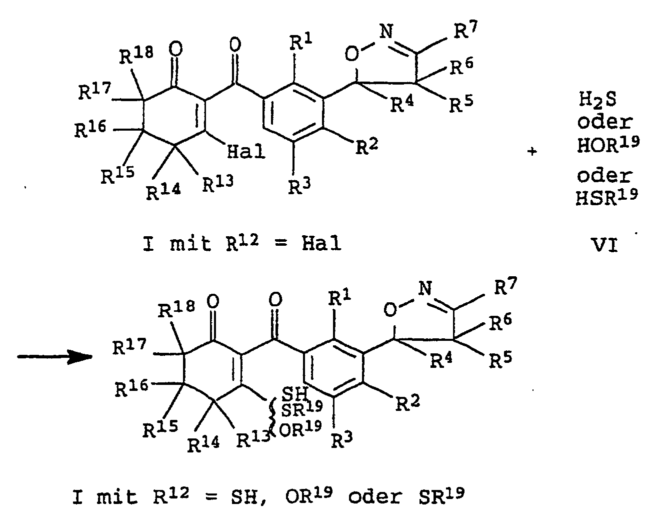

Die Ausgangsverbindungen werden in der Regel im äquimolaren Verhältnis eingesetzt. Es kann aber auch von Vorteil sein, die eine oder andere Komponente im Überschuss einzusetzen.

Gegebenenfalls kann es auch von Vorteil sein, die Umsetzung in Gegenwart einer Base durchzuführen. Die Reaktanden und die Base werden dabei zweckmäßigerweise in äquimolaren Mengen eingesetzt. Ein Überschuss an Base, von z. B. 1,5 bis 3 Moläquivalente, bezogen auf I mit R⁸ = Hal, kann unter Umständen von Vorteil sein.

Als Basen eignen sich tertiäre Alkylamine, wie Triethylamin, aromatische Amine, wie Pyridin, Alkalimetallcarbonate, z. B. Natriumcarbonat oder Kaliumcarbonat, Alkalimetallhydrogencarbonate, wie Natriumhydrogencarbonat oder Kaliumhydrogencarbonat, Alkalimetallalkoholate, wie Natriummethanolat, Natriumethanolat, Kalium-tert-butanolat oder Alkalimetallhydride, wie z. B. Natriumhydrid. Bevorzugt verwendet man Natriumhydrid oder Kalium-tertbutanolat.

Als Lösungsmittel kommen z. B. chlorierte Kohlenwasserstoffe, wie Methylenchlorid oder 1,2-Dichlorethan, aromatische Kohlenwasserstoffe, z. B. Toluol, Xylol oder Chlorbenzol, Ether, wie Diethylether, Methyl-tert-butylether, Tetrahydrofuran oder Dioxan, polare aprotische Lösungsmittel, wie Acetonitril, Dimethylformamid oder Dimethylsulfoxid oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

### Verfahren E:

Weiterhin können Verbindungen der Formel I mit R¹² = SOR²⁰ oder SO₂R²⁰ durch Reaktion mit einem Oxidationsmittel erhalten werden.

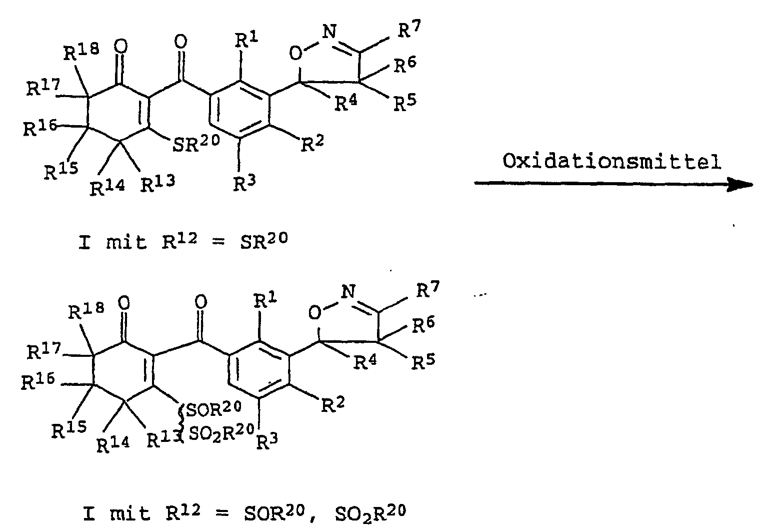

Als Oxidationsmittel kommen beispielsweise m-Chlorperbenzoesäure, Peroxyessigsäure, Trifluorperoxyessigsäure, Wasserstoffperoxid, gegebenenfalls in Gegenwart eines Katalysators, wie Wolframat, in Betracht.

Die Ausgangsverbindungen werden in der Regel in äquimolarem Verhältnis eingesetzt. Es kann von Vorteil sein, die eine oder andere Komponente im Überschuss einzusetzen.

Als Lösungsmittel kommen z. B. chlorierte Kohlenwasserstoffe, wie Methylenchlorid oder 1,2-Dichlorethan, aromatische Kohlenwasserstoffe, z. B. Toluol, Xylol oder Chlorbenzol, Ether, wie Diethylether, Methyl-tert-butylether, Tetrahydrofuran oder Dioxan, polare aprotische Lösungsmittel, wie Acetonitril oder Dimethylformamid oder Ester, wie Essigsäureethylester, oder Gemische hiervon in Betracht.

In der Regel liegt die Reaktionstemperatur im Bereich von 0 °C bis zur Höhe des Siedepunktes des Reaktionsgemisches.

Die Aufarbeitung kann in an sich bekannter Weise zum Produkt hin erfolgen.

Die als Ausgangsmaterialien verwendeten Cyclohexenone der Formel II (mit R¹² = OH) sind bekannt oder können nach an sich bekannten Verfahren hergestellt werden (z.B. EP-A 240 001 und J. Prakt. Chem. 315, 383 (1973)).

3-(4,5-Dihydroisoxazol-5-yl)-Carbonsäurederivate der Formel III sind neu, 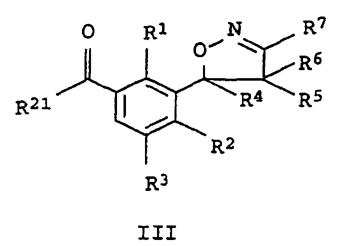 wobei die Variablen folgende Bedeutung haben.
- R¹, R²: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R³: Wasserstoff, Halogen oder C₁-C₆-Alkyl;
- R⁴: Wasserstoff oder C₁-C₄-Alkyl;
- R⁵, R⁶: Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di-(C₁-C₄-alkyl)-amino-C₁-C₄-alkyl, Di-(C₁-C₄-alkyl)- amino-imino-C₁-C₄-alkyl, Hydroxy-imino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl,
C₁-C₄-Alkoxy oder C1-C4-Halogenalkoxy;
oder
- R⁵ und R⁶: bilden gemeinsam eine C₂-C₆-Alkandiyl-Kette, die einbis vierfach durch C₁-C₄-Alkyl substituiert sein kann und/oder durch Sauerstoff oder einen gegebenenfalls C₁-C₄-Alkyl substituierten Stickstoff unterbrochen sein kann;
- R⁷: Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-alkyl)amino, Di-(C₁-C₄-alkoxy)methyl, Hydroxyimino-C₁-C₄-alkyl, C₁-C₆-Alkoxyimino-C₁-C₄-alkyl oder COR⁸;
- R⁸: Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
- R⁹: Wasserstoff oder C₁-C₄-Alkyl;
- R¹⁰: C₁-C₄-Alkyl;
- R²¹: Hydroxy oder abhydrolysierbarer Rest;

Beispiele für abhydrolysierbare Reste sind Alkoxy-, Phenoxy-, Alkylthio-, Phenylthio-Reste, die gegebenenfalls substituiert sein können, Halogenide, Hetarylreste, die über Stickstoff gebunden sind, Amino, Imino-Reste, die gegebenenfalls substituiert sein können etc.

Bevorzugt sind 3-(4,5-Dihydroisoxazol-5-yl)-Carbonsäurehalogenide der Formel IIIα', mit L^{1'} = Halogen (≙ III mit R²¹ = Halogen), 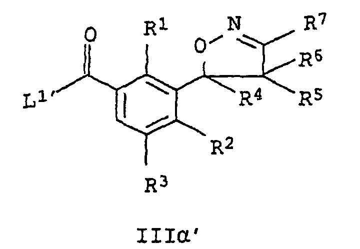 wobei die Variablen R¹ bis R⁷ die unter Formel III genannte Bedeutung haben und
- L^{1'}: Halogen, insbesondere Chlor oder Brom bedeutet.

Ebenso bevorzugt sind 3-(4,5-Dihydroisoxazol-5-yl)-Carbonsäuren der Formel IIIβ (≙ III mit R²¹ = Hydroxy), 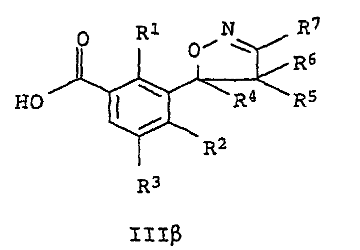 wobei die Variablen R¹ bis R⁷ die unter Formel III genannte Bedeutung haben.

Ebenso bevorzugt sind 3-(4,5-Dihydroisoxazol-5-yl)-Carbonsäureester der Formel IIIγ (≙ III mit R²¹ = C₁-C₆-Alkoxy), 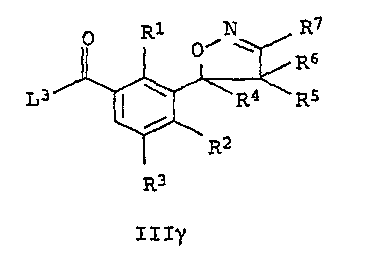 wobei die Variablen R¹ bis R⁷ die unter Formel III genannte Bedeutung haben und
- L³: C₁-C₆-Alkoxy bedeutet.

Die besonders bevorzugten Ausführungsformen der 3-(4,5-Dihydroisoxazol-5-yl)-Carbonsäurederivate der Formel III in Bezug auf die Variablen R¹ bis R⁷ entsprechen denen der 3-(4,5-Dihydroisoxazol-5-yl)cyclohexenone der Formel I.

Die 3-(4,5-Dihydroisoxazol-5-yl)-Carbonsäurehalogenide der Formel IIIα' (mit L^{1'} = Cl, Br,) können auf an sich bekannte Art und Weise durch Umsetzung der 3-(4,5-Dihydroisoxazol-5-yl)-Carbonsäuren der Formel IIIβ mit Halogenierungsreagentien wie Thionylchlorid, Thionylbromid, Phosgen, Diphosgen, Triphosgen, Oxalylchlorid, Oxalylbromid hergestellt werden.

Die 3-(4,5-Dihydroisoxazol-5-yl)-Carbonsäuren der Formel IIIβ können in bekannter Weise durch saure oder basische Hydrolyse aus den entsprechenden Estern der Formel IIIγ (L³ = C₁-C₆-Alkoxy) hergestellt werden.

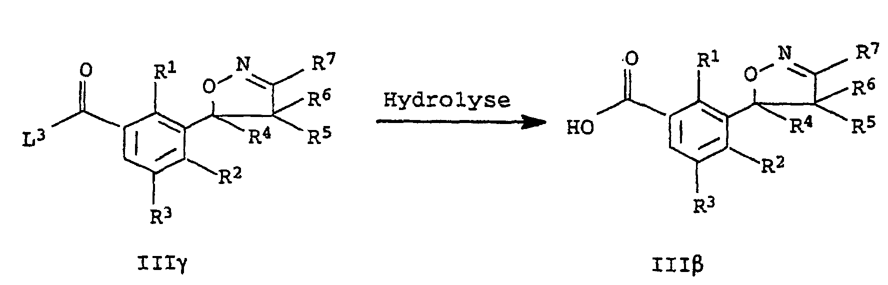

Ebenso können die 3-(4,5-Dihydroisoxazol-5-yl)-Carbonsäuren der Formel IIIβ durch Umsetzung von entsprechenden Halogen-substituierten Verbindungen der Formel VI (L⁴ = Ha1), insbesondere die Iod- oder Bromverbindungen, in Gegenwart eines Palladium-, Nickel-, Cobalt- oder Rhodium-Übergangsmetallkatalysators und einer Base mit Kohlenmonoxid und Wasser unter erhöhtem Druck erhalten werden.

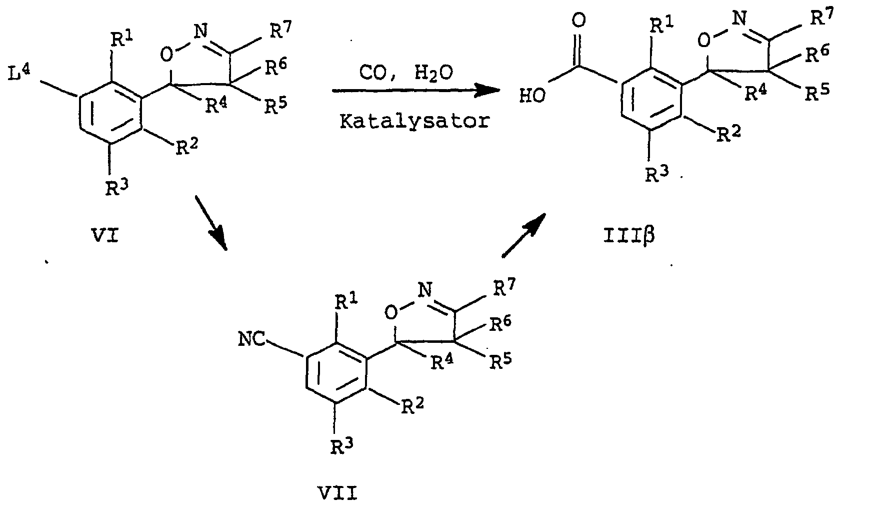

Weiterhin ist es möglich.durch Rosenmund-von Braun-Reaktion Verbindungen der Formel VI in die entsprechenden Nitrile der Formel VII zu überführen (vgl. z.B. Org. Synth. Bd III, 212 (1955)) und diese durch nachfolgende Verseifung in die Verbindungen der Formel IIIß umzuwandeln.

Die Verbindungen der Formel VI bzw. VII sind ebenfalls neu, 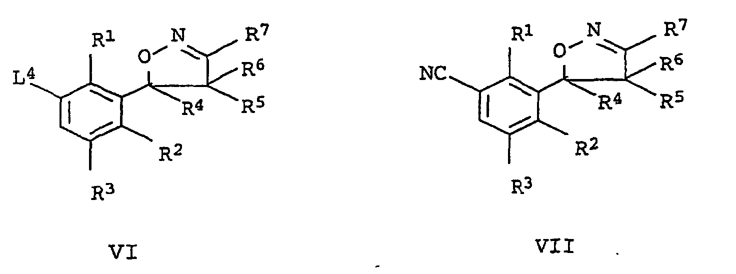 wobei die Varibalen folgende Bedeutung haben:
- R¹, R²: Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R³: Wasserstoff, Halogen oder C₁-C₆-Alkyl;
- R⁴: Wasserstoff oder C₁-C₄-Alkyl;
- R⁵, R⁶: Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄-alkyl, Di-(C₁-C₄-alkyl)-amino-C₁-C₄-alkyl, Di-(C₁-C₄-alkyl)-amino-imino-C₁-C₄-alkyl, Hydroxy-imino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl) amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl,
C₁-C₄-Alkoxy oder C₁-C₄-Halogenalkoxy;
oder
- R⁵ und R⁶: bilden gemeinsam eine C₂-C₆-Alkandiyl-Kette, die einbis vierfach durch C₁-C₄-Alkyl substituiert sein kann und/oder durch Sauerstoff oder einen gegebenenfalls C₁-C₄-Alkyl substituierten Stickstoff unterbrochen sein kann;
- R⁷: Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di(C₁-C₆-Alkyl)amino, Di-(C₁-C₄-alkoxy)methyl, Hydroxyimino-C₁-C₄-alkyl, C₁-C₆-Alkoxyimino-C₁-C₄-alkyl oder COR⁸;
- R⁸: Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
- R⁹: Wasserstoff oder C₁-C₄-Alkyl;
- R¹⁰: C₁-C₄-Alkyl;
- L⁴: Halogen.

Die besonderen Ausführungsformen der Verbindungen der Formel VI bzw. VII in Bezug auf die Variablen R¹ bis R⁷ entsprechen denen der 3-(4,5-Dihydroisoxazol-5-yl)benzoylcyclohexenone der Formel I.

Die Ester der Formel IIIγ bzw. die Halogenverbindungen der Formel VI können durch 1,3-dipolare Cycloaddition von Nitriloxiden an entsprechende Alkene der Formel VIII bzw. IX dargestellt werden.

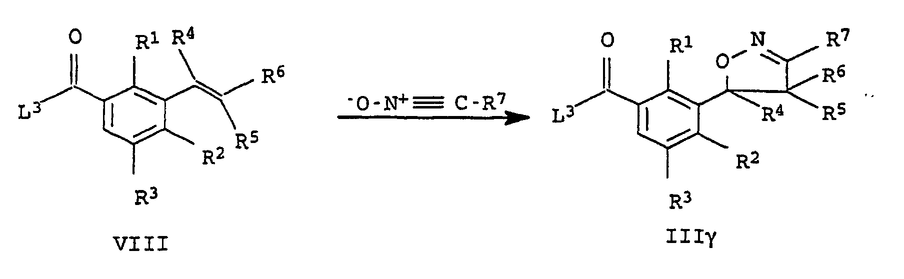

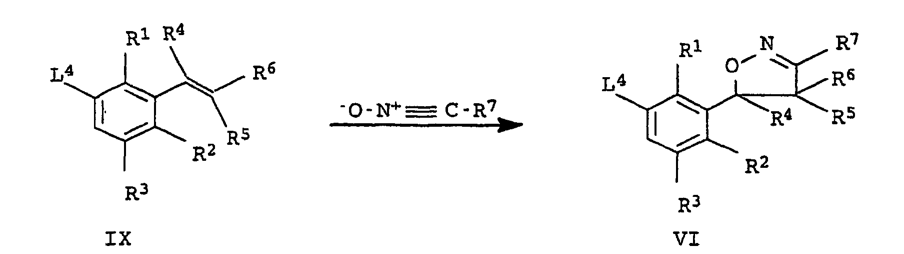

Die Nitriloxide werden in situ auf an sich bekannte Art und Weise erzeugt. Als Edukte dienen hierzu beispielsweise Aldoxime (vgl. z.B. Houben-Weyl X5, S. 858ff) oder Nitroalkane (vgl. z.B. Houben-Weyl E5/2, S. 1594ff). Die Synthese der Verbindungen der Formel VIII bzw. IX ist bekannt (vgl. z.B. WO 98/50337 bzw. WO 98/50366) oder erfolgt in Analogie zu literaturbekannten Verfahren.

### Herstellungsbeispiele:

### 2-[2-Methyl-3-(3-methyl-4,5-dihydroisoxazol-5-yl)-4-methylsulfonyl-benzoyl]-1-hydroxy-cyclohex-1-en-3-on

Zu einer Lösung von 0,86 g (7,7 mmol) 1-Hydroxy-cyclohex-1-en-3-on und 2,13 ml (15,4 mmol) Triethylamin in 50 ml Acetonitril tropfte man bei 0-5°C eine Lösung von 2,3 g (7,7 mmol) 2-Methyl-3-(3-methyl-4,5-dihydroisoxazol-5-yl)-4-methylsulfonylbenzoylchlorid in 50 ml Acetonitril. Nach drei Stunden Rühren bei Raumtemperatur werden weitere 0,64 ml (4,6 mmol) Triethylamin und 0,16 g (15 mmol) Trimethylsilylcyanid zugegeben und zwölf Stunden bei Raumtemperatur gerührt. Das Reaktionsgemisch wurde dann in 800 ml Wasser eingerührt und mit Methylenchlorid gewaschen. Die wäßrige Phase wurde dann mit 10%iger Salzsäure auf einen pH-Wert von 4 gebracht und mit Methylenchlorid extrahiert. Nach Trocknen der organischen Phase entfernte man das Lösungsmittel, nahm den Rückstand in Diethylether auf und digerierte diesen. Der resultierende Rückstand wurde abgesaugt. Man erhielt 1,3 g (3,3 mmol; 43 % d. Th.) der Titelverbindungen.
Fp. 135-140°C

In den Tabellen 2 und 3 sind neben der voranstehenden Verbindung noch weitere Derivate der Formel I aufgeführt, die in analoger Weise hergestellt wurden oder herstellbar sind.

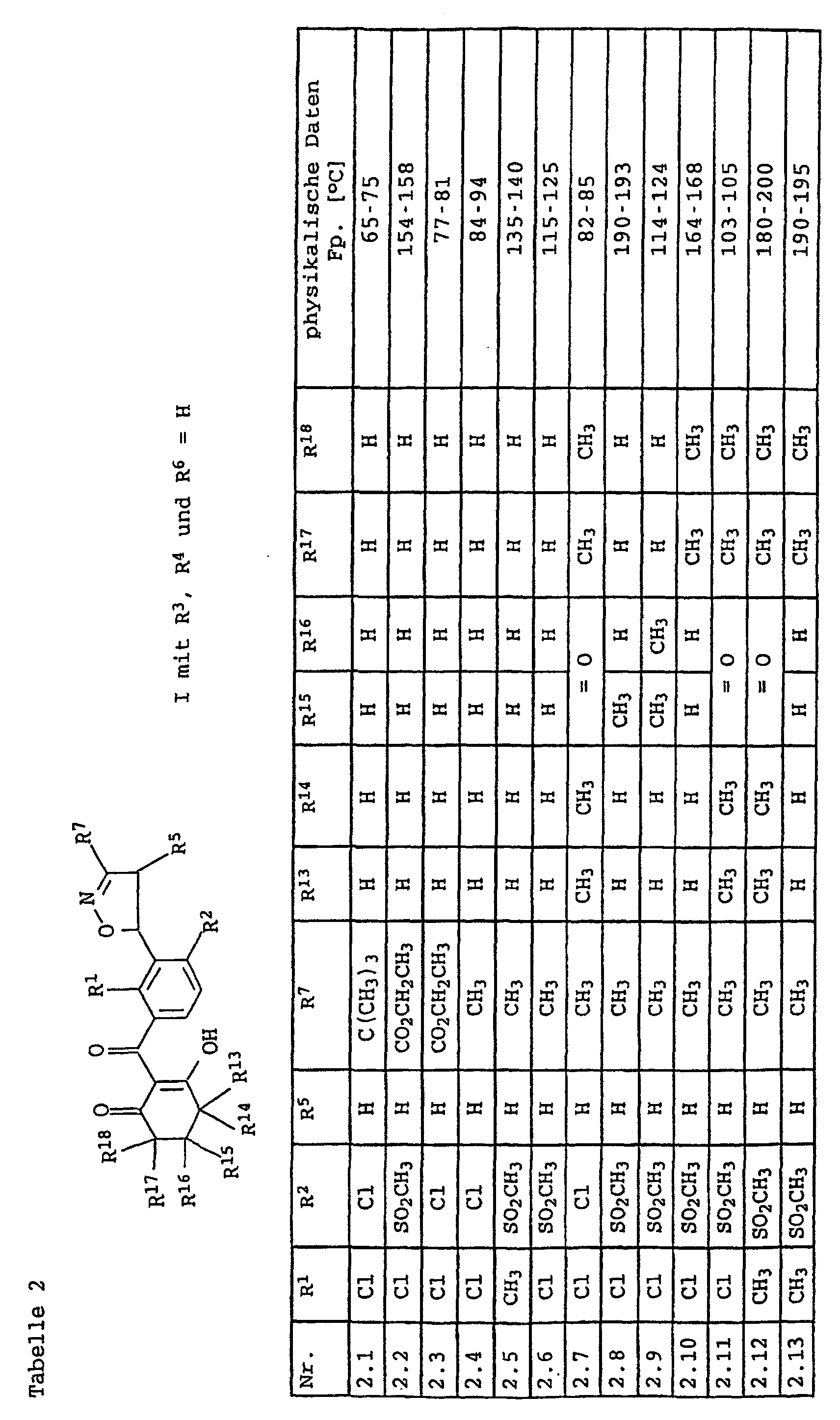

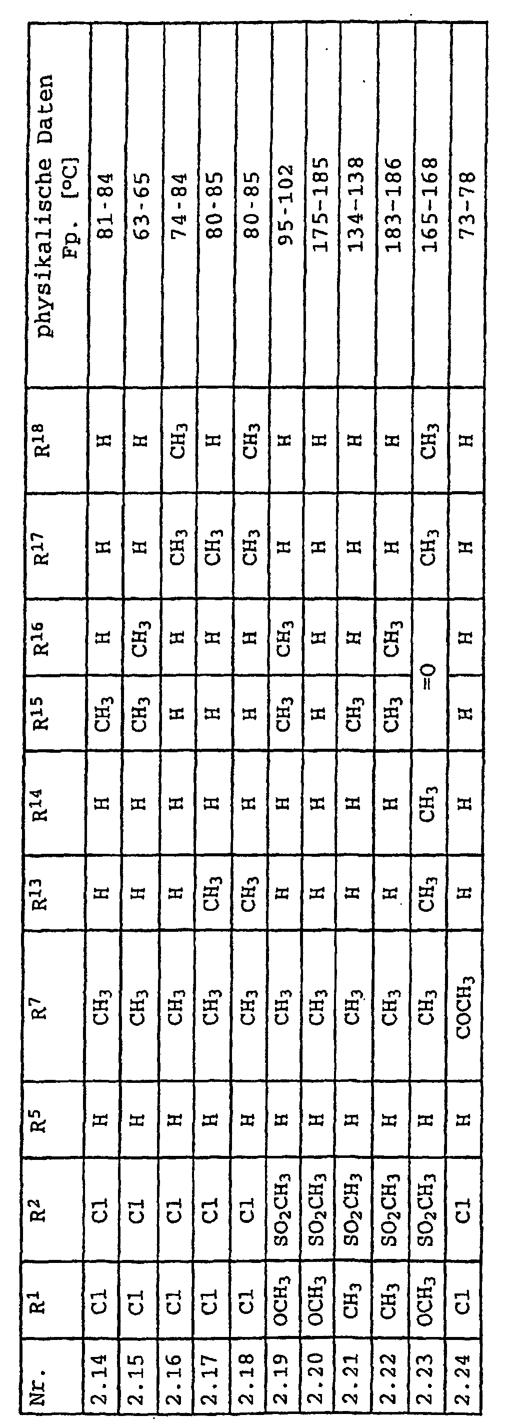

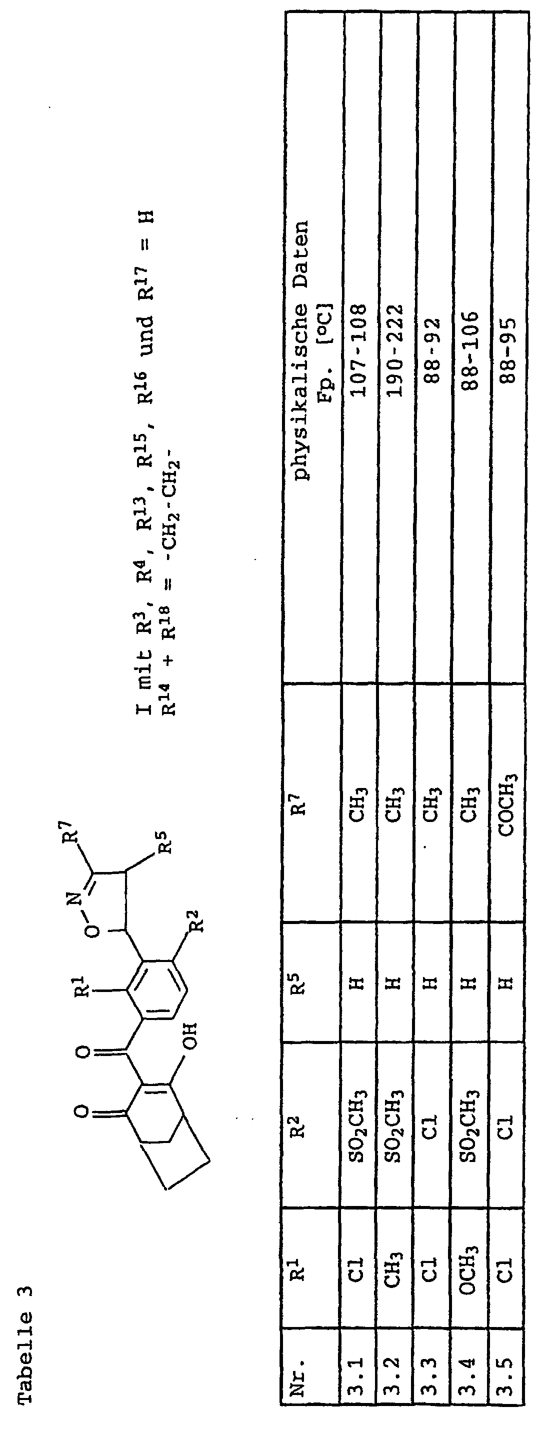

### Nachfolgend sind die Synthesen einiger Ausgangsstoffe aufgeführt:

### 2,4-Dichlor-3-(3-t-butyl-4,5-dihydroisoxazol-5-yl)-benzoesäuremethylester

Zu einer Lösung von 2,6 g (10 mmol) 2,4-Dichlor-3-ethenylbenzoesäuremethylester und 1 g (10 mmol) 2,2-Dimethylpropionaldoxim in 100 ml Dichlormethan wurden bei Raumtemperatur 17 ml einer 12 %igen wäßrigen Natriumhypochloritlösung unter heftigen Rühren getropft. Nach jeweils 2 Stunden Rühren wurden zweimal weitere 0,5 g (5 mmol) des Oxims und 9 ml der Hypochloritlösung zugegeben. Es wurde weiter 12 Stunden bei Raumtemperatur gerührt und dann das Reaktionsgemisch in 350 ml Wasser eingerührt. Nach Extraktion mit Dichlormethan, Trocknen und Entfernen des Lösungsmittels wurde der Rückstand chromatographiert.

Ausbeute: 1,5 g (45 % d. Th.) gelbes Harz

2-Chlor-3-(3-ethoxycarbonyl-4,5-dihydroisoxazol-5-yl)-4-methylsulfonylbenzoesäuremethylester.

Zu einer Lösung von 8,5 g (31 mmol) 2-Chlor-3-ethenyl-4-methylsulfonylbezoesäuremethylester und 7,2 g (46 mmol) 2-Chlor-2-hydroxyiminoessigsäureethylester in 200 ml Dichlormethan wurden bei Raumtempertur langsam 6,4 ml (46 mmol) Triethylamin getropft. Nach jeweils 2 Stunden Rühren wurden 3 weitere Portionen von jeweils 4,8 g (31 mmol) 2-Chlor-2-hydroxyiminoessigsäureethylester und danach 4,3 ml (31 mmol) Triethylamin zugegeben. Nach weiteren 12 Stunden Rühren bei Raumtemperatur wurde das Reaktionsgemisch in 600 ml Wasser eingerührt. Nach Extraktion mit Dichlormethan, Trocknen und Entfernen des Lösungsmittels wurde der Rückstand chromatographiert.

Ausbeute: 5,7 g (47 % d. Th.) klares Harz.

### 2-Chlor-3-(3-methyl-4,5-dihydroisoxazol-5-yl)-4-methylsulfonylbenzoesäure

a) 2-Chlor-3-(3-methyl-4,5-dihydroisoxazol-5-yl)-4-methylsulfonylbenzoesäuremethylester.
   Zu einer Lösung von 40 g (145 mmol) 2-Chlor-3-ethenyl-4-methylsulfonylbenzoesäuremethylester und 50 g (220 mmol) Di-t-butyldicarbonat in 300 ml Acetonitril gab man eine Spatelspitze 4-Dimethylaminopyridin und tropfte dann langsam 50 g (640 mmol) Nitroethan zu. Nach 12 Stunden Rühren bei Raumtemperatur wurden weitere 32,8 g (145 mmol) Di-t-butyldicarbonat und 22,9 g (290 mmol) Nitroethan zugegeben. Nach weitere 12 Sunden wurde das Lösungsmittel abdestilliert und der Rückstand mit Essigsäureethylester digeriert. Nach Absaugen verblieben 32,1 g der gewünschten Verbindung. Aus der Mutterlauge erhielt man nach Entfernen des Lösungsmittel und anschließender Chromatographie weitere 6,3 g.
   Ausbeute: 39,4 g (82 % d. Th.)
   Fp.: 175°C
b) 2-Chlor-3-(3-methyl-4,5-dihydroisoxazol-5-yl)-4-methylsulfonylbenzoesäure

Zu einer Lösung von 38,4 g (116 mmol) 2-Chlor-3-(3-methyl-45-dihydroisoxazol-5-yl)-4-methylsulfonylbenzoesäuremethylester in 400 ml Methanol und 400 ml Tetrahydrofuran gab man 69,4 g (174 mmol) 10 %ig Natronlauge. Man rührt 12 Stunden bei Raumtemperatur, reduzierte das Lösungsmittelvolumen auf die Hälfte und goß in 1 1 Wasser. Mit 10 %ige Salzsäure wurde der pH-Wert von 1 eingestellt und der sich bildende Niederschlag abgesaugt.
Ausbeute: 35,2 g (96 % d. Th.)
Fp.: >220°C

### 2-Methyl-3-(3-methyl-4,5-dihydroisoxazol-5-yl)-4-methylsulfonylbenzoesäure

a) 2-Methyl-3-(3-methyl-4,5-dihydroisoxazol-5-yl)-methylsulfonylbenzoesäure
   Zu einer Lösung von 13,6 g (50 mmol) 2-Methyl-3-ethenyl-4-methylsulfonyl-brombenzol und 16,7 g (74 mmol) Di-t-butyldicarbonat in 100 ml Acetonitril gab man eine Spatelspitze 4-Dimethylaminopyridin und tropfte dann langsam 17,9 g (229 mmol) Nitroethan zu. Nach 6 Stunden Rühren bei Raumtemperatur wurden weitere 11,1 g (50 mmol) Di-t-butyldicarbonat und 7,8 g (100 mmol) Nitroethan zugegeben und 12 Stunden bei Raumtemperatur gerührt. Nach Entfernen des Lösungsmittels wurde der Rückstand chromatographiert.
   Ausbeute: 6,4 g (38 % d. Th.)
b) 2-Methyl-3-(3-methyl-4,5-dihydroisoxazol-5-yl)-4-methylsulfonylbenzoesäure
   6,4 g (19 mmol) 2-Methyl-3-(3-methyl-4,5-dihydroisoxazol-5 yl)-4-methylsulfonyl-brombenzol wurden in 65 ml Toluol und 30 ml Wasser gelöst und mit 240 mg (1 mmol) Palladiumacetat, 1,1 g (4 mmol) Tricyclohexylphosphan, 810 mg (19 mmol) Lithiumchlorid und 5,4 ml (38 mmol) Triethylamin versetzt. Die entstandene Lösung wurde dann in einem Autoklaven im für 36 Stunden bei 140°C unter einen Kohlenmonoxiddruck von 20 bar gerührt. Nach Abkühlen und Entspannen wurden unlösliche Bestandteile abfiltriert und die Phasen getrennt. Die organische Phase wurde anschließend zweimal mit Wasser das etwas Triethylamin enthielt, extrahiert. Dann wurden die vereinigten wäßrigen Phasen mit 10 % Salzsäure auf pH 1 gebracht und der entstandene Niederschlag abfiltriert.
   Ausbeute: 2,5 g (44 % d. Th.)
   Fp. : 199-205°C

In der nachfolgende Tabelle 4 sind neben den voranstehend beschriebenen Verbindungen weitere 3-(4,5-Dihydroisoxazol-5-yl)-Carbonsäurederivate der Formel III aufgeführt, die in analoger Weise hergestellt werden oder herstellbar sind.

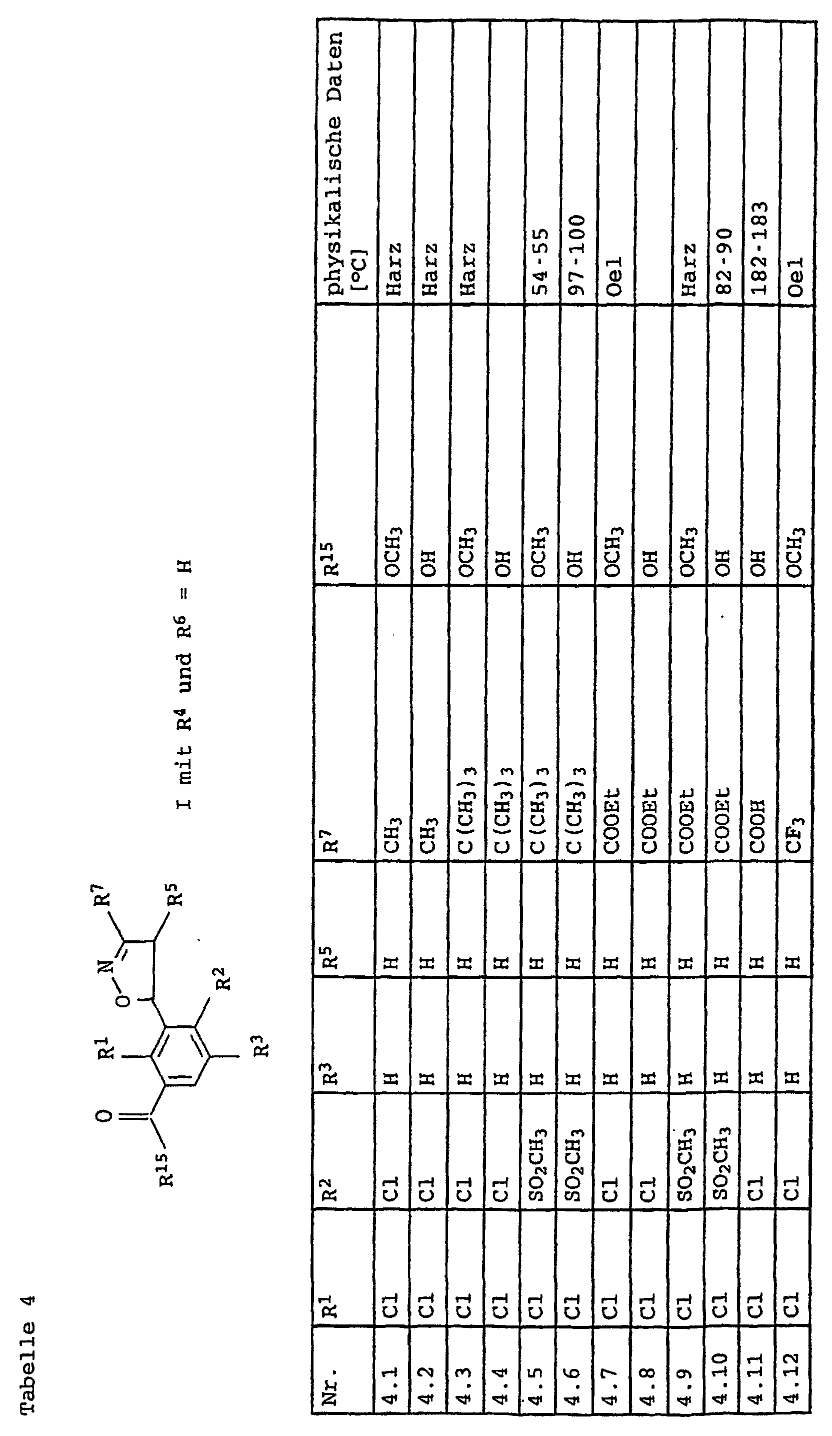

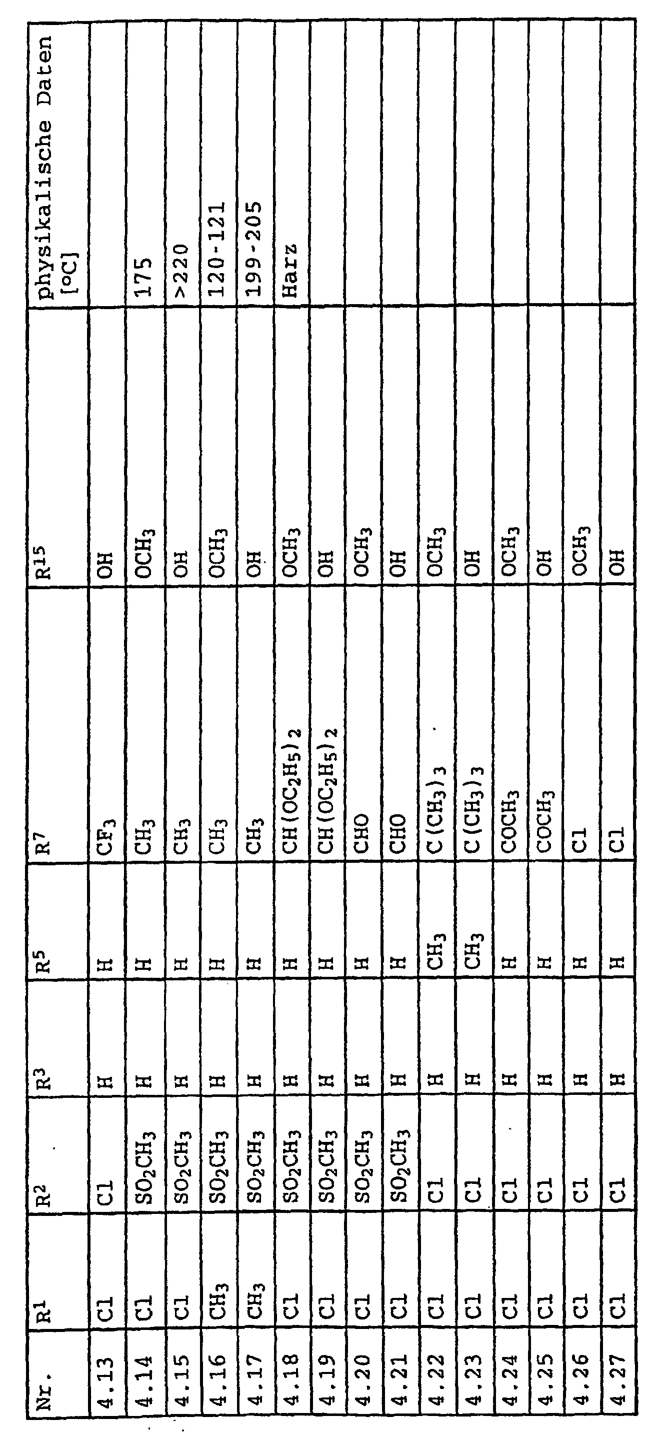

Die 3-(4,5-Dihydroisoxazol-5-yl)benzoylcyclohexenone der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

### Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 3-(4,5-Dihydroisoxazol-5-yl)benzoylcyclohexenone als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe.und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.5 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 2.9 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile des Wirkstoffs Nr. 2.5 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 2.9 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 2.5 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.9 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.5 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.4 wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf-, im Nachauflaufverfahren oder zusammen mit dem Saatgut einer Kulturpflanze erfolgen. Es besteht auch die Möglichkeit, die Verbindungen der Formel I bzw. die herbiziden Mittel dadurch zu applizieren, daß mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 3-(4,5-Dihydroisoxazol-5-yl)benzoylcyclohexenone der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der 3-(4,5-Dihydroisoxazol-5-yl)benzoylcyclohexenone der Formel I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 125 bzw. 62,5 g/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retroflexus | Zurückgekrümmter Ackerfuchsschwanz | redroot pigweed |
| Chenopodium album | Weißer Gänsefuß | lambsquaters (goosefoot) |
| Digitaria Sanguinalis | Blutfingerhirse | hairy fingergrass |
| Panicum dichotomiflorum | Gabelblütige Hirse | fall panicum |
| Polygonum persicaria | Flohknöterich | ladysthumb |
| Solanum nigrum | Schwarzer Nachtschatten | black nightshade |
| Stellaria media | Vogelsternmiere | common chickweed |
| Matricaria | Kamille | false chamomile |
| Brachiaria platyphylla | | broadleaf signal grass |
| Lamium amplexicanle | Stengelumfassende Taubnessel | Renbit |

Bei einer Aufwandmenge von 125 bzw. 62,5 g/ha a.s. zeigte die Verbindung 2.4 sehr gute Wirkung gegen Brachiaria platyphylla, weißen Gänsefuß, Lamium amplexicanle, Kamille und Vogelsternmiere. Ebenso bekämpfte die Verbindung 2.9 unter analogen Bedingungen zurückgekrümmten Ackerfuchsschwanz, weißer Gänsefuß, Blutfingerhirse, Flohknöterich und gabelblutige Hirse sehr gut. Verbindung 2.5 zeigt im Vorauflauf der Aufwandmengen von 125 bzw. 62,5 g/ha einen hohen Bekämpfungsgrad von zurückgekrümmtem Ackerfuchsschwanz, Blutfingerhirse, weißer Gänsefuß und schwarzer Nachschatten.

## Patentansprüche

1. 3-(4,5-Dihydroisoxazol-5-yl)benzoylcyclohexenone der Formel I 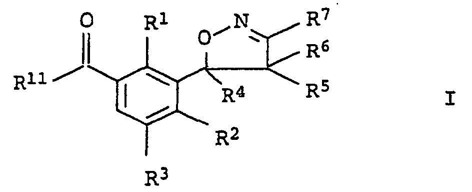 in der die Variablen folgende Bedeutungen haben:
R¹, R² Wasserstoff, Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
R³ Wasserstoff, Halogen oder C₁-C₆-Alkyl;
R⁴ Wasserstoff oder C₁-C₄-Alkyl;
R⁵, R⁶ Wasserstoff, Halogen, Cyano, Nitro, C₁-C₄-Alkyl, C₁-C₄-Alkoxy-C₁-C₄-alkyl, Di-(C₁-C₄-alkoxy)-C₁-C₄alkyl, Di-(C₁-C₄-alkyl)-amino-C₁-C₄-alkyl, Di-(C₁-C₄-alkyl)-amino-imino-C₁-C₄-alkyl, Hydroxyimino-C₁-C₄-alkyl, C₁-C₆-Alkoxyimino-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl,
C₁-C₄-Alkoxy oder C₁-C₄-Halogenalkoxy;
oder
R⁵ und R⁶ bilden gemeinsam eine C₂-C₆-Alkandiyl-Kette, die ein- bis vierfach durch C₁-C₄-Alkyl substituiert sein kann und/oder durch Sauerstoff oder einen gegebenenfalls C₁-C₄-Alkyl substituierten Stickstoff unterbrochen sein kann;
R⁷ Halogen, Cyano, Hydroxy, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, Amino, C₁-C₆-Alkylamino, Di-(C₁-C₆-alkyl)amino, Di- (C₁-C₄-alkoxy)methyl, Hydroxyimino-C₁-C₄-alkyl, C₁-C₆-Alkoxyimino-C₁₋C₄-alkyl oder COR⁸;
R⁸ Wasserstoff, C₁-C₄-Alkyl, C₁-C₄-Halogenalkyl, Hydroxy, C₁-C₄-Alkoxy, C₁-C₄-Alkoxy-C₂-C₄-alkoxy, C₁-C₄-Halogenalkoxy, C₃-C₆-Alkenyloxy, C₃-C₆-Alkinyloxy oder NR⁹R¹⁰;
R⁹ Wasserstoff oder C₁-C₄-Alkyl;
R¹⁰ C₁-C₄-Alkyl;
R¹¹ ein in 2-Stellung verknüpftes Cyclohexenon der Formel II 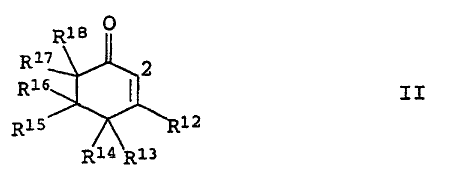 wobei
R¹² Hydroxy, Mercapto, Halogen, OR¹⁹, SR¹⁹, SOR²⁰ oder SO₂R²⁰;
R¹³, R¹⁷ Wasserstoff, C₁-C₄-Alkyl, C₁-C₄-Alkylthio oder C₁-C₄-Alkoxycarbonyl;
R¹⁴, R¹⁶, R¹⁸ Wasserstoff oder C₁-C₄-Alkyl;
R¹⁵ Wasserstoff, Hydroxy, Halogen, C₁-C₆-Alkyl, C₁-C₆-Halogen-alkyl, Di(C₁-C₆-alkoxy)methyl, (C₁-C₆-Alkoxy) (C₁-C₆-alkylthio)methyl, Di(C₁-C₆-alkylthio)methyl, C₁-C₆-Alkoxy, C₁-C₆-Halogenalkoxy, C₁-C₆-Alkylthio, C₁-C₆-Halogenalkylthio, C₁-C₆-Alkoxycarbonyl, C₁-C₆-Halogenalkoxycarbonyl, 1,3-Dioxolan-2-yl, 1,3-Dioxan-2-yl, 1,3-Oxathiolan-2-yl, 1,3-Oxathian-2-yl, 1,3-Dithiolan-2-yl oder 1,3-Dithian-2-yl steht, wobei die sechs letztgenannten Reste einen, zwei oder drei unter C₁-C₄-Alkyl ausgewählte Substituenten tragen könnnen;
oder
R¹³ und R¹⁴ oder R¹⁷ und R¹⁸ gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
R¹⁴ und R¹⁵ oder R¹⁵ und R¹⁸ gemeinsam für eine chemische Bindung oder C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹⁴ und R¹⁸ gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹⁵ und R¹⁶ gemeinsam für -O-(CH₂)ₚ-O-, -O-(CH₂)ₚ-S-, -S-(CH₂)ₚ-S-, -O-(CH₂)_{q}- oder -S-(CH₂)_{q}- stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹⁵ und R¹⁶ gemeinsam für ein Sauerstoffatom stehen;
R¹⁹ C₁-C₆-Alkyl, C₃-C₆-Alkenyl, C₃-C₆-Halogenalkenyl, C₃-C₆-Alkinyl, C₃-C₆-Cycloalkyl, C₁-C₂₀-Alkylcarbonyl, C₂-C₂₀-Alkenylcarbonyl, C₂-C₆-Alkinylcarbonyl, C₁-C₆-Alkoxycarbonyl, C₃-C₆-Alkenyloxycarbonyl, C₃-C₆-Alkinyloxycarbonyl, C₁-C₆-Alkylthiocarbonyl, C₁-C₆-Alkylaminocarbonyl; C₃-C₆-Alkenylaminocarbonyl, C₃-C₆-Alkinylaminocarbonyl, N,N-Di(C₁-C₆-alkyl)aminocarbonyl, N-(C₃-C₆-Alkenyl)-N-(C₁-C₆-alkyl)aminocarbonyl, N-(C₃-C₆-Alkinyl)-N-(C₁-C₆-alkyl) aminocarbonyl, N- (C₁-C₆-Alkoxy) -N- (C₁-C₆-alkyl)aminocarbonyl, N,N-Di(C₁-C₆-alkyl)aminothiocarbonyl, C₁-C₆-Alkoxyimino-C₁-C₆-alkyl steht, wobei die genannten Alkyl-, Alkoxy- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können;
Phenyl, Phenyl-C₁-C₆-alkyl, Phenylcarbonyl-C₁-C₆-alkyl, Phenylcarbonyl, Phenoxycarbonyl, Phenoxythiocarbonyl, Heterocycyl, Heterocyclyl-C₁-C₆-alkyl, Heterocyclylcarbonyl-C₁-C₆-alkyl, Heterocyclylcarbonyl, Heterocyclyloxycarbonyl, Heterocyclyloxythiocarbonyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
R²⁰ C₁-C₆-Alkyl, C₃-C₆-Alkenyl, C₃-C₆-Halogenalkenyl, C₃-C₆-Alkinyl oder C₃-C₆-Cycloalkyl steht, wobei die genannten Alkyl- und Cycloalkylreste partiell oder vollständig halogeniert sein können und/oder einen, zwei oder drei unter Cyano, C₁-C₄-Alkoxy, C₁-C₄-Alkylthio, Di(C₁-C₄-alkyl)amino, C₁-C₄-Alkylcarbonyl, C₁-C₄-Alkoxycarbonyl, C₁-C₄-Alkoxy-C₁-C₄-alkoxycarbonyl, C₁-C₄-Alkylaminocarbonyl, N,N-Di-(C₁-C₄-alkyl)aminocarbonyl oder C₃-C₆-Cycloalkyl ausgewählte Substituenten tragen können;' Phenyl, Phenyl-C₁-C₆-alkyl, Phenylcarbonyl-C₁-C₆-alkyl, Heterocycyl, Heterocyclyl-C₁-C₆-alkyl oder Fieterocyclylcarbonyl-C₁-C₆-alkyl, wobei der Phenyl- oder Heterocyclylrest der genannten Reste partiell oder vollständig halogeniert sein kann und/oder einen, zwei oder drei unter Nitro, Cyano, C₁-C₄-Alkyl, C₁-C₄-Halogenalkyl, C₁-C₄-Alkoxy oder C₁-C₄-Halogenalkoxy ausgewählte Substituenten tragen kann;
p 2, 3 oder 4;
q 1, 2, 3, 4 oder 5;
bedeuten;
sowie deren landwirtschaftlich brauchbaren Salze.

2. 3-(4,5-Dihydroisoxazol-5-yl)-benzoylcyclohexenone der Formel I nach Anspruch 1, wobei
R¹, R² Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alyklsulfinyl, C₁-C₆-Halogenalkylsulfinyl, C₁-C₆-Alkylsulfonyl oder C₁-C₆-Halogenalkylsulfonyl;
bedeuten.

3. 3-(4,5-Dihydroisoxazol-5-yl)-benzoylcyclohexenone der Formel I nach den Ansprüchen 1 oder 2, wobei R³ Wasserstoff bedeutet.

4. 3-(4,5-Dihydroisoxazol-5-yl)-benzoylcyclohexenone der Formel I nach den Ansprüchen 1 bis 3, wobei
R⁵ Wasserstoff, Halogen, Cyano, Nitro, C₁-C₄-Alkyl, C₁-C₄-Alkoxy-C₁-C₄-alkyl, C₁-C₄-Alkoxycarbonyl-C₁-C₄-alkyl, C₁-C₄-Alkylthio-C₁-C₄-alkyl, C₁-C₄-Halogenalkyl, C₁-C₄-Cyanoalkyl, C₃-C₈-Cycloalkyl, C₁-C₄-Alkoxy, C₁-C₄-Alkoxy-C₂-C₄-alkoxy, C₁-C₄-Halogenalkoxy, C₁-C₄-Alkylthio, C₁-C₄-Halogenalkylthio, Di-(C₁-C₄-alkyl)-amino, COR⁸, Phenyl oder Benzyl, wobei die beiden letztgenannten Substituenten partiell oder vollständig halogeniert sein können und/oder eine bis drei der folgenden Gruppen tragen können:
Nitro, Cyano, C₁-C₄-Alkyl, C₁-C₄-Halogenalkyl,
C₁-C₄-Alkoxy oder C₁-C₄-Halogenalkoxy;
R⁶ Wasserstoff oder C₁-C₄-Alkyl;
bedeuten.

5. 3-(4,5-Dihydroisoxazol-5-yl)-benzoylcyclohexenone der Formel I nach den Ansprüchen 1 bis 4, wobei R⁵ und R⁶ für Wasserstoff stehen.

6. 3-(4,5-Dihydroisoxazol-5-yl)-benzoylcyclohexenone der Formel I nach den Ansprüchen 1 bis 5, wobei
R¹² Hydroxy, OR¹⁹ oder SR¹⁹; und
R¹⁹ C₁-C₆-Alkyl, C₁-C₂₀-Alkylcarbonyl, C₁-C₆-Alkoxycarbonyl, C₁-C₆-Alkylthiocarbonyl, wobei die Alkylund Alkoxyreste partiell oder vollständig halogeniert sein können und/oder einen, zwei oder drei unter Cyano, C₁-C₄-Alkoxy, C₁-C₄-Alkylthio, C₁-C₄-Alkylcarbonyl, C₁-C₄-Alkoxycarbonyl oder C₃-C₆-Cycloalkyl ausgewählte Substituenten tragen können;
Phenyl, Phenyl-C₁-C₄-alkyl, Phenylcarbonyl-C₁-C₄-alkyl, Phenylcarbonyl, Phenoxycarbonyl, Heterocyclyl, Heterocyclyl-C₁-C₄-alkyl, Heterocyclylcarbonyl-C₁-C₄-alkyl, Heterocyclyloxycarbonyl steht, wobei der Phenyl- oder Heterocyclylrest der genannten Reste partiell oder vollständig halogeniert sein kann und/oder einen, zwei oder drei unter Nitro, Cyano, C₁-C₄-Alkyl,
C₁-C₄-Halogenalkyl, C₁-C₄-Alkoxy oder C₁-C₄-Halogenalkoxy ausgewählten Substituenten tragen kann;
bedeuten.

7. 3-(4,5-Dihydroisoxazol-5-yl)-benzoylcyclohexenone der Formel I nach den Ansprüchen 1 bis 6, wobei R¹² für Hydroxy steht.

8. 3-(4,5-Dihydroisoxazol-5-yl)-benzoylcyclohexenone der Formel I nach den Ansprüchen 1 bis 7, wobei
R¹³ und R¹⁷ unabhängig voneinander Wasserstoff, C₁-C₄-Alkyl oder C₁-C₄-Alkylthio;
R¹⁴, R¹⁶, R¹⁸ unabhängig voneinander Wasserstoff oder Methyl;
R¹⁵ Wasserstoff, Hydroxy, C₁-C₆-Alkyl oder Di(C₁-C₆alkoxy)methyl;
bedeuten;
oder
R¹³ und R¹⁴ oder R¹⁴ und R¹⁵ oder R¹⁵ und R¹⁸ oder R¹⁴ und R¹⁸ oder R¹⁷ und R¹⁸ gemeinsam für C₁-C₅-Alkandiyl stehen, das einen, zwei oder drei unter Halogen, Cyano, C₁-C₄-Alkyl, C₁-C₄-Halogenalkyl oder C₁-C₄-Alkoxycarbonyl ausgewählte Substituenten tragen kann;
oder
R¹⁵ und R¹⁶ gemeinsam für ein Sauerstoffatom stehen.

9. Verfahren zur Herstellung von 3-(4,5-Dihydroisoxazol-5-yl)-benzoylcyclohexenone der Formel I gemäß Anspruch 1, **dadurch gekennzeichnet, daß** man das Cyclohexenon der Formel II mit R¹² = OH, wobei die Variablen R¹³ bis R¹⁸ die unter Anspruch 1 genannten Bedeutungen haben, 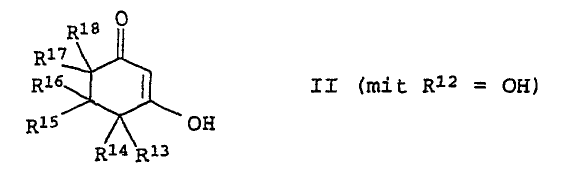 mit einer aktivierten Carbonsäure IIIα oder mit einer Carbonsäure IIIß, 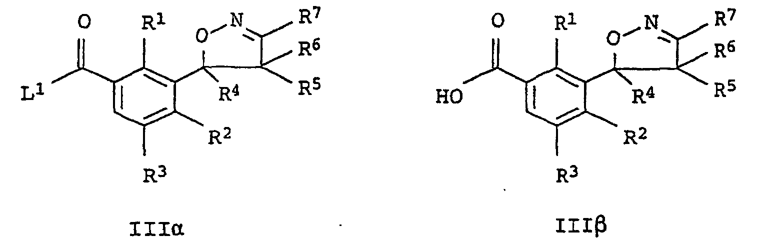 wobei die Variablen R¹ bis R⁷, die unter Anspruch 1 genannten Bedeutungen haben und L¹ für eine nucleophil verdrängbare Abgangsgruppe steht, acyliert und das Acylierungsprodukt in Gegenwart eines Katalysators zu den Verbindungen I (mit R¹² = OH) umlagert.

10. Verfahren zur Herstellung von 3-(4,5-Dihydroisoxazol-5-yl)-benzoylcyclohexenonen der Formel I mit R¹² = OR¹⁹ oder SR¹⁹ nach Anspruch 1, bei dem man eine Verbindung der Formel I mit R¹² = OH oder SH, 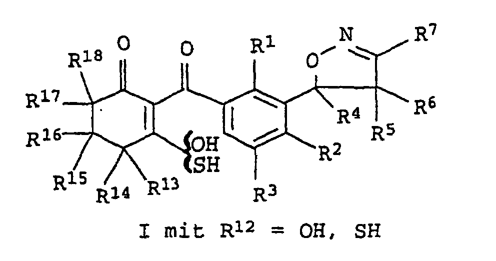 worin R¹ bis R⁷ und R¹³ bis R¹⁸ die in Anspruch 1 angegebene Bedeutung haben, mit einer Verbindung der Formel V
L²-R¹⁹ V
wobei die variable R¹⁹ die in Anspruch 1 angegebene Bedeutung hat und L² für eine nucleophil verdrängbare Abgangsgruppe steht, umsetzt.

11. Verfahren zur Herstellung von 3-(4,5-Dihydroisoxazol-5-yl)-benzoylcyclohexenonen der Formel I mit R¹² = Halogen nach Anspruch 1, bei dem man eine Verbindung der Formel I mit R¹² = OH, 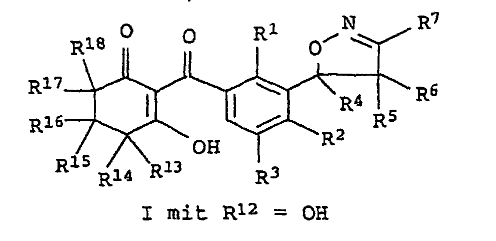 wobei die Variablen R¹ bis R⁷ und R¹³ bis R¹⁸ die in Anspruch 1 genannte Bedeutung haben, mit einem Halogenierungsmittel umsetzt.

12. Verfahren zur Herstellung von 3-(4,5-Dihydroisoxazol-5-yl)-benzoylcyclohexenonen der Formel I mit R¹² = Mercapto, OR¹⁹ oder SR¹⁹ nach Anspruch 1, bei dem man eine Verbindung der Formel I mit R¹² = Halogen, 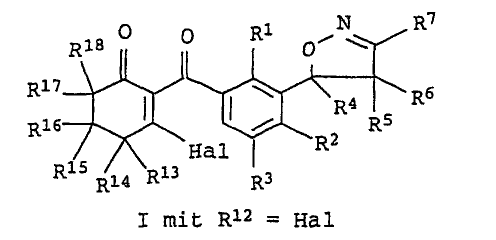 worin die Variablen R¹ bis R⁷ und R¹³ bis R¹⁸ die in Anspruch 1 angegebene Bedeutung haben, mit einer Verbindung der Formel VI
H₂S oder HOR¹⁹ oder HSR¹⁹ VI
wobei R¹⁹ die in Anspruch 1 angegebene Bedeutung hat, gegebenenfalls in Gegenwart einer Base umsetzt.

13. Verfahren zur Herstellung von 3-(4,5-Dihydroisoxazol-5-yl)-benzoylcyclohexenonen der Formel I mit R¹² = SOR²⁰ oder SO₂R²⁰ nach Anspruch 1, bei dem man eine Verbindung der Formel I mit R¹² = SR²⁰, 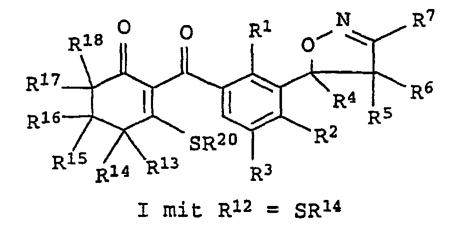 wobei die Variablen R¹ bis R⁷ und R¹³ bis R¹⁸ die in Anspruch 1 genannte Bedeutung haben, mit einem Oxidationsmittel umsetzt.

14. Mittel, enthaltend eine herbizid wirksame Menge mindestens eines 3-(4,5-Dihydroisoxazol-5-yl)-benzoylcyclohexenons der Formel I oder eines landwirtschaftlich brauchbaren Salzes davon gemäß den Ansprüchen 1 bis 8, und für die Formulierung von Pflanzenschutzmitteln übliche Hilfsmittel.

15. Verfahren zur Herstellung von Mitteln gemäß Anspruch 14, **dadurch gekennzeichnet, daß** man eine herbizid wirksame Menge mindestens eines 3-(4,5-Dihydroisoxazol-5-yl)-benzoylcyclohexenons der Formel I oder eines landwirtschaftlich brauchbaren Salzes von I gemäß den Ansprüchen 1 bis 8 und für die Formulierung von Pflanzenschutzmitteln übliche Hilfsmittel mischt.

16. Verfahren zur Bekämpfung von unerwünschtem Pflanzenwuchs, **dadurch gekennzeichnet, dass** man eine herbizid wirksame Menge mindestens eines 3-(4,5-Dihydroisoxazol-5-yl)-benzoylcyclohexenons der Formel I oder eines landwirtschaftlich brauchbaren Salzes davon I gemäß den Ansprüchen 1 bis 8, auf Pflanzen, deren Lebensraum und/oder auf Samen einwirken lässt.

17. Verwendung der 3-(4,5-Dihydroisoxazol-3-yl)-benzoylcyclohexenone der Formel I und/oder deren landwirtschaftlich brauchbaren Salze gemäß den Ansprüchen 1 bis 8 als Herbizide.

## Claims

1. A 3-(4,5-dihydroisoxazol-5-yl)benzoylcyclohexenone of the formula I 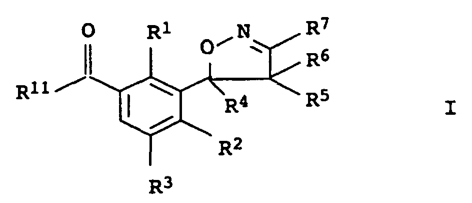 in which the variables have the following meanings:
R¹, R² are hydrogen, nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl;
R³ is hydrogen, halogen or C₁-C₆-alkyl;
R⁴ is hydrogen or C₁-C₄-alkyl;
R⁵, R⁶ are hydrogen, halogen, cyano, nitro, C₁-C₄-alkyl, C₁-C₄-alkoxy-C₁-C₄-alkyl, di(C₁-C₄-alkoxy)-C₁-C₄-alkyl, di(C₁-C₄-alkyl)amino-C₁-C₄-alkyl, di(C₁-C₄-alkyl)aminoimino-C₁-C₄-alkyl, hydroxyimino-C₁-C₄-alkyl, C₁-C₆-alkoxyimino-C₁-C₄-alkyl, C₁-C₄-alkoxycarbonyl-C₁-C₄-alkyl, C₁-C₄-alkylthio-C₁-C₄-alkyl, C₁-C₄-haloalkyl, C₁-C₄-cyanoalkyl, C₃-C₈-cycloalkyl, C₁-C₄-alkoxy, C₁-C₄-alkoxy-C₂-C₄-alkoxy, C₁-C₄-haloalkoxy, C₁-C₄-alkylthio, C₁-C₄-haloalkylthio, di(C₁-C₄-alkyl)amino, COR⁸, phenyl or benzyl, it being possible for the two last-mentioned substituents to be partially or fully halogenated and/or to have attached to them one to three of the following groups:
nitro, cyano, C₁-C₄-alkyl, C₁-C₄-haloalkyl,
C₁-C₄-alkoxy or C₁-C₄-haloalkoxy;
or
R⁵ and R⁶ together form a C₂-C₆-alkanediyl chain which can be mono- to tetrasubstituted by C₁-C₄-alkyl and/or can be interrupted by oxygen or by unsubstituted or C₁-C₄-alkyl-substituted nitrogen;
R⁷ is halogen, cyano, hydroxyl, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl, amino, C₁-C₆-alkylamino, di(C₁-C₆-alkyl)amino, di(C₁-C₄-alkoxy)methyl, hydroxyimino-C₁-C₄-alkyl, C₁-C₆-alkoxyimino-C₁-C₄-alkyl or COR⁸;
R⁸ is hydrogen, C₁-C₄-alkyl, C₁-C₄-haloalkyl, hydroxyl, C₁-C₄-alkoxy, C₁-C₄-alkoxy-C₂-C₄-alkoxy, C₁-C₄-haloalkoxy, C₃-C₆-alkenyloxy, C₃-C₆-alkynyloxy or NR⁹R¹⁰;
R⁹ is hydrogen or C₁-C₄-alkyl;
R¹⁰ is C₁-C₄-alkyl;
R¹¹ is a cyclohexenone of the formula II 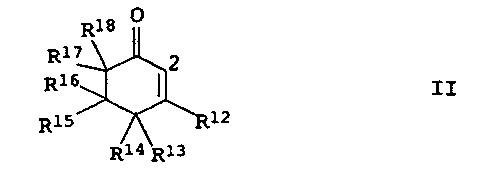 which is linked in the 2-position and where
R¹² is hydroxyl, mercapto, halogen, OR¹⁹, SR¹⁹, SOR²⁰ or SO₂R²⁰;
R¹³, R¹⁷ are hydrogen, C₁-C₄-alkyl, C₁-C₄-alkylthio or C₁-C₄-alkoxycarbonyl;
R¹⁴, R¹⁶, R¹⁸ are hydrogen or C₁-C₄-alkyl;
R¹⁵ is hydrogen, hydroxyl, halogen, C₁-C₆-alkyl, C₁-C₆-haloalkyl, di(C₁-C₆-alkoxy)methyl, (C₁-C₆-alkoxy)(C₁-C₆-alkylthio)methyl, di(C₁-C₆-alkylthio)methyl, C₁-C₆-alkoxy, C₁-C₆-haloalkoxy, C₁-C₆-alkylthio, C₁-C₆-haloalkylthio, C₁-C₆-alkoxycarbonyl, C₁-C₆-haloalkoxycarbonyl,
1,3-dioxolan-2-yl, 1,3-dioxan-2-yl, 1,3-oxathiolan-2-yl, 1,3-oxathian-2-yl, 1,3-dithiolan-2-yl or 1,3-dithian-2-yl, it being possible for the six last-mentioned radicals to have attached to them one, two or three substituents selected from amongst C₁-C₄-alkyl;
or
R¹³ and R¹⁴ or R¹⁷ and R¹⁸ together are C₁-C₅-alkanediyl which can have attached to it one, two or three substituents selected from amongst halogen, cyano, C₁-C₄-alkyl, C₁-C₄-haloalkyl or C₁-C₄-alkoxycarbonyl;
R¹⁴ and R¹⁵ or R¹⁵ and R¹⁸ together are a chemical bond or C₁-C₅-alkanediyl which can have attached to it one, two or three substituents selected from amongst halogen, cyano, C₁-C₄-alkyl, C₁-C₄-haloalkyl or C₁-C₄-alkoxycarbonyl;
or
R¹⁴ and R¹⁸ together are C₁-C₅-alkanediyl which can have attached to it one, two or three substituents selected from amongst halogen, cyano, C₁-C₄-alkyl, C₁-C₄-haloalkyl or C₁-C₄-alkoxycarbonyl;
or
R¹⁵ and R¹⁶ together are -O-(CH₂)ₚ-O-, -O-(CH₂)ₚ-S-, - S-(CH₂)ₚ-S-, -O-(CH₂)_{q}- or -S-(CH₂)_{q}-, each of which can have attached to it one, two or three substituents selected from amongst halogen, cyano, C₁-C₄-alkyl, C₁-C₄-haloalkyl or C₁-C₄-alkoxycarbonyl;
or
R¹⁵ and R¹⁶ together are an oxygen atom;
R¹⁹ is C₁-C₆-alkyl, C₃-C₆-alkenyl, C₃-C₆-haloalkenyl, C₃-C₆-alkynyl, C₃-C₆-cycloalkyl, C₁-C₂₀-alkylcarbonyl, C₂-C₂₀-alkenylcarbonyl, C₂-C₆-alkynylcarbonyl, C₁-C₆-alkoxycarbonyl, C₃-C₆-alkenyloxycarbonyl, C₃-C₆-alkynyloxycarbonyl, C₁-C₆-alkylthiocarbonyl, C₁-C₆-alkylaminocarbonyl, C₃-C₆-alkenylaminocarbonyl, C₃-C₆-alkynylaminocarbonyl, N,N-di(C₁-C₆-alkyl)aminocarbonyl, N-(C₃-C₆-alkenyl)-N-(C₁-C₆-alkyl)aminocarbonyl, N-(C₃-C₆-alkynyl)-N-(C₁-C₆-alkyl)aminocarbonyl, N-(C₁-C₆-alkoxy)-N-(C₁-C₆-alkyl)aminocarbonyl, N,N-di(C₁-C₆-alkyl)aminothiocarbonyl, C₁-C₆-alkoxyimino-C₁-C₆-alkyl, it being possible for the abovementioned alkyl, alkoxy and cycloalkyl radicals to be partially or fully halogenated and/or to have attached to them one, two or three substituents selected from amongst cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, C₁-C₄-alkylaminocarbonyl, N,N-di(C₁-C₄-alkyl)aminocarbonyl or C₃-C₆-cycloalkyl; phenyl, phenyl-C₁-C₆-alkyl, phenylcarbonyl-C₁-C₆-alkyl, phenylcarbonyl, phenoxycarbonyl, phenoxythiocarbonyl, heterocyclyl, heterocyclyl-C₁-C₆-alkyl, heterocyclylcarbonyl-C₁-C₆-alkyl, heterocyclylcarbonyl, heterocyclyloxycarbonyl, heterocyclyloxythiocarbonyl, it being possible for the phenyl or heterocyclyl radical of the abovementioned radicals to be partially or fully halogenated and/or to have attached to it one, two or three substituents selected from amongst nitro, cyano, C₁-C₄-alkyl, C₁-C₄-haloalkyl, C₁-C₄-alkoxy or C₁-C₄-haloalkoxy;
R²⁰ is C₁-C₆-alkyl, C₃-C₆-alkenyl, C₃-C₆-haloalkenyl, C₃-C₆-alkynyl or C₃-C₆-cycloalkyl, it being possible for the abovementioned alkyl and cycloalkyl radicals to be partially or fully halogenated and/or to have attached to them one, two or three substituents selected from amongst cyano, C₁-C₄-alkoxy, C₁-C₄-alkylthio, di(C₁-C₄-alkyl)amino, C₁-C₄-alkylcarbonyl, C₁-C₄-alkoxycarbonyl, C₁-C₄-alkoxy-C₁-C₄-alkoxycarbonyl, C₁-C₄-alkylaminocarbonyl, N,N-di(C₁-C₄-alkyl)aminocarbonyl or C₃-C₆-cycloalkyl; phenyl, phenyl-C₁-C₆-alkyl, phenylcarbonyl-C₁-C₆-alkyl, heterocyclyl, heterocyclyl-C₁-C₆-alkyl or heterocyclylcarbonyl-C₁-C₆-alkyl, it being possible for the phenyl or heterocyclyl radical of the abovementioned radicals to be partially or fully halogenated and/or to have attached to it one, two or three substituents selected from amongst nitro, cyano, C₁-C₄-alkyl, C₁-C₄-haloalkyl, C₁-C₄-alkoxy or C₁-C₄-haloalkoxy;
p is 2, 3 or 4;
q is 1, 2, 3, 4 or 5;
or an agriculturally useful salt thereof.

2. A 3-(4,5-dihydroisoxazol-5-yl)benzoylcyclohexenone of the formula I as claimed in claim 1, where
R¹, R² are nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl.

3. A 3-(4,5-dihydroisoxazol-5-yl)benzoylcyclohexenone of the formula I as claimed in claim 1 or 2, where R³ is hydrogen.

4. A 3-(4,5-dihydroisoxazol-5-yl)benzoylcyclohexenone of the formula I as claimed in any of claims 1 to 3, where
R⁵ is hydrogen, halogen, cyano, nitro, C₁-C₄-alkyl, C₁-C₄-alkoxy-C₁-C₄-alkyl, C₁-C₄-alkoxycarbonyl-C₁-C₄-alkyl, C₁-C₄-alkylthio-C₁-C₄-alkyl, C₁-C₄-haloalkyl, C₁-C₄-cyanoalkyl, C₃-C₈-cycloalkyl, C₁-C₄-alkoxy, C₁-C₄-alkoxy-C₂-C₄-alkoxy, C₁-C₄-haloalkoxy, C₁-C₄-alkylthio, C₁-C₄-haloalkylthio, di(C₁-C₄-alkyl)amino, COR⁸, phenyl or benzyl, it being possible for the two last-mentioned substituents to be partially or fully halogenated and/or to have attached to them one to three of the following groups:
nitro, cyano, C₁-C₄-alkyl, C₁-C₄-haloalkyl,
C₁-C₄-alkoxy or C₁-C₄-haloalkoxy;
R⁶ is hydrogen or C₁-C₄-alkyl.

5. A 3-(4,5-dihydroisoxazol-5-yl)benzoylcyclohexenone of the formula I as claimed in any of claims 1 to 4, where R⁵ and R⁶ are hydrogen.

6. A 3-(4,5-dihydroisoxazol-5-yl)benzoylcyclohexenone of the formula I as claimed in any of claims 1 to 5, where
R¹² is hydroxyl, OR¹⁹ or SR¹⁹; and
R¹⁹ is C₁-C₆-alkyl, C₁-C₂₀-alkylcarbonyl, C₁-C₆-alkoxycarbonyl, C₁-C₆-alkylthiocarbonyl, it being possible for the alkyl and alkoxy radicals to be partially or fully halogenated and/or to have attached to them one, two or three substituents selected from amongst cyano, C₁-C₄-alkoxy, C₁-C₄-alkylthio, C₁-C₄-alkylcarbonyl, C₁-C₄-alkoxycarbonyl or C₃-C₆-cycloalkyl;
phenyl, phenyl-C₁-C₄-alkyl, phenylcarbonyl-C₂-C₄-alkyl, phenylcarbonyl, phenoxycarbonyl, heterocyclyl, heterocyclyl-C₁-C₄-alkyl, heterocyclylcarbonyl-C₁-C₄-alkyl, heterocyclyloxycarbonyl, it being possible for the phenyl or heterocyclyl radical of the abovementioned radicals to be partially or fully halogenated and/or have attached to it one, two or three substituents selected from amongst nitro, cyano, C₁-C₄-alkyl, C₁-C₄-haloalkyl, C₁-C₄-alkoxy or C₁-C₄-haloalkoxy.

7. A 3-(4,5-dihydroisoxazol-5-yl)benzoylcyclohexenone of the formula I as claimed in any of claims 1 to 6, where R¹² is hydroxyl.

8. A 3-(4,5-dihydroisoxazol-5-yl)benzoylcyclohexenone of the formula I as claimed in any of claims 1 to 7, where
R¹³ and R¹⁷ independently of one another are hydrogen, C₁-C₄-alkyl or C₁-C₄-alkylthio;
R¹⁴, R¹⁶, R¹⁸ independently of one another are hydrogen or methyl;
R¹⁵ is hydrogen, hydroxyl, C₁-C₆-alkyl or di(C₁-C₆-alkoxy)methyl;
or
R¹³ and R¹⁴ or R¹⁴ and R¹⁵ or R¹⁵ and R¹⁸ or R¹⁴ and R¹⁸ or R¹⁷ and R¹⁸ together are C₁-C₅-alkanediyl which can have attached to it one, two or three substituents selected from amongst halogen, cyano, C₁-C₄-alkyl, C₁-C₄-haloalkyl or C₁-C₄-alkoxycarbonyl;
or
R¹⁵ and R¹⁶ together are an oxygen atom.

9. A process for the preparation of a 3-(4,5-dihydroisoxazol-5-yl)benzoylcyclohexenone of the formula I as claimed in claim 1, which comprises acylating the cyclohexenone of the formula II where R¹² = OH, where the variables R¹³ to R¹⁸ have the meanings stated in claim 1, 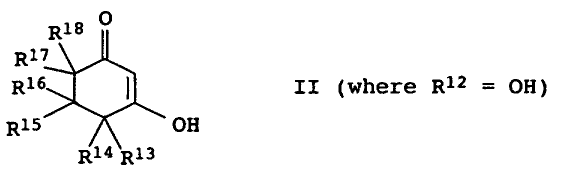 with an activated carboxylic acid IIIα or with a carboxylic acid IIIβ, 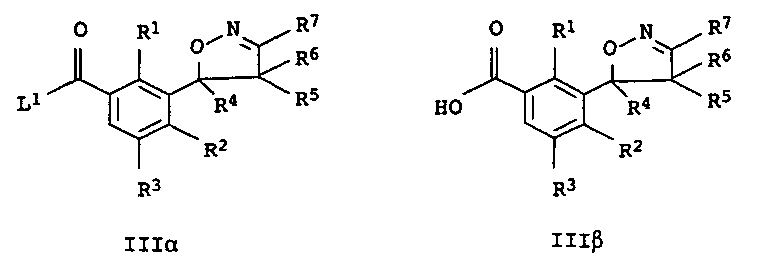 where the variables R¹ to R⁷ have the meanings stated in claim 1 and L¹ is a nucleophilically displaceable leaving group, and subjecting the acylation product to a rearrangement reaction in the presence of a catalyst to give the compounds I (where R¹² = OH).

10. A process for the preparation of a 3-(4,5-dihydroisoxazol-5-yl)benzoylcyclohexenone of the formula I where R¹² = OR¹⁹ or SR¹⁹ as claimed in claim 1, in which a compound of the formula I where R¹² = OH or SH, 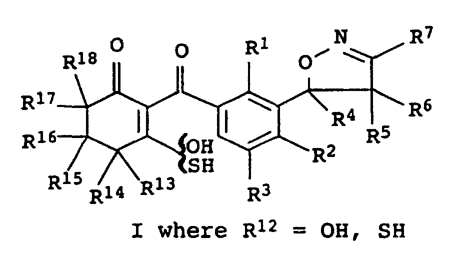 where R¹ to R⁷ and R¹³ to R¹⁸ have the meaning stated in claim 1 is reacted with a compound of the formula V
L²-R¹⁹ V
where the variable R¹⁹ has the meaning stated in claim 1 and L² is a nucleophilically displaceable leaving group.

11. A process for the preparation of a 3-(4,5-dihydroisoxazol-5-yl)benzoylcyclohexenone of the formula I where R¹² = halogen as claimed in claim 1, in which a compound of the formula I where R¹² = OH, 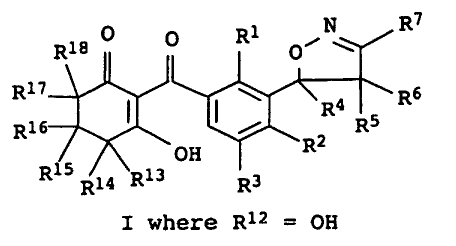 where the variables R¹ to R⁷ and R¹³ to R¹⁸ have the meaning stated in claim 1 is reacted with a halogenating agent.

12. A process for the preparation of a 3-(4,5-dihydroisoxazol-5-yl)benzoylcyclohexenone of the formula I where R¹² = mercapto, OR¹⁹ or SR¹⁹ as claimed in claim 1, in which a compound of the formula I where R¹² = halogen, 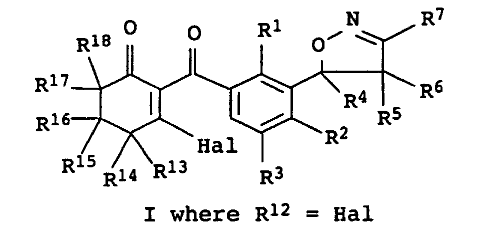 where the variables R¹ to R⁷ and R¹³ to R¹⁸ have the meaning stated in claim 1 is reacted with a compound of the formula VI
H₂S or HOR¹⁹ or HSR¹⁹ VI
where R¹⁹ has the meaning stated in claim 1, in the presence or absence of a base.

13. A process for the preparation of a 3-(4,5-dihydroisoxazol-5-yl)benzoylcyclohexenone of the formula I where R¹² = SOR²⁰ or SO₂R²⁰ as claimed in claim 1, in which a compound of the formula I where R¹² = SR²⁰, 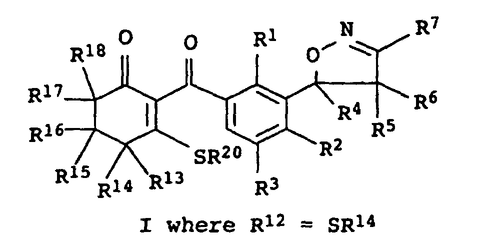 where the variables R¹ to R⁷ and R¹³ to R¹⁸ have the meaning stated in claim 1, is reacted with an oxidant.

14. A composition comprising a herbicidally active amount of at least one 3-(4,5-dihydroisoxazol-5-yl)benzoylcyclohexenone of the formula I or of an agriculturally useful salt thereof as claimed in claims 1 to 8 and adjuvants conventionally used for the formulation of crop protection products.

15. A process for the preparation of a composition as claimed in claim 14, which comprises mixing a herbicidally active amount of at least one 3-(4,5-dihydroisoxazol-5-yl)benzoylcyclohexenone of the formula I or of an agriculturally useful salt of I as claimed in claims 1 to 8 and adjuvants conventionally used for the formulation of crop protection products.

16. A method of controlling undesired vegetation, which comprises allowing a herbicidally active amount of at least one 3-(4,5-dihydroisoxazol-5-yl)benzoylcyclohexenone of the formula I or of an agriculturally useful salt thereof as claimed in claims 1 to 8 to act on plants, their environment and/or on seeds.

17. The use of a 3-(4,5-dihydroisoxazol-3-yl)benzoylcyclohexenone of the formula I and/or its agriculturally useful salts as claimed in claims 1 to 8 as herbicide.

## Revendications

1. 3-(4,5-dihydroisoxazol-5-yl)benzoylcyclohexénones de formule I 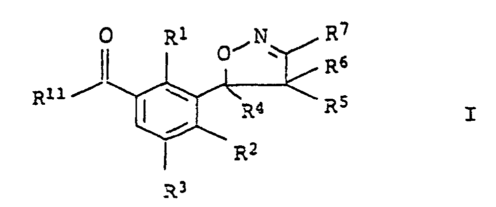 dans laquelle les variables prennent les significations suivantes :
R¹, R² représentent un atome d'hydrogène, un groupe nitro, un atome d'halogène, un groupe cyano, alkyle en C₁ à C₆, halogénoalkyle en C₁ à C₆, alcoxy en C₁ à C₆, halogénoalcoxy en C₁ à C₆, alkylthio en C₁ à C₆, halogénoalkylthio en C₁ à C₆, alkylsulfinyle en C₁ à C₆, halogénoalkylsulfinyle en C₁ à C₆, alkylsulfonyle en C₁ à C₆, ou halogénoalkylsulfonyle en C₁ à C₆;
R³ représente un atome d'hydrogène, d'halogène ou un groupe alkyle en C₁ à C₆;
R⁴ représente un atome d'hydrogène ou un groupe alkyle en C₁ à C₄;
R⁵, R⁶ représentent un atome d'hydrogène, d'halogène, un groupe cyano, nitro, alkyle en C₁ à C₄ (alcoxy en C₁ à C₄)-(alkyle en C₁ à C₄), di-(alcoxy en C₁ à C₄)-(alkyle en C₁ à C₄), di-(alkyle en C₁ à C₄)-amino-(alkyle en C₁ à C₄), di-(alkyle en C₁ à C₄)-amino-imino-(alkyle en C₁ à C₄), hydroxyimino-(alkyle en C₁ à C₄), (alcoxy en C₁ à C₆)-imino-(alkyle en C₁ à C₄), (alcoxy en C₁ à C₄)-carbonyl-(alkyle en C₁ à C₄), (alkyle en C₁ à C₄)-thio-(alkyle en C₁ à C₄), halogénoalkyle en C₁ à C₄, cyano-(alkyle en C₁ à C₄), cycloalkyle en C₃ à C₈, alcoxy en C₁ à C₄, (alcoxy en C₁ à C₄)-(alcoxy en C₂ à C₄), halogénoalcoxy en C₁ à C₄, alkylthio en C₁ à C₄, halogénoalkylthio en C₁ à C₄, di-(alkyle en C₁ à C₄)-amino, COR⁸, phényle ou benzyle, les deux substituants cités en dernier pouvant être partiellement ou complètement halogénés et/ou pouvant porter un à trois des groupes suivants :
un groupe nitro, cyano, alkyle en C₁ à C₄, halogénoalkyle en C₁ à C₄, alcoxy en C₁ à C₄ ou halogénoalcoxy en C₁ à C₄;
ou bien
R⁵ et R⁶ forment ensemble une chaîne alcanediyle en C₂ à C₆, qui peut être substituée une fois à quatre fois par un groupe alkyle en C₁ à C₄ et/ou peut être interrompue par un atome d'oxygène ou un atome d'azote éventuellement substitué par un groupe alkyle en C₁ à C₄;
R⁷ représente un atome d'halogène, un groupe cyano, hydroxy, alkyle en C₁ à C₆, halogénoalkyle en C₁ à C₆, alcoxy en C₁ à C₆, halogénoalcoxy en C₁ à C₆, alkylthio en C₁ à C₆, halogénoalkylthio en C₁ à C₆, alkylsulfinyle en C₁ à C₆, halogénoalkylsulfinyle en C₁ à C₆, alkylsulfonyle en C₁ à C₆, halogénoalkylsulfonyle en C₁ à C₆, amino, alkylamino en C₁ à C₆, di-(alkyle en C₁ à C₆)-amino, di-(alcoxy en C₁ à C₄)méthyle, hydroxyimino-(alkyle en C₁ à C₄), (alcoxy en C₁ à C₆)-imino-(alkyle en C₁ à C₄) ou COR⁸;
R⁸ représente un atome d'hydrogène, un groupe alkyle en C₁ à C₄, halogénoalkyle en C₁ à C₄, hydroxy, alcoxy en C₁ à C₄, (alcoxy en C₁ à C₄)-(alcoxy en C₂ à C₄), halogénoalcoxy en C₁ à C₄, alcényloxy en C₃ à C₆, alcynyloxy en C₃ à C₆, ou NR⁹R¹⁰;
R⁹ représente un atome d'hydrogène ou un groupe alkyle en C₁ à C₄;
R¹⁰ représente un groupe alkyle en C₁ à C₄;
R¹¹ représente un groupe cyclohexénone lié à la position 2, de formule II 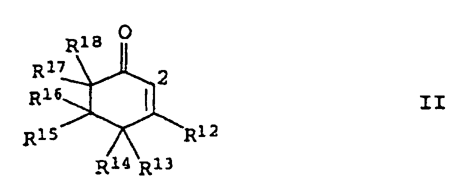 dans laquelle
R¹² représente un groupe hydroxy, mercapto, un atome d'halogène, OR¹⁹, SR¹⁹, SOR²⁰, ou SO₂R²⁰;
R¹³, R¹⁷ représentent un atome d'hydrogène, un groupe alkyle en C₁ à C₄, alkylthio en C₁ à C₄ ou (alcoxy en C₁ à C₄)-carbonyle;
R¹⁴, R¹⁶, R¹⁸ représentent un atome d'hydrogène ou un groupe alkyle en C₁ à C₄;
R¹⁵ représente un atome d'hydrogène, un groupe hydroxy, un atome d'halogène, un groupe alkyle en C₁ à C₆, halogénoalkyle en C₁ à C₆, di-(alcoxy en C₁ à C₆)méthyle, (alcoxy en C₁ à C₆)-(alkylthio en C₁ à C₆)méthyle, di(alkylthio en C₁ à C₆)méthyle, alcoxy en C₁ à C₆, halogénoalcoxy en C₁ à C₆, alkylthio en C₁ à C₆, halogénoalkylthio en C₁ à C₆, (alcoxy en C₁ à C₆)carbonyle, (halogénoalcoxy en C₁ à C₆)carbonyle, 1,3-dioxolan-2-yle, 1,3-dioxan-2-yle, 1,3-oxathiolan-2-yle, 1,3-oxathian-2-yle, 1,3-dithiolan-2-yle, ou 1,3-dithian-2-yle, les six résidus cités en dernier pouvant porter un, deux ou trois des substituants choisis parmi un groupe alkyle en C₁ à C₄;
ou
R¹³ et R¹⁴ ou R¹⁷et R¹⁸ représentent conjointement un groupe alcanediyle en C₁ à C₅, qui peut porter un, deux, ou trois des substituants choisis parmi un atome d'halogène, un groupe cyano, alkyle en C₁ à C₄, halogénoalkyle en C₁ à C₄ ou (alcoxy en C₁ à C₄)carbonyle;
R¹⁴ et R¹⁵ ou R¹⁵et R¹⁸ représentent conjointement une liaison chimique ou un groupe alcane-diyle en C₁ à C₅, qui peut porter un, deux ou trois des substituants choisis parmi un atome d'halogène, un groupe cyano, alkyle en C₁ à C₄, halogénoalkyle en C₁ à C₄ ou (alcoxy en C₁ à C₄)carbonyle;
ou
R¹⁴ et R¹⁸ représentent conjointement un groupe alcane-diyle en C₁ à C₅, qui peut porter un, deux ou trois des substituants choisis parmi un atome d'halogène, un groupe cyano, alkyle en C₁ à C₄, halogénoalkyle en C₁ à C₄ ou (alcoxy en C₁ à C₄)carbonyle;
ou
R¹⁵ et R¹⁶ représentent conjointement -O-(CH₂)ₚ-O-, -O-(CH₂)ₚ-S-, - S-(CH₂)ₚ-S-, -O-(CH₂)_{q}- ou -S-(CH₂)_{q}-, qui peuvent porter un, deux ou trois des substituants choisis parmi un atome d'halogène, un groupe cyano, alkyle en C₁ à C₄, halogénoalkyle en C₁ à C₄ ou (alcoxy en C₁ à C₄)carbonyle;
ou
R¹⁵ et R¹⁶ représentent conjointement un atome d'oxygène;
R¹⁹ représente un groupe alkyle en C₁ à C₆, alcényle en C₃ à C₆, halogénoalcényle en C₃ à C₆, alcynyle en C₃ à C₆, cycloalkyle en C₃ à C₆, (alkyle en C₁ à C₂₀)carbonyle, (alcényle en C₂ à C₂₀)carbonyle, (alcynyle en C₂ à C₆)carbonyle, (alcoxy en C₁ à C₆)carbonyle, (alcényle en C₃ à C₆)oxycarbonyle, (alcynyle en C₃ à C₆)oxycarbonyle, (alkyle en C₁ à C₆)thiocarbonyle, (alkyle en C₁ à C₆)aminocarbonyle, (alcényle en C₃ à C₆)aminocarbonyle, (alcynyle en C₃ à C₆)aminocarbonyle, N,N-di(alkyle en C₁ à C₆)aminocarbonyle, N-(alcényle en C₃ à C₆)-N-(alkyle en C₁ à C₆)aminocarbonyle, N-(alcynyle en C₃ à C₆)-N-(alkyle en C₁ à C₆)aminocarbonyle, N-(alcoxy en C₁ à C₆)-N-(alkyle en C₁ à C₆)aminocarbonyle, N,N-di(alkyle en C₁ à C₆)aminothiocarbonyle, (alcoxy en C₁ à C₆)imino-(alkyle en C₁ à C₆), les résidus alkyle, alcoxy et cycloalkyle cités pouvant être partiellement ou complètement halogénés et/ou pouvant porter un, deux ou trois des substituants choisis parmi un groupe cyano, alcoxy en C₁ à C₄, alkylthio en C₁ à C₄, di(alkyle en C₁ à C₄)amino, (alkyle en C₁ à C₄)carbonyle, (alcoxy en C₁ à C₄)carbonyle, (alcoxy en C₁ à C₄)-(alcoxy en C₁ à C₄)carbonyle, (alkyle en C₁ à C₄)aminocarbonyle, N,N-di-(alkyle en C₁ à C₄)aminocarbonyle ou cycloalkyle en C₃ à C₆;
un groupe phényle, phényl-(alkyle en C₁ à C₆), phénylcarbonyl-(alkyle en C₁ à C₆), phénylcarbonyle, phénoxycarbonyle, phénoxythiocarbonyle, hétérocyclyle, hétérocyclyl-(alkyle en C₁ à C₆), hétérocyclylcarbonyl-(alkyle en C₁ à C₆), hétérocyclylcarbonyle, hétérocyclyloxycarbonyle, hétérocyclyloxythiocarbonyle, le résidu phényle ou hétérocyclyle des résidus cités pouvant être partiellement ou complètement halogénés et/ou pouvant porter deux ou trois substituants choisis parmi un groupe nitro, cyano, alkyle en C₁ à C₄, halogénoalkyle en C₁ à C₄, alcoxy en C₁ à C₄ ou halogénoalcoxy en C₁ à C₄;
R²⁰ représente un groupe alkyle en C₁ à C₆, alcényle en C₃ à C₆, halogénoalcényle en C₃ à C₆, alcynyle en C₃ à C₆ ou cycloalkyle en C₃ à C₆, les résidus alkyle et cycloalkyle cités pouvant être partiellement ou complètement halogénés et/ou pouvant porter un, deux ou trois substituants choisis parmi un groupe cyano, alcoxy en C₁ à C₄, alkylthio en C₁ à C₄, di-(alkyle en C₁ à C₄)amino, (alkyle en C₁ à C₄)carbonyle, (alcoxy en C₁ à C₄)carbonyle, (alcoxy en C₁ à C₄)-(alcoxy en C₁ à C₄)carbonyle, (alkyle en C₁ à C₄)aminocarbonyle, N,N-di-(alkyle en C₁ à C₄)aminocarbonyle, ou cycloalkyle en C₃ à C₆; un groupe phényle, phényl-(alkyle en C₁ à C₆), phénylcarbonyl-(alkyle en C₁ à C₆), hétérocyclyle, hétérocyclyl-(alkyle en C₁ à C₆) ou hétérocyclylcarbonyl-(alkyle en C₁ à C₆), le résidu phényle ou hétérocyclyle des résidus cités pouvant être partiellement ou complètement halogénés et/ou pouvant porter un, deux ou trois substituants choisis parmi un groupe nitro, cyano, alkyle en C₁ à C₄, halogénoalkyle en C₁ à C₄, alcoxy en C₁ à C₄, ou halogénoalcoxy en C₁ à C₄;
p vaut 2, 3 ou 4;
q vaut 1, 2, 3, 4 ou 5;
ainsi que leurs sels utilisables en agriculture.

2. 3-(4,5-dihydroisoxazol-5-yl)benzoylcyclohexénones de formule I selon la revendication 1, dans laquelle
R¹, R² représentent un groupe nitro, un atome d'halogène, un groupe cyano, alkyle en C₁ à C₆, halogénoalkyle en C₁ à C₆, alcoxy en C₁ à C₆, halogénoalcoxy en C₁ à C₆, alkylthio en C₁ à C₆, halogénoalkylthio en C₁ à C₆, alkylsulfinyle en C₁ à C₆, halogénoalkylsulfinyle en C₁ à C₆, alkylsulfonyle en C₁ à C₆, ou halogénoalkylsulfonyle en C₁ à C₆.

3. 3-(4,5-dihydroisoxazol-5-yl)benzoylcyclohexénones de formule I selon les revendications 1 ou 2, dans laquelle R³ représente un atome d'hydrogène.

4. 3-(4,5-dihydroisoxazol-5-yl)benzoylcyclohexénones de formule I selon les revendications 1 à 3, dans laquelle
R⁵ représente un atome d'hydrogène, d'halogène, un groupe cyano, nitro, alkyle en C₁ à C₄, (alcoxy en C₁ à C₄)-(alkyle en C₁ à C₄), (alcoxy en C₁ à C₄)carbonyl-(alkyle en C₁ à C₄), (alkylthio en C₁ à C₄)-(alkyle en C₁ à C₄), halogénoalkyle en C₁ à C₄, cyano-(alkyle en C₁ à C₄), cycloalkyle en C₃ à C₈, alcoxy en C₁ à C₄, (alcoxy en C₁ à C₄)-(alcoxy en C₂ à C₄), halogénoalcoxy en C₁ à C₄, alkylthio en C₁ à C₄, halogénoalkylthio en C₁ à C₄, di-(alkyle en C₁ à C₄)-amino, COR⁸, phényle ou benzyle, les deux substituants cités en dernier pouvant être partiellement ou complètement halogénés et/ou pouvant porter un à trois des groupes suivants :
un groupe nitro, cyano, alkyle en C₁ à C₄, halogénoalkyle en C₁ à C₄, alcoxy en C₁ à C₄ ou halogénoalcoxy en C₁ à C₄;
R⁶ représente un atome d'hydrogène ou un groupe alkyle en C₁ à C₄.

5. 3-(4,5-dihydroisoxazol-5-yl)benzoylcyclohexénones de formule I selon les revendications 1 à 4, dans laquelle R⁵ et R⁶ représentent un atome d'hydrogène.

6. 3-(4,5-dihydroisoxazol-5-yl)benzoylcyclohexénones de formule I selon les revendications 1 à 5, dans laquelle
R¹² représente un groupe hydroxy, OR¹⁹ ou SR¹⁹; et
R¹⁹ représente un groupe alkyle en C₁ à C₆, (alkyle en C₁ à C₂₀)carbonyle, (alcoxy en C₁ à C₆)carbonyle, (alkyle en C₁ à C₆)thiocarbonyle, les résidus alkyle et alcoxy pouvant être partiellement ou complètement halogénés et/ou pouvant porter un, deux ou trois substituants choisis parmi un groupe cyano, alcoxy en C₁ à C₄, alkylthio en C₁ à C₄, (alkyle en C₁ à C₄)carbonyle, (alcoxy en C₁ à C₄)carbonyle ou cycloalkyle en C₃ à C₆;
un groupe phényle, phényl-(alkyle en C₁ à C₄), phénylcarbonyl-(alkyle en C₁ à C₄), phénylcarbonyle, phénoxycarbonyle, hétérocyclyle, hétérocyclyl-(alkyle en C₁ à C₄), hétérocyclylcarbonyl-(alkyle en C₁ à C₄), hétérocyclyloxycarbonyle, le résidu phényle ou hétérocyclyle des résidus cités pouvant être partiellement ou complètement halogénés et/ou pouvant porter un, deux ou trois substituants choisis parmi un groupe nitro, cyano, alkyle en C₁ à C₄, halogénoalkyle en C₁ à C₄, alcoxy en C₁ à C₄) ou halogénoalcoxy en C₁ à C₄.

7. 3-(4,5-dihydroisoxazol-5-yl)benzoylcyclohexénones de formule I selon les revendications 1 à 6, dans laquelle R¹² représente un groupe hydroxy.

8. 3-(4,5-dihydroisoxazol-5-yl)benzoylcyclohexénones de formule I selon les revendications 1 à 7, dans laquelle
R¹³ et R¹⁷ représentent indépendamment l'un de l'autre un atome d'hydrogène, un groupe alkyle en C₁ à C₄ ou alkylthio en C₁ à C₄;
R¹⁴, R¹⁶, R¹⁸ représentent indépendamment l'un de l'autre un atome d'hydrogène ou un groupe méthyle;
R¹⁵ représente un atome d'hydrogène, un groupe hydroxy, alkyle en C₁ à C₆ ou di(alcoxy en C₁ à C₆)méthyle;
ou
R¹³ et R¹⁴ ou R¹⁴ et R¹⁵ ou R¹⁵ et R¹⁸ ou R¹⁴ et R¹⁸ ou R¹⁷ et R¹⁸ représentent conjointement un groupe alcanediyle en C₁ à C₅, qui peut porter un, deux ou trois substituants choisis parmi un atome d'halogène, un groupe cyano, alkyle en C₁ à C₄, halogénoalkyle en C₁ à C₄, ou (alcoxy en C₁ à C₄)carbonyle;
ou
R¹⁵ et R¹⁶ représentent conjointement un atome d'oxygène.

9. Procédé de préparation des 3-(4,5-dihydroisoxazol-5-yl)benzoylcyctohexénones de formule I selon la revendication 1, **caractérisé en ce que**, on acyle la cyclohexénone de formule II avec R¹² = OH, dans laquelle les variables R¹³ à R¹⁸ prennent les significations citées sous la revendication 1, 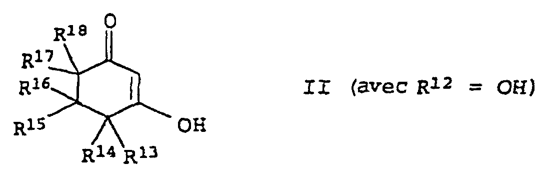 avec un acide carboxylique activé IIIα ou avec un acide carboxylique IIIβ, 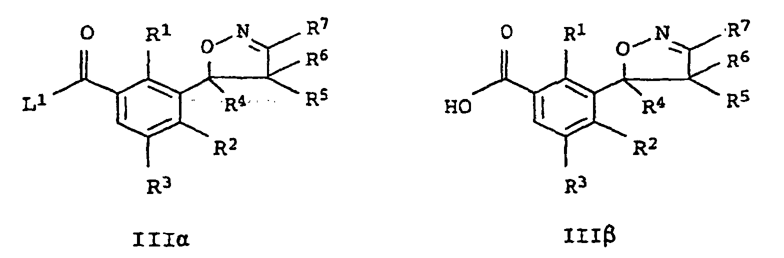 dans lesquelles les variables R¹ à R⁷ prennent les significations citées sous la revendication 1, et L¹ représente un groupe sortant remplaçable de façon nucléophile, et on réarrange le produit d'acylation en composés I (avec R¹² = OH) en présence d'un catalyseur.

10. Procédé de préparation des 3-(4,5-dihydroisoxazol-5-yl)benzoylcyclohexénones de formule I avec R¹² = OR¹⁹ ou SR¹⁹ selon la revendication 1, dans lequel on fait réagir un composé de formule I avec R¹² = OH ou SH, 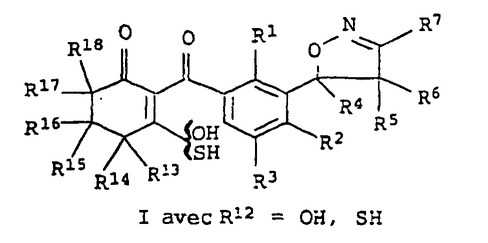 dans laquelle R¹ à R⁷ et R¹³ à R¹⁸ prennent la signification indiquée à la revendication 1, avec un composé de formule V
L²-R¹⁹ V
dans laquelle la variable R¹⁹ prend la signification indiquée à la revendication 1, et L² représente un groupe sortant remplaçable de façon nucléophile.

11. Procédé de préparation des 3-(4,5-dihydroisoxazol-5-yl)benzoylcyclohexénones de formule 1 avec R¹² = un atome d'halogène selon la revendication 1, dans lequel on fait réagir un composé de formule I avec R¹² = OH, 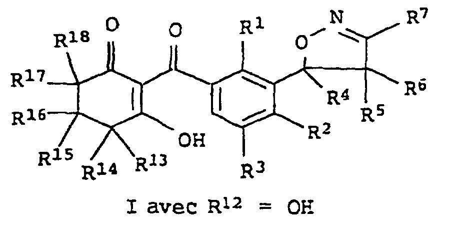 dans laquelle les variables R¹ à R⁷ et R¹³ à R¹⁸ prennent la signification citée à la revendication 1, avec un agent d'halogénation.

12. Procédé de préparation des 3-(4,5-dihydroisoxazol-5-yl)benzoylcyclohexénones de formule I, avec R¹² = un groupe mercapto, OR¹⁹ ou SR¹⁹ selon la revendication 1, dans lequel on fait réagir un composé de formule 1 avec R¹² = un atome d'halogène, 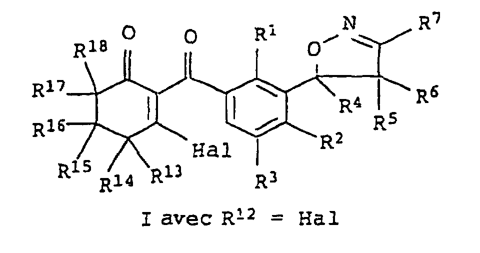 dans laquelle les variables R¹ à R⁷ et R¹³ à R¹⁸ prennent la signification citée à la revendication 1, avec un composé de formule VI.
H₂S ou HOR¹⁹ ou HSR¹⁹ VI
dans lesquelles R¹⁹ prend la signification indiquée à la revendication 1, éventuellement en présence d'une base.

13. Procédé de préparation des 3-(4,5-dihydroisoxazol-5-yl)benzoylcyclohexénones de formule I, avec R¹² = un groupe SOR²⁰ ou SO₂R²⁰ selon la revendication 1, dans lequel on fait réagir un composé de formule I avec R¹² = SR²⁰, 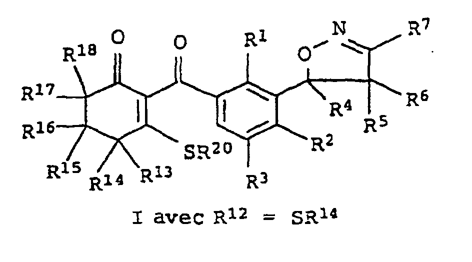 dans laquelle les variables R¹ à R⁷ et R¹³ à R¹⁸ prennent la signification citée à la revendication 1, avec un agent d'oxydation.

14. Agents, contenant une quantité efficace en tant qu'herbicide d'au moins une 3-(4,5-dihydroisoxazol-5-yl)benzoylcyclohexénone de formule I, ou un sel utilisable en agriculture de celui-ci selon les revendications 1 à 8, et des adjuvants habituels pour la formulation d'agents phytosanitaires.

15. Procédé de préparation d'agents selon la revendication 14, **caractérisé en ce que** l'on mélange une quantité efficace en tant qu'herbicide d'au moins une 3-(4,5-dihydroisoxazol-5-yl)benzoylcyclohexénone de formule I, ou un sel utilisable en agriculture de I selon les revendications 1 à 8 et des adjuvants habituels pour la formulation d'agents phytosanitaires.

16. Procédé de lutte contre le développement végétal non souhaité, **caractérisé en ce que** l'on laisse agir une quantité efficace en tant qu'herbicide d'au moins une 3-(4,5-dihydroisoxazol-5-yl)benzoylcyclohexénone de formule I, ou un sel utilisable en agriculture de I de celle-ci, selon les revendications 1 à 8, sur des plantes, leurs biotopes et/ou sur des semences.

17. Utilisation des 3-(4,5-dihydroisoxazol-5-yl)benzoylcyclohexénones de formule I, et/ou leurs sels utilisables en agriculture selon les revendications 1 à 8, en tant qu'herbicides.
